# EUROPEAN PATENT APPLICATION

(11) **EP 4 741 007 A2**
(43) Date of publication of application: **13.05.2026**
(21) Application number: 26167208.3
(22) Date of filing: 31.01.2022
(51) Int. Cl.: A61P 9/10

(54) **METHOD OF TREATING OR PREVENTING AN ADVERSE SECONDARY NEUROLOGICAL OUTCOME FOLLOWING A HAEMORRHAGIC STROKE**

(30) Priority: 01.02.2021 US 202163144043 P
(62) Divisional of application: 22709950.4
(71) Applicant: CSL Behring AG, 3014 Bern (CH); Universität Zürich, 8006 Zürich (CH)
(72) Inventor: The designation of the inventor has not yet been filed
(74) Representative: Kraus & Lederer PartGmbB

(57) **Abstract**

The present disclosure relates generally to methods of treating or preventing an adverse secondary neurological outcome in a subject following a haemorrhagic stroke accompanied by extravascular erythrolysis and release of cell-free heme and/or cell-free haemoglobin (Hb) into a cerebral spinal fluid (CSF), the method comprising exposing the CSF of a subject in need thereof to a therapeutically effective amount of hemopexin (Hx) and for a period of time sufficient to allow the Hx to form a complex with, and thereby neutralise, the cell-free heme and, optionally, exposing the CSF of the subject to a therapeutically effective amount of haptoglobin (Hp) and for a period of time sufficient to allow the Hp to form a complex with, and thereby neutralise, the cell-free Hb.

## Description

### TECHNICAL FIELD

The present invention relates generally to methods and compositions for treating and/or preventing an adverse secondary neurological outcome in a subject following a haemorrhagic stroke into a cerebral spinal fluid (CSF) compartment, in particular following subarachnoid hemorrhage (SAH).

### BACKGROUND

All references, including any patents or patent application, cited in this specification are hereby incorporated by reference to enable full understanding of the invention. Nevertheless, such references are not to be read as constituting an admission that any of these documents forms part of the common general knowledge in the art, in Australia or in any other country.

Haemorrhagic stroke involves the rupture of a blood vessel in or on the surface of the brain with bleeding into the surrounding tissue. Examples of haemorrhagic stroke include i) intracerebral haemorrhage (herein referred to as ICH) which involves a blood vessel in the brain bursting; ii) intraventricular haemorrhage (herein referred to as IVH) which is bleeding into the brains ventricular system; and iii) subarachnoid haemorrhage (herein referred to as SAH) which involves bleeding in the space between the brain and the tissue covering the brain known as the subarachnoid space. Most often SAH is caused by a burst aneurysm (herein referred to as aSAH). Other causes of SAH include head injury, bleeding disorders and the use of blood thinners.

Aneurysmal subarachnoid hemorrhage (aSAH) is the most common cause of SAH and is associated with the highest rates of mortality and long-term neurological disabilities. Despite advances in aneurysm repair and neurointensive care, the median in-hospital case fatality rate in Europe is 44.4% and 32.2% in the United States. 35% of the survivors report a poor overall quality of life 1 year after the bleeding event with 83-94% not able to return to work. The estimated incidence of aSAH from a ruptured intracranial aneurysm in the U.S. is 1 case per 10,000 persons, yielding approximately 27,000 new cases each year.

Additionally, aSAH is more common in women than in men (2:1); the peak incidence is in persons 55 to 60 years old.

Besides early brain injury within the first 72 hours (Sehba *et al.,* 2012), patient outcomes after aSAH are determined by delayed secondary brain injury, which occurs between day 4 to 10 after aneurysm rupture (Macdonald, 2014). Delayed secondary brain injury is assumed to be multifactorial involving macro- and microvascular dysfunction, neuroinflammation, neuronal apoptosis, and pathological electrical activity of the brain (Macdonald, 2014). Two-thirds of patients after aSAH develop angiographic vasospasms of large cerebral arteries (aVSP) (Dorsch and King, 1994). Delayed cerebral ischemia (DCI) with radiologic demarcation of ischemic brain areas and clinically evident delayed ischemic neurologic deficits (DIND) are found in one-third of patients (Rowland *et al.,* 2012). The occurrence of at least one of these secondary manifestations defines subarachnoid hemorrhage related secondary brain injury (SAH-SBI).

The lag-time between aneurysm rupture and the onset of SAH-SBI provides a window of opportunity for preventative and therapeutic interventions, defining an unmet need for identifying patients at high risk for SAH-SBI as well as new drug targets. So far, the only preventative intervention that has been shown to moderately improve neurological outcomes after aSAH is oral nimodipine (Class I, Level A) (Diringer *et al.,* 2011; Connolly *et al.,* 2012). In symptomatic patients, therapeutic options are currently limited to rescue therapies with induction of systemic arterial hypertension and, in selected patients, mechanical or chemical angioplasty to resolve aVSP (Diringer *et al.,* 2011; Connolly *et al.,* 2012). Hence, there is an urgent and unmet need for specific therapies to treat and / or prevent SAH-SBI in patients following aSAH.

Despite enormous research efforts, there is still no clinically established biomarker for reliable monitoring of SAH-SBI. Although widely used, clinical scores (Hunt and Hess, 1968; Teasdale *et al.,* 1988), radiological scores (Fisher *et al.,* 1980; Frontera *et al.,* 2006; Wilson *et al.* , 2012), and day-to-day assessment with transcranial doppler sonography (TCD) (Diringer *et al.,* 2011; Connolly *et al.,* 2012) show a limited accuracy for the detection of patients at risk (de Rooij *et al.,* 2013).

Cell-free hemoglobin (Hb) accumulates in patient-CSF and has been considered as an upstream driver of SAH-SBI (Pluta *et al.,* 2009; Hugelshofer *et al.,* 2019; Buehler *et al.,* 2020). Data from a small pilot study with daily CSF spectrophotometry in 18 patients indicated that patients with a high cumulative CSF-Hb exposure over two weeks after aneurysm rupture may have an increased risk to develop SAH-SBI (Hugelshofer *et al.,* 2018). However, so far no correlation between the concentration of cell-free Hb in the patient's CSF (CSF-Hb) and the incidence of subarachnoid haemorrhage related secondary brain injury (SAH-SBI) has been clinically demonstrated. In particular, no clinically relevant CSF-Hb concentration is known which could be used as relevant biomarker of SAH-SBI.

Collectively, there is an urgent clinical need for diagnostic tools to predict and monitor for SAH-SBI and for targeted therapeutic approaches to prevent and treat SAH-SBI.

### SUMMARY OF THE INVENTION

The present inventors undertook a prospective study in a cohort of patients with aSAH and unexpectedly found a significant correlation between the concentration of cell-free Hb in the CSF of these patients (CSF-Hb) and the incidence of subarachnoid haemorrhage related secondary brain injury (SAH-SBI). The diagnostic accuracy of the correlation of CSF-Hb with SAH-SBI was identified within a narrow clinically relevant CSF-Hb concentration range and was found to markedly exceed other physiological and biochemical biomarkers of aSAH, as well as radiological and clinical scores. Remarkably, the significant correlation between CSF-Hb and the incidence of SAH-SBI was observed for all three manifestations of SAH-SBI; namely angiographic vasospasms of large cerebral arteries (aVSP), delayed cerebral ischemia (DCI) and delayed ischemic neurologic deficits (DIND). The present inventors also undertook an in-depth CSF proteomis analysis and *ex vivo* functional assays that identified CSF-Hb as a pathophysiological driver and therapeutic target.

The present inventors have also surprisingly found that hemopexin (Hx) can reduce or otherwise prevent cell-free heme-mediated adverse secondary neurological outcomes (*e.g*., oxidative tissue damage, neuroinflammation), including functional and radiological neurological impairment. It was found that Hx is capable of selectively neutralising the lipid oxidation activity of patient's CSF-Hb within the herein identified clinically relevant concentration range of CSF-Hb. The present inventors have further surprisingly found that Haptoglobin (Hp) exerts its anti-vasospastic and anti-oxidative effects within the herein identified clinically relevant CSF-Hb concentration range. In addition, the present inventors have further surprisingly found that Haptoglobin (Hp) and Hemopexin (Hx) may synergistically exert their protective function.

Thus, in an aspect of the present invention, there is provided a method of treating or preventing an adverse secondary neurological outcome in a subject following a haemorrhagic stroke accompanied by extravascular erythrolysis and release of cell-free heme and/or cell-free haemoglobin (Hb) into a cerebral spinal fluid (CSF), the method comprising exposing the CSF of a subject in need thereof to a therapeutically effective amount of hemopexin (Hx) and for a period of time sufficient to allow the Hx to form a complex with, and thereby neutralise, the cell-free heme. In an embodiment, the method further comprises exposing the CSF of the subject to a therapeutically effective amount of haptoglobin (Hp) and for a period of time sufficient to allow the Hp to form a complex with, and thereby neutralise, cell-free Hb.

In another aspect disclosed herein, there is provided a pharmaceutical composition for treating or preventing an adverse secondary neurological outcome in a subject following a haemorrhagic stroke in accordance with the methods described herein, the composition comprising a therapeutically effective amount of hemopexin (Hx) and a pharmaceutically acceptable carrier. In an embodiment, the composition further comprises a therapeutically effective amount of haptoglobin (Hp).

In another aspect disclosed herein, there is provided a pharmaceutical composition for use in treating or preventing an adverse secondary neurological outcome in a subject following a haemorrhagic stroke in accordance with the methods described herein, the composition comprising a therapeutically effective amount of hemopexin (Hx) and a pharmaceutically acceptable carrier. In an embodiment, the composition further comprises a therapeutically effective amount of haptoglobin (Hp).

In another aspect disclosed herein, there is provided use of a therapeutically effective amount of hemopexin (Hx) in the manufacture of a medicament for treating or preventing an adverse secondary neurological outcome in a subject following a haemorrhagic stroke in accordance with the methods described herein. In an embodiment, the therapeutically effective amount of Hx is formaulated for administration with a therapeutically effective amount of haptoglobin (Hp).

In another aspect disclosed herein, there is provided a therapeutically effective amount of hemopexin (Hx) for use in the treatment or prevention of an adverse secondary neurological outcome in a subject following a haemorrhagic stroke in accordance with the method described herein. In an embodiment, the therapeutically effective amount of Hx is formulated for use with a therapeutically effective amount of haptoglobin (Hp).

In another aspect disclosed herein, there is provided an artificial CSF comprising Hx and, optionally, Hp, as described herein. The present disclosure also extends to kits comprising an artificial CSF, or a composition, as described herein.

In another aspect disclosed herein, there is provided a method of determining whether a subject is at risk of developing an adverse secondary neurological outcome following a haemorrhagic stroke accompanied by extravascular erythrolysis and release of cell-free heme and/or cell-free haemoglobin (Hb) into a cerebral spinal fluid (CSF), the method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).

In some embodiments, the method further comprises treating the subject determined to be at risk of an adverse secondary neurological outcome, wherein said treatment comprises exposing the CSF of the subject to (i) a therapeutically effective amount of hemopexin (Hx) and for a period of time sufficient to allow the Hx to form a complex with, and thereby neutralise, the cell-free heme, as described herein; and / or (ii) a therapeutically effective amount of haptroglobin (Hp) and for a period of time sufficient to allow the Hp to form a complex with, and thereby neutralise, the cell-free Hb, as described herein. Thus, in another aspect disclosed herein, there is provided a method of stratifying a subject to a treatment for an adverse secondary neurological outcome in a subject following a haemorrhagic stroke accompanied by extravascular erythrolysis and release of cell-free heme and/or cell-free haemoglobin (Hb) into a cerebral spinal fluid (CSF), wherein the method comprises (a) determining whether a subject is at risk of developing an adverse secondary neurological outcome, as herein described, and (b) treating the subject determined to be at risk of an adverse secondary neurological outcome in accordance with the methods described herein.

### BRIEF DESCRIPTION OF THE FIGURES

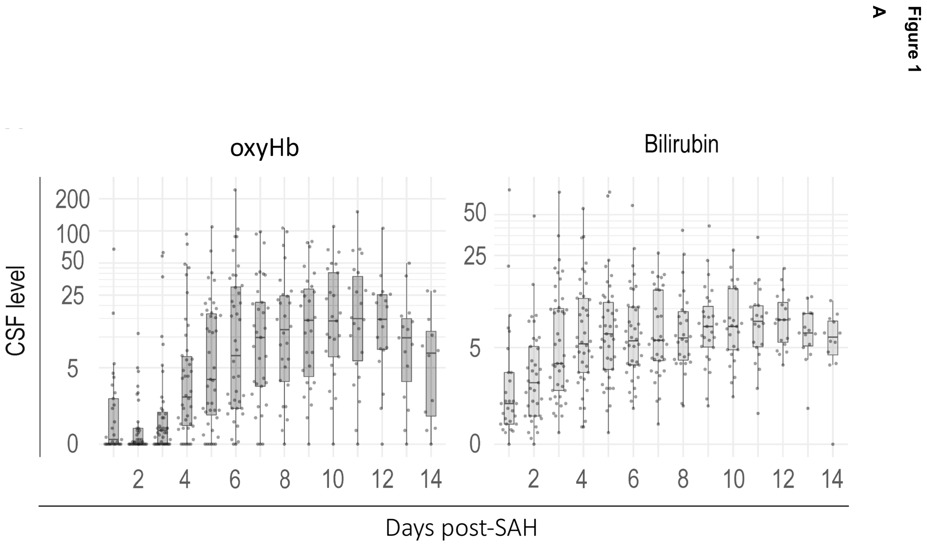

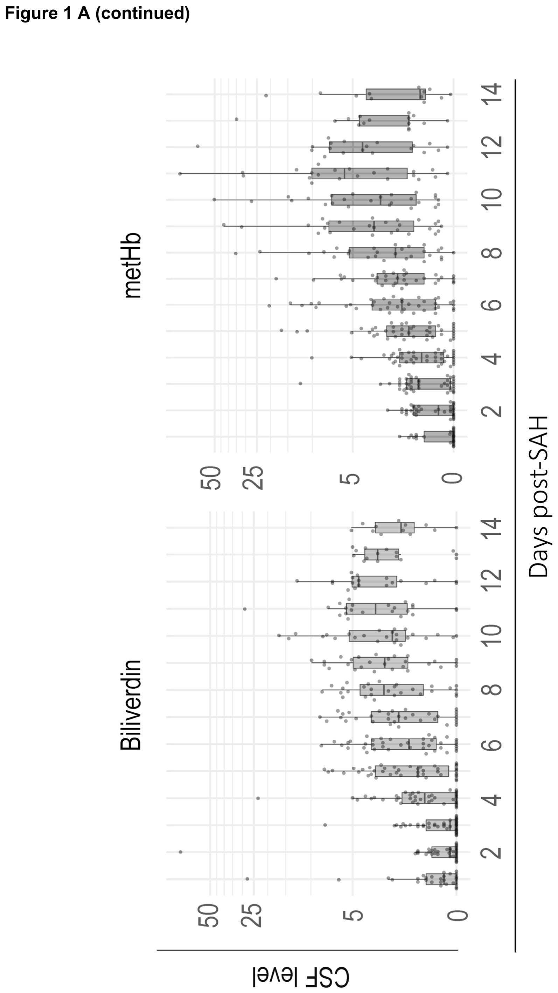

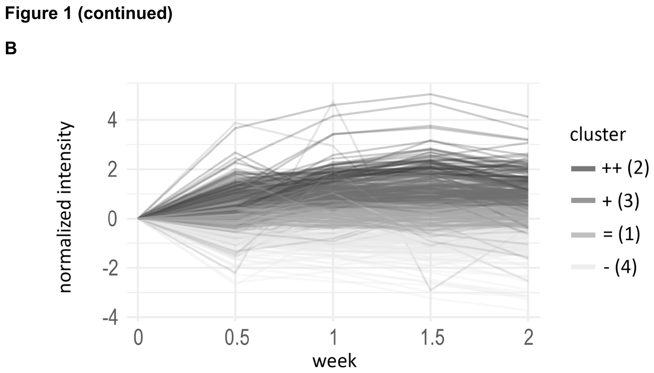

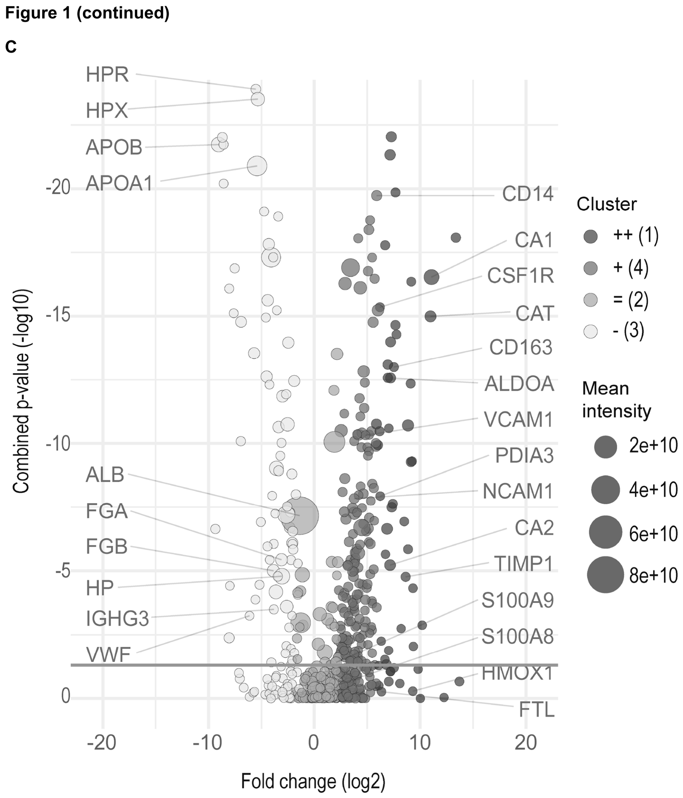

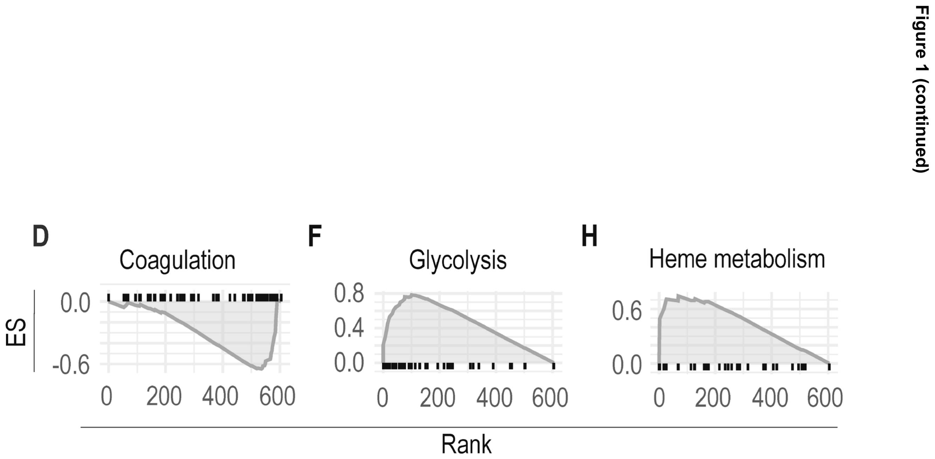

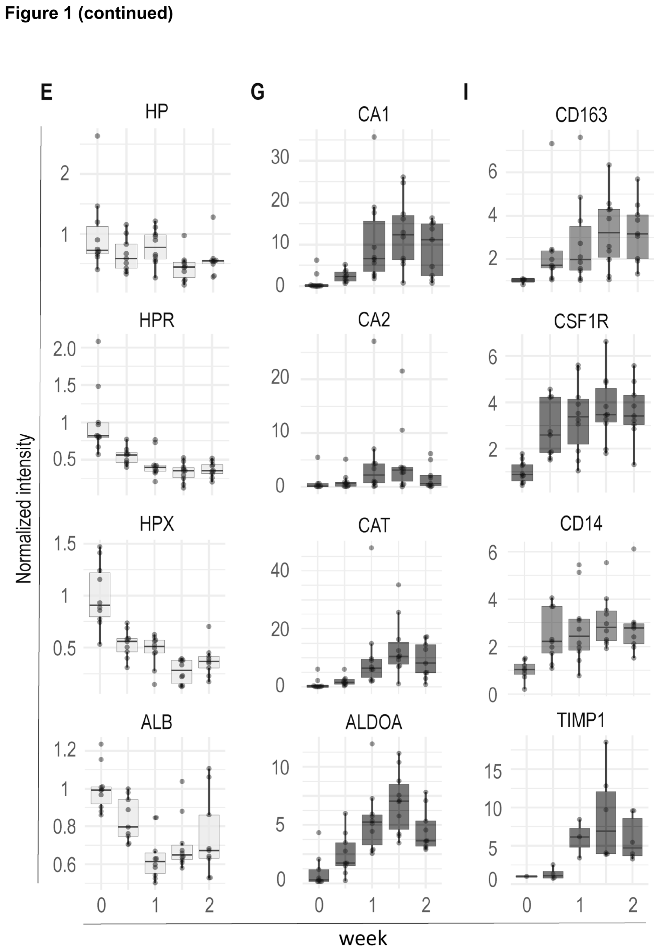

Figure 1 shows changes in the cerebrospinal fluid proteome after aneurysmal subarachnoid haemorrhage; A. Temporal profiles of cerebrospinal fluid (CSF) oxyhemoglobin (oxyHb), bilirubin, biliverdin and methemoglobin (metHb). A. K-means clustering of the proteins identified in the CSF after aSAH with LC-MS/MS. B . Volcano plot with the overall fold change and the combined p-value in the CSF proteome. Color represents the cluster and the size of the dots represent the raw mean intensity of the protein (not normalized). C. Enrichment plot of the top negatively enriched hallmark gene set coagulation identified in the GSEA of the CSF proteome after aSAH. D. Temporal course of the normalized protein intensities of haptoglobin (HP), haptoglobin related protein (HPR), hemopexin (HPX) and albumin (ALB) in the CSF after aSAH. E. Enrichment plot of the top positively enriched hallmark gene set glycolysis identified in the GSEA of the CSF proteome after aSAH. F. Enrichment plot of the hallmark gene set heme metabolism identified in the GSEA of the CSF proteome after aSH. G. Temporal course of the normalized protein intensities of carboanhydrase 1 (CA1), carboanhydrase 2 (CA2), catalase (CAT) and aldolase A (ALDOA) in the CSF. H. Temporal course of the normalized protein intensities of CD163, colony stimulating factor 1 receptor (CSF1R), CD14 and metalloproteinase inhibitor 1 (TIMP) in the CSF.

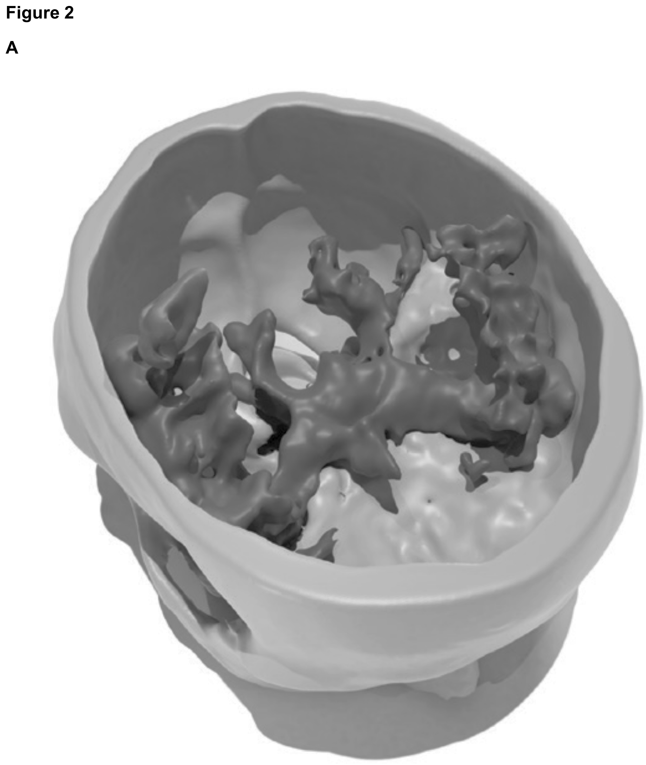

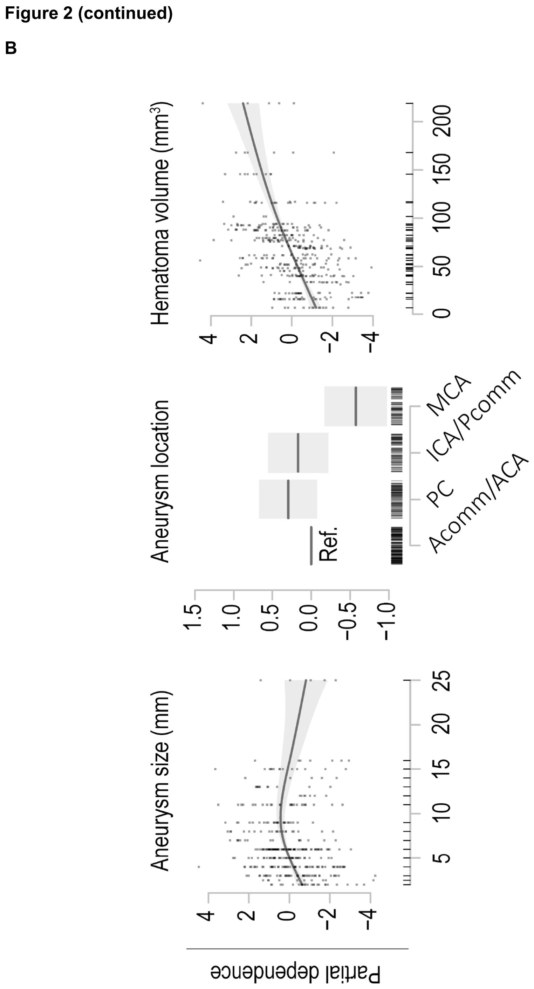

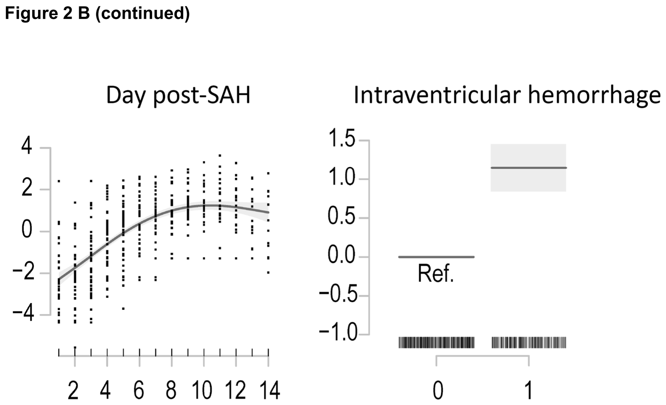

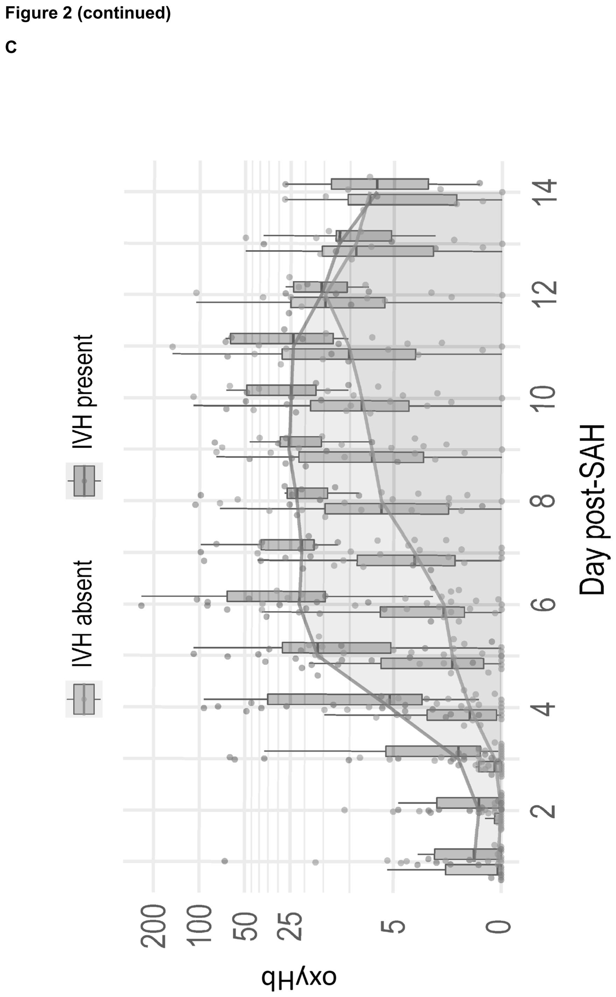

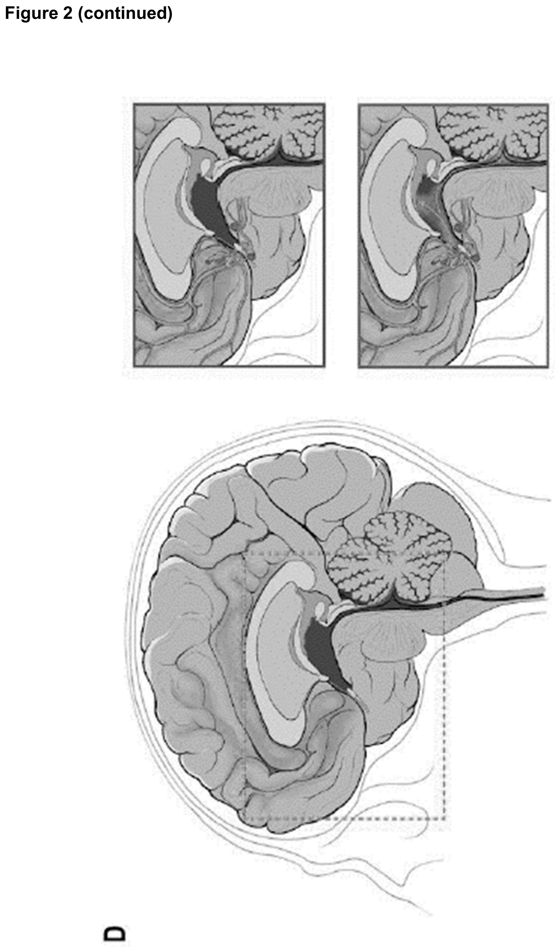

Figure 2 shows determinants of CSF-Hb. A. Example of a 3D rendered subarachnoid hematoma. B. Partial dependence of CSF Hb levels on aneurysm size, location of the aneurysm, hematoma volume, presence of intraventricular haemorrhage (IVH), and day after aSAH according to a generalized additive model. C. The temporal course of CSF hemoglobin after aSAH stratified for the presence of an IVH. D. Schematic illustration of a subarachnoid hemorrhage originating from the rupture of an aneurysm of the anterior communicating artery comparing the situation without (blue inset) or with (red inset) IVH.

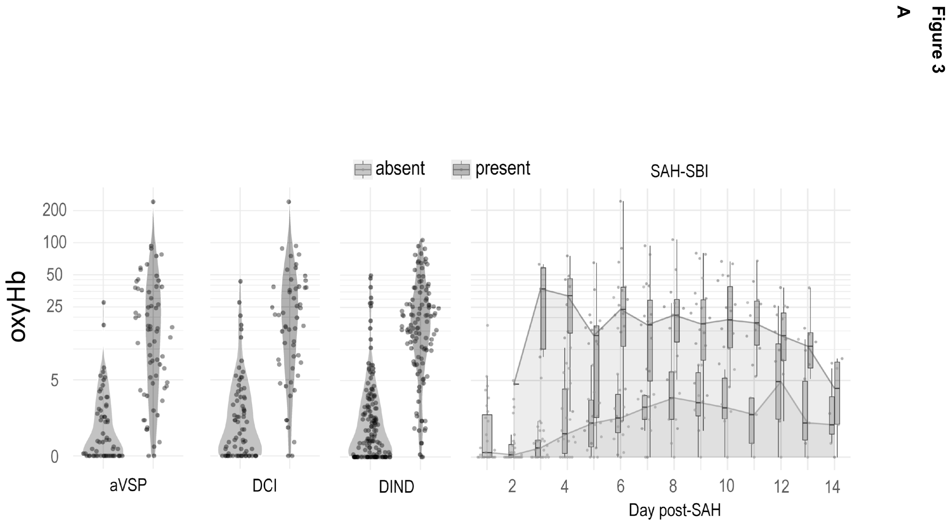

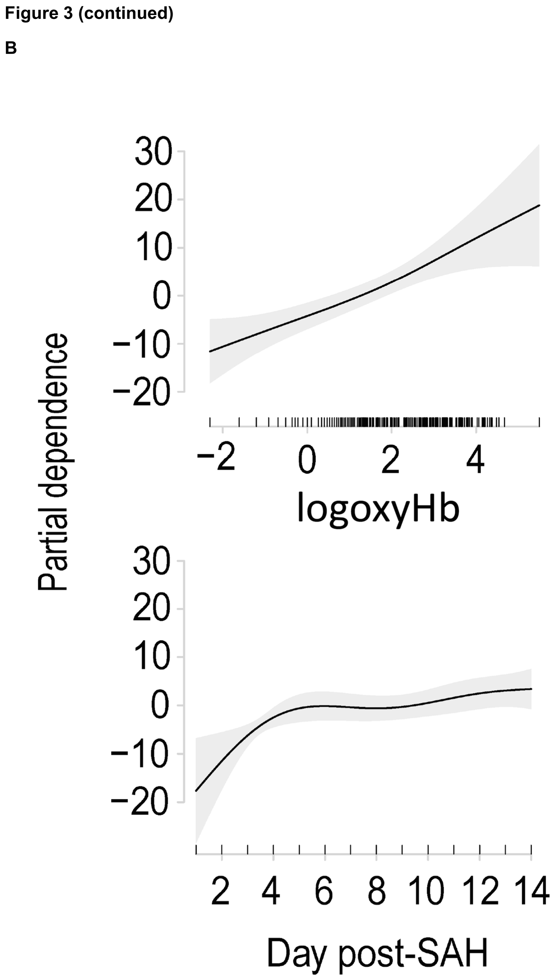

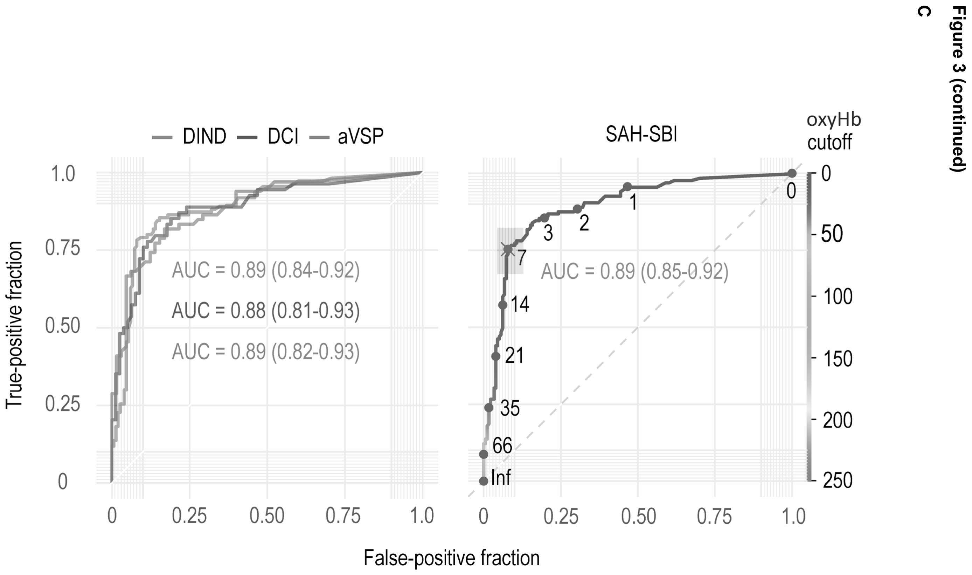

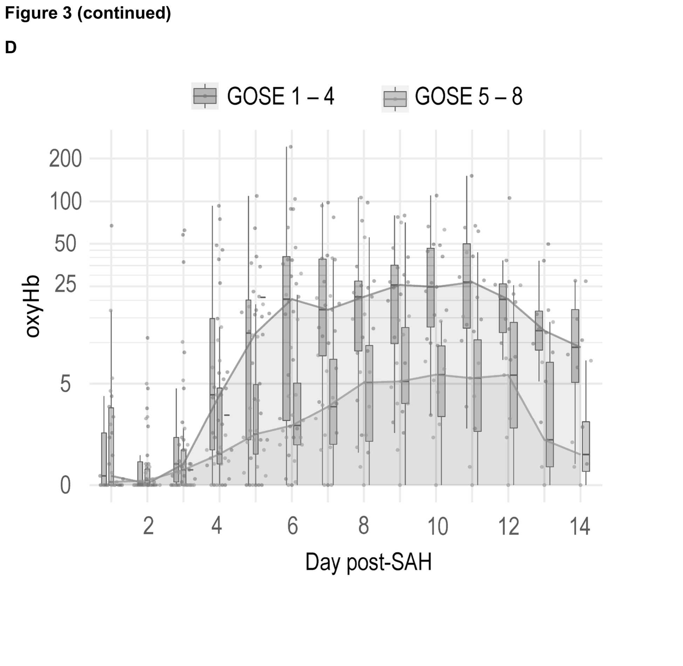

Figure 3 shows the association of cerebrospinal fluid hemoglobin with secondary brain damage after aneurysmal subarachnoid hemorrhage; A. Cerebrospinal fluid hemoglobin (Hb) in patients after aneurysmal subarachnoid hemorrhage stratified by angiographic vasospasm (aVSP), delayed cerebral ischemia (DCI), delayed ischemic neurological deficit (DIND) and the composite outcome secondary brain injury per day (SAH-SBI). B. Partial dependence of SAH-SBI on CSF-Hb (log-scale) and the day after aSAH according to a generalized additive model. C. Receiver operating characteristics (ROC) curve and area under the curve (AUC) of Hb for the presence of DIND, DCI and aVSP (left). ROC curve of Hb with the respective values for the occurrence of SAH-SBI. D. The temporal course of CSF Hb stratified by GOSE at 3-months follow-up (good vs. poor).

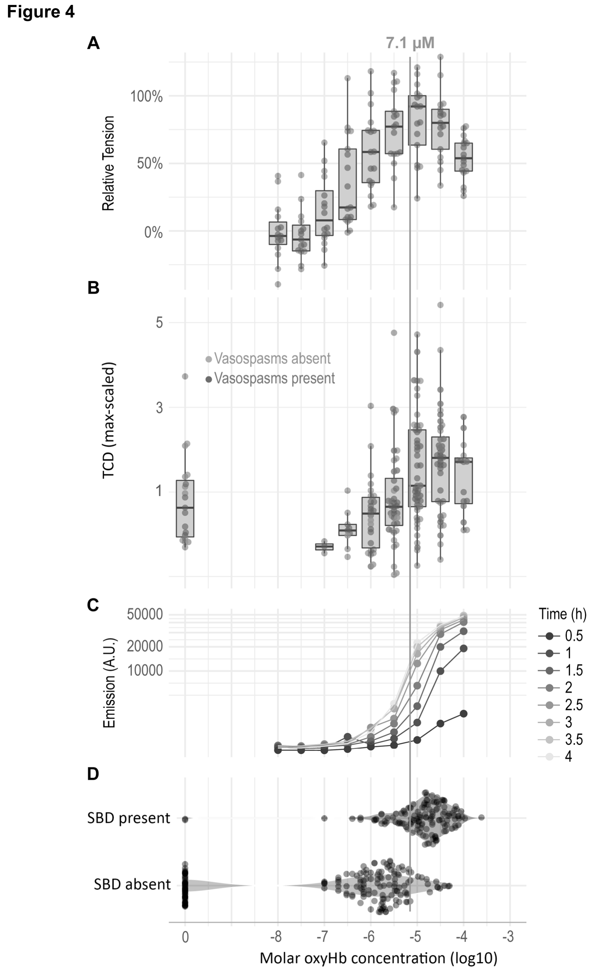

Figure 4 shows the vasoconstrictive and oxidative potential of haemoglobin; A. Vascular tension dose response to hemoglobin (Hb). B. Transcranial doppler (TCD) velocity (max. TCD scaled) measured in patients correlated to CSF Hb levels. The presence of aVSP is given as color-overlay. C. Formation of malondialdehyde in response to different concentrations of Hb in a TBARS assay. D. Correlation of measured Hb concentrations in the CSF to the occurrence of secondary brain injury (SBI).

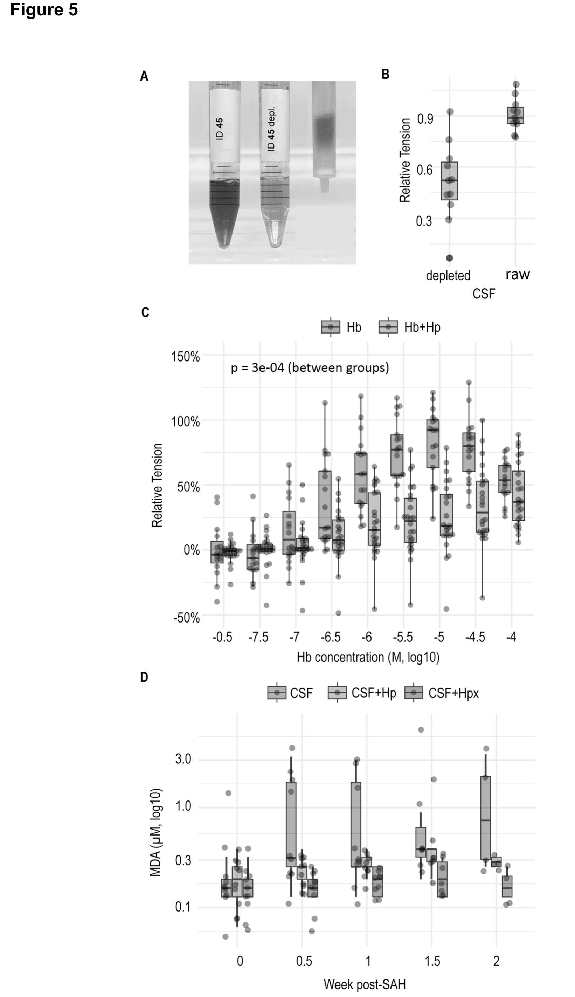

Figure 5 shows the protective effects of haptoglobin on the vascular and oxidative Hb-effects; A. Hb-depletion of CSF using a Hp-affinity column. B. Vascular tension from vessels immersed in depleted CSF, and CSF containing Hb. C. dose response to Hb with (data from Figure 4A) or without haptoglobin (Hp). D. Temporal course of the oxidative potential of patient CSF after aSAH with and without addition of Hp or Hemopexin (Hpx) assessed by malondialdehyde with the thiobarbituric acid reactive substances (TBARS) assay.

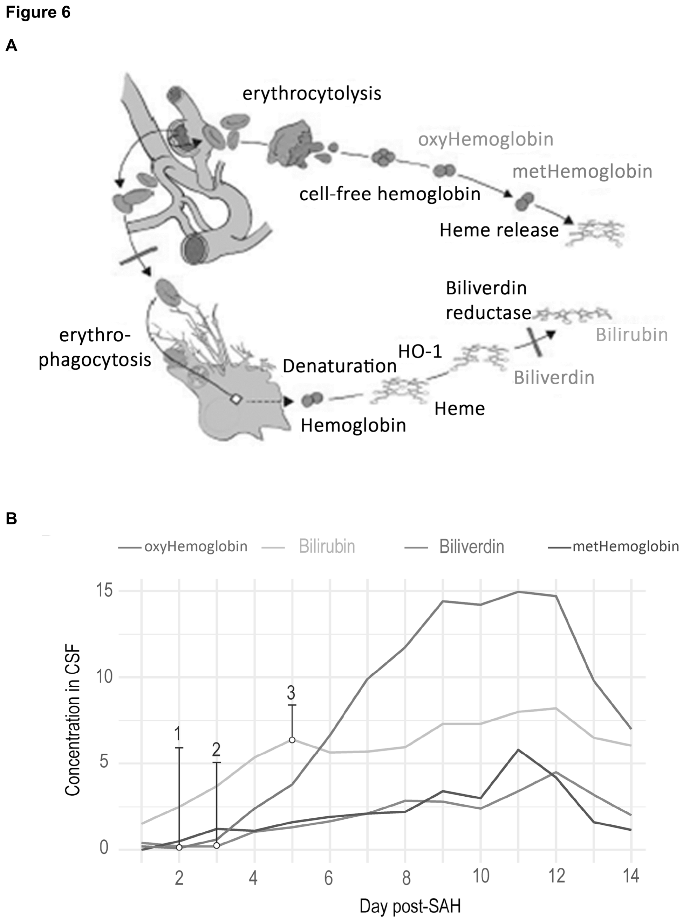

Figure 6 shows the pathophysiology of cerebrospinal fluid hemoglobin and heme metabolites after aneurysmal subarachnoid haemorrhage; A. Schematic illustration of the pathophysiological processes in the subarachnoid microenvironment after aneurysmal subarachnoid hemorrhage. The erythrophagocytosis and the consecutive intracellular processes of hemoglobin denaturation and heme metabolization is shown in the bottom area. The saturations of the biliverdin reductase and the erythrophagocytosis are indicated by red bars. Erythrocytolysis in the subarachnoid CSF space is shown in the upper part of the scheme. Liberated oxyhemoglobin is oxidized to methemoglobin before Heme is released. B. CSF concentrations of Hb, bilirubin, biliverdin and metHb over time with the assumed times of saturation of the biliverdin reductase and of the phagocytosis are indicated by vertical bars.

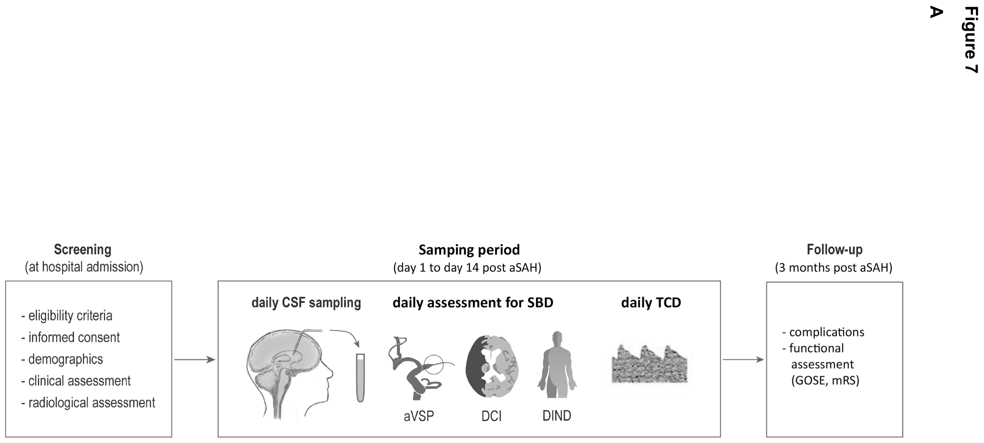

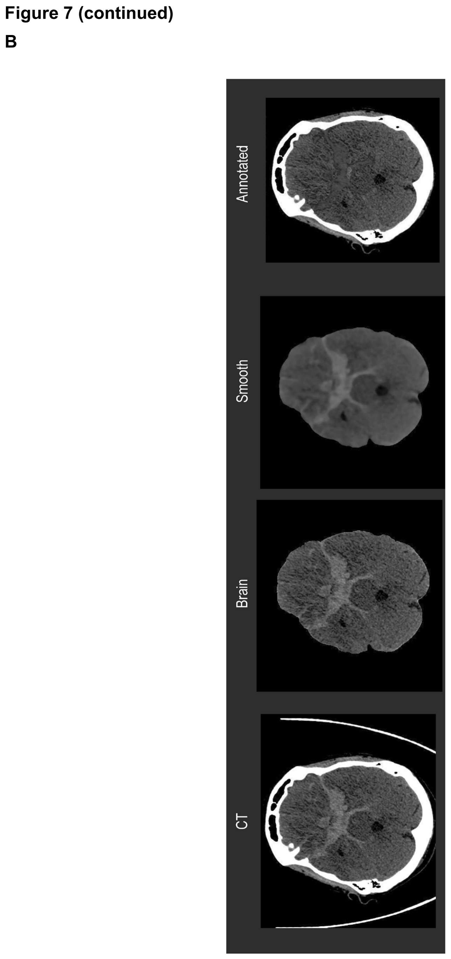

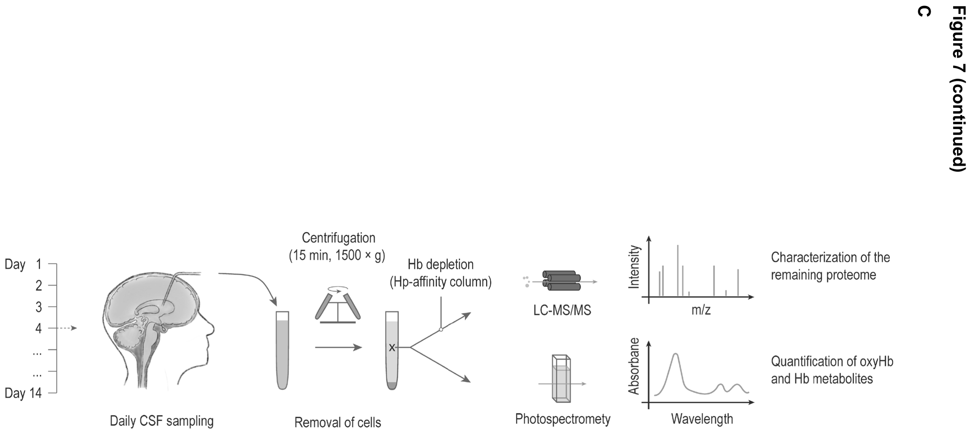

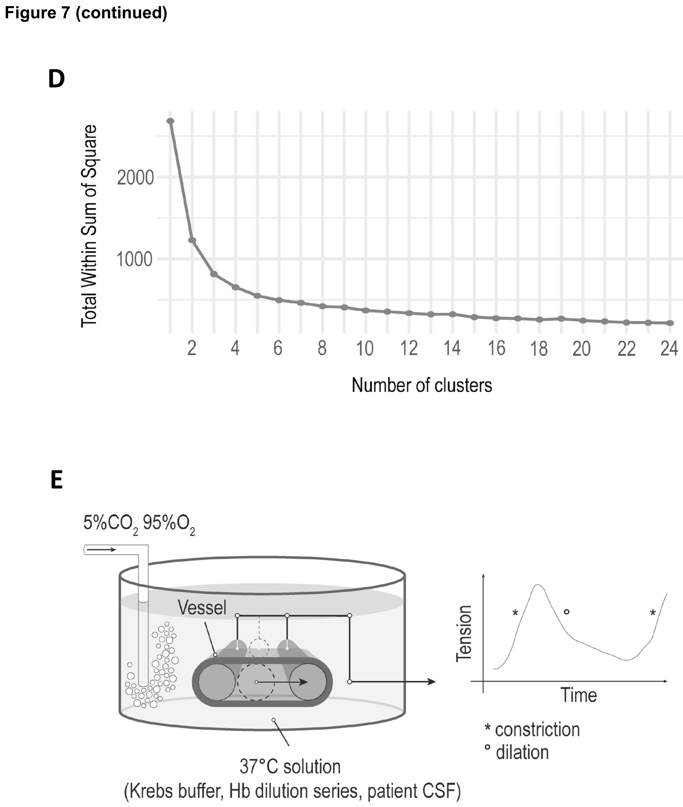

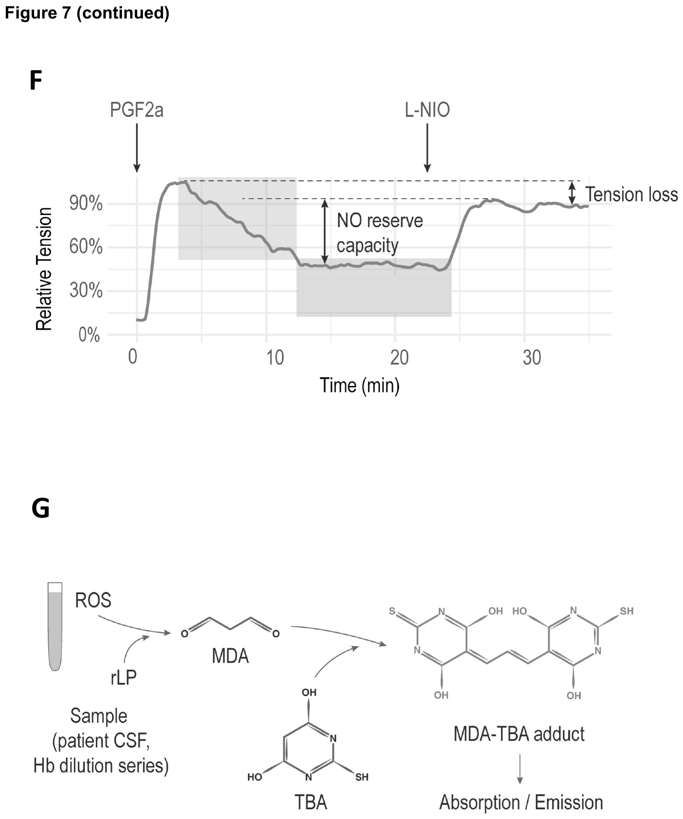

Figure 7 shows A. Flow diagram of the clinical observational study. B. Schematic representation of the daily CSF sampling via an EVD, centrifugation of the CSF and proteome analysis of the supernatant using photospectrometry and LC-MS/MS. C. Steps of subarachnoid hemorrhage segmentation for volumetric analysis. D. Elbow plot to determine the optimal number of protein clusters. E. Schematic representation of the neurovascular functions experimental setup. F. Normalization method of the recorded vessel tension to the individual NO reserve capacity of the respective vessel. G. Schematic representation of the steps in the TBARS assay.

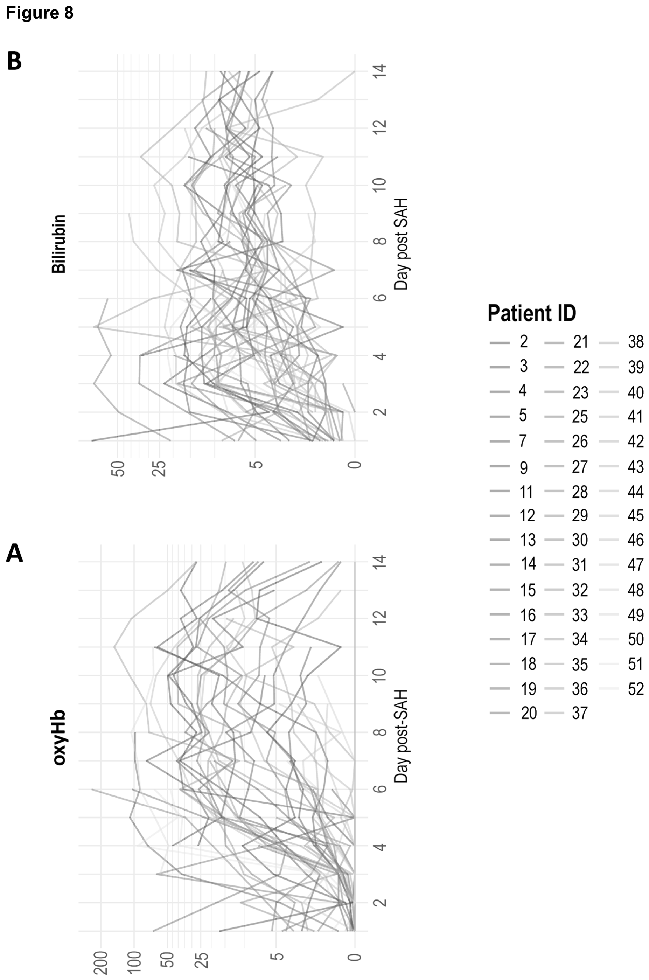

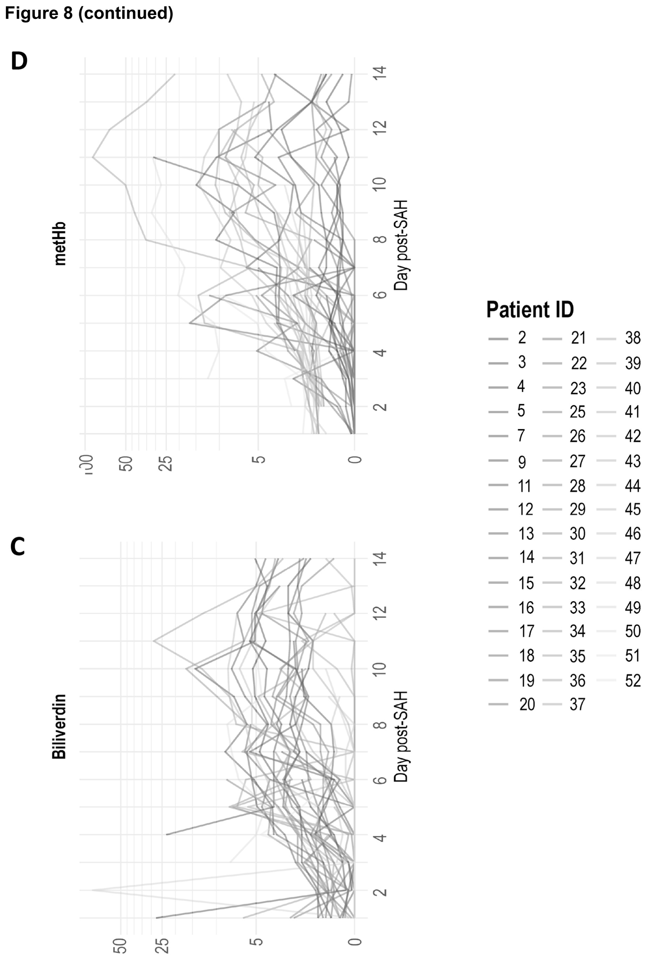

Figure 8 shows individual profiles of CSF-Hb and heme metabolites; A. Individual temporal profiles of oxyhemoglobin (oxyHb). B. Individual temporal profiles of bilirubin. C. Individual temporal profiles of biliverdin. D. Individual temporal profiles of methemoglobin (metHb).

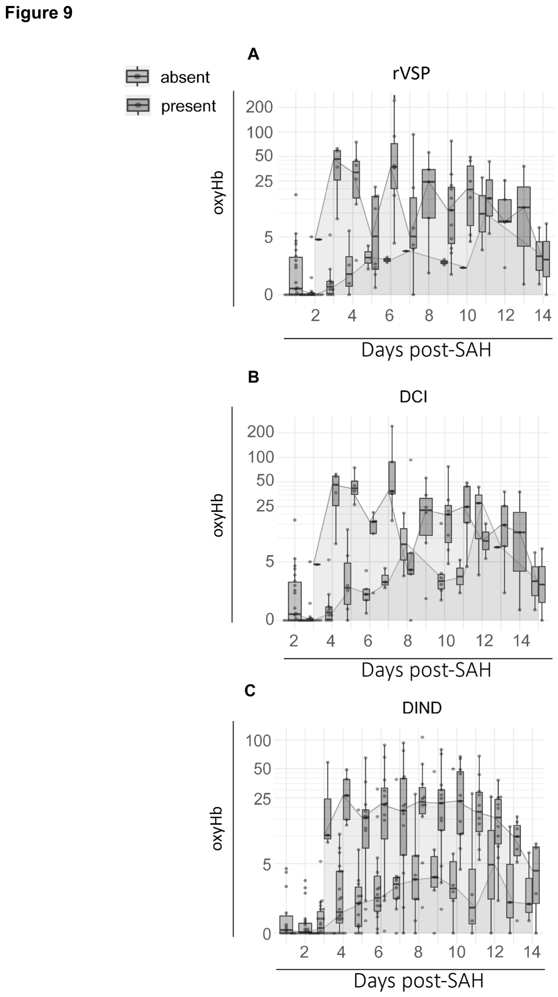

Figure 9 shows temporal profiles of oxyhemoglobin stratified by aVSP, DCI and DIND; A. The temporal course of oxyhemoglobin (oxyHb) stratified by angiographic vasospasm (aVSP). B. The temporal course of oxyHb stratified by delayed cerebral ischemia (DCI). C. The temporal course of oxyHb stratified by delayed ischemic neurological deficits (DIND).

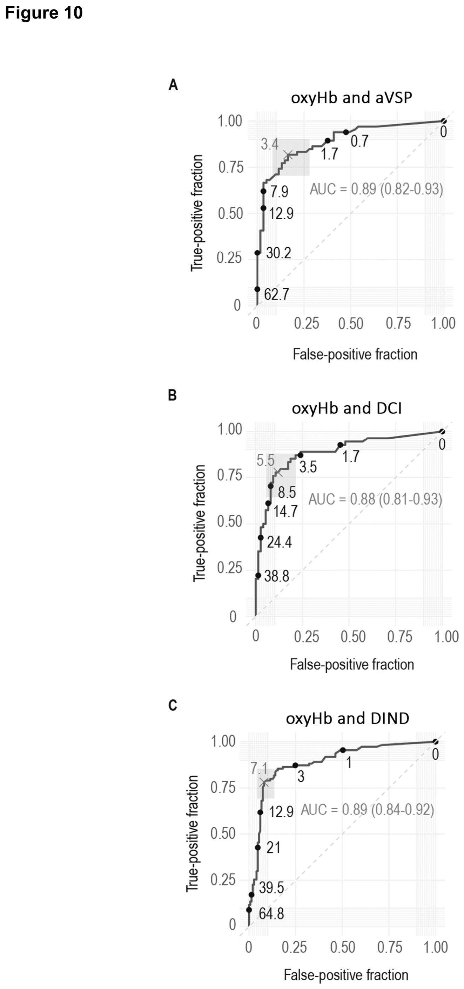

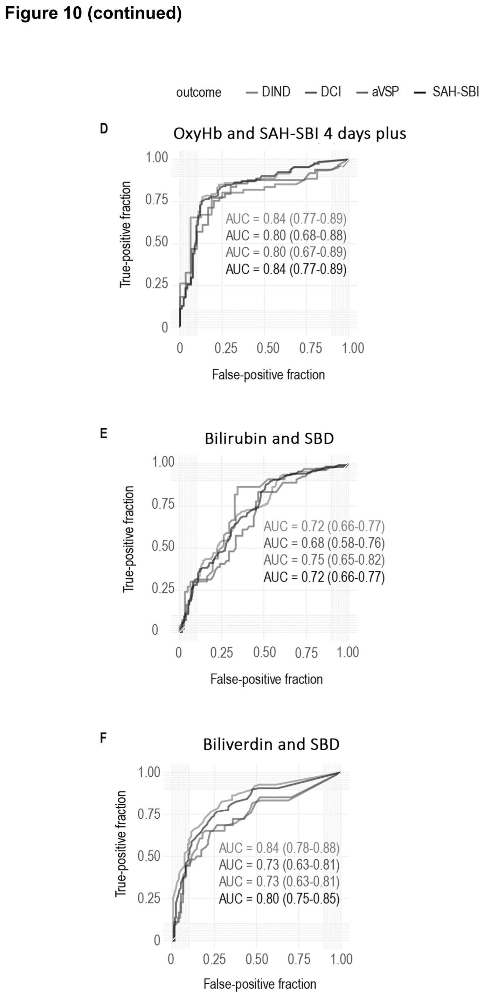

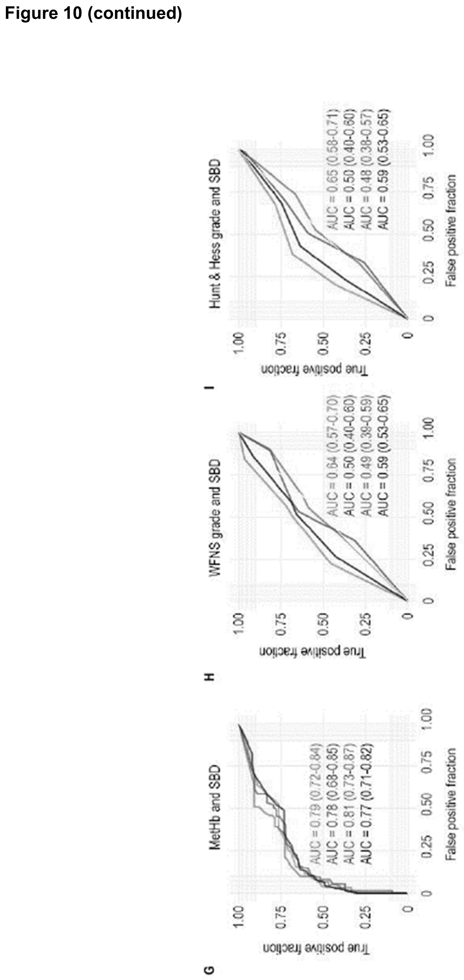

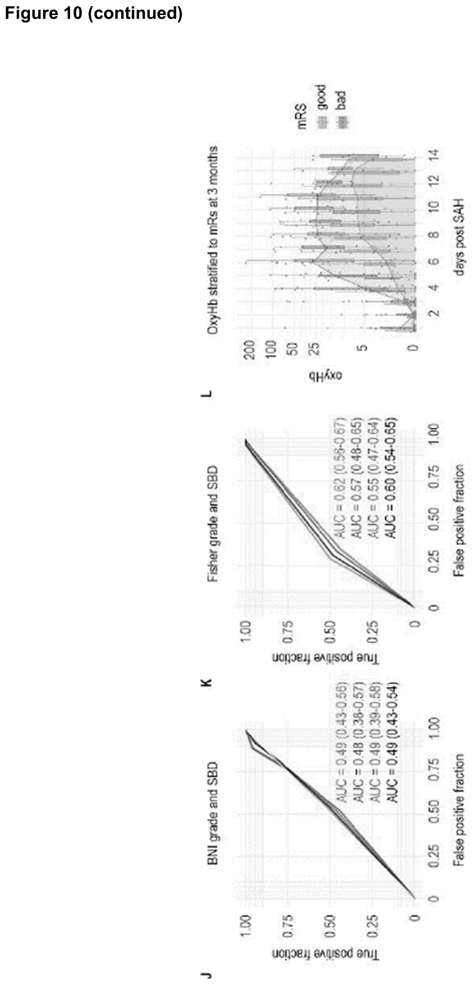

Figure 10 shows the association of cerebrospinal fluid hemoglobin, clinical and radiological scores with secondary brain injury after aneurysmal subarachnoid haemorrhage; A-C. Receiver operating characteristics (ROC) curve and area under the curve (AUC) of CSF-Hb with the respective values for the occurrence of angiographic vasospasm (aVSP, A. ), delayed ischemic neurological deficits (DIND, B.) and delayed ischemic neurological deficits (DIND, C.). The optimal Youden-Index and its 95% confidence interval is indicated in red. D. ROC curves and AUC of CSF-Hb for the occurence of aVSP, DCI, DIND and SAH-SBI with data limited to the high-risk period (day 4-14). E-G. ROC curves and AUC of CSF-Bilirubin (E.), CSF-Biliverdin (F.) and CSF-methemoglobin (metHb) (G.) for the occurence of aVSP, DCI, DIND and SAH-SBI. H-K. ROC curves and AUC of WFNS (H.), Hunt and Hess (I.), BNI (J.) and Fisher (K.) grade for the occurence of aVSP, DCI, DIND and SAH-SBI. L. The temporal course of CSF-Hb stratified by modified Rankin scale (mRS) at 3-months follow-up (good vs. poor).

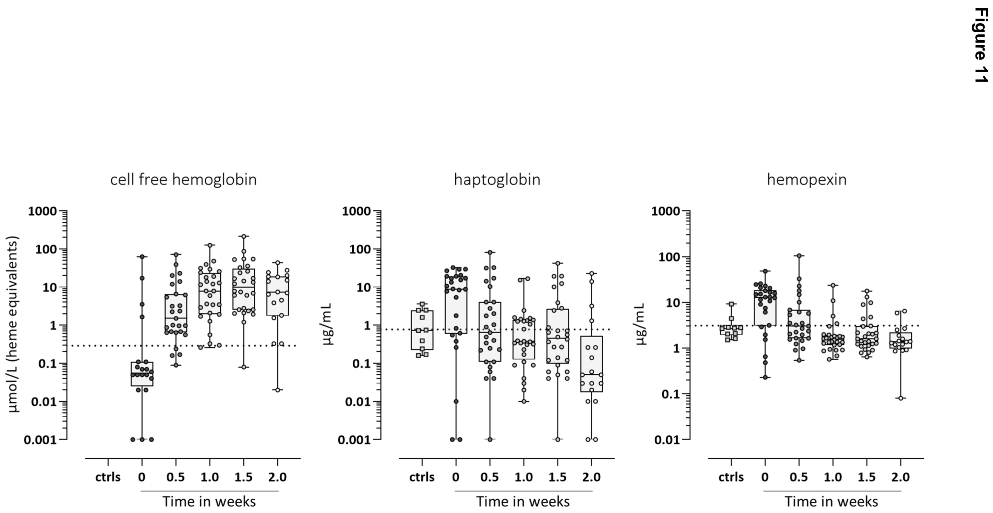

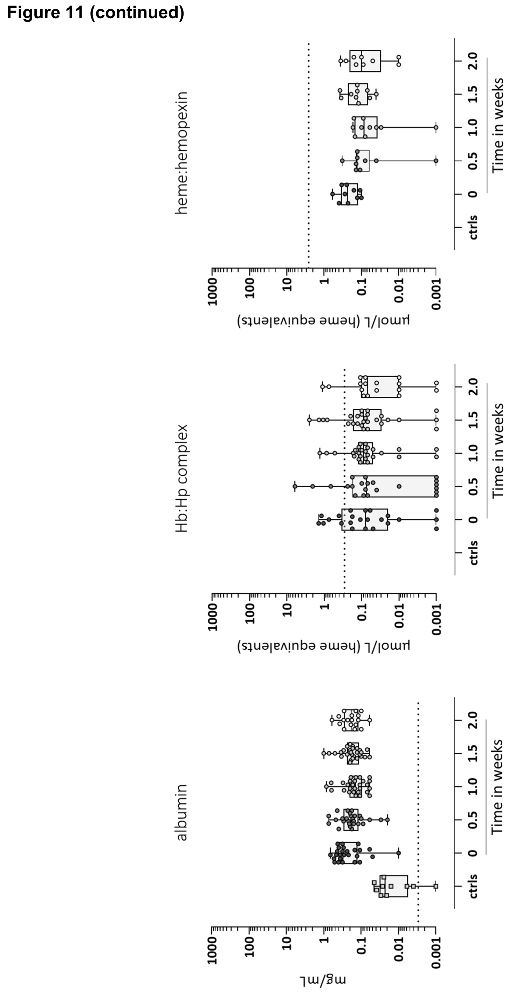

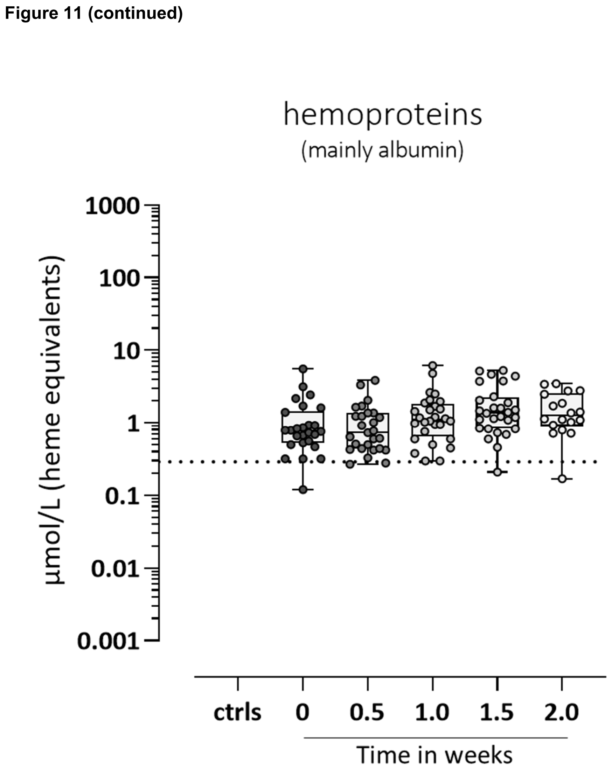

Figure 11 shows quantification of haemoglobin and heme scavenger proteins in patient CSF samples. Upper panel from left to right shows the quantification of cell free Hb, haptoglobin, hemopexin and albumin in patient CSF from collected timepoints as indicated in weeks compared to healthy control samples (ctrls). Lower panel from left to right shows the scavenger proteins complexed with haemoglobin (Hb:Hp complex) or with heme (heme:hemopexin or heme bound proteins; hemoproteins). Data is presented as box and whiskers plots representing means ± Min to Max including individual data points (overlaid). Dashed line indicates the lower limit of quantification (LLOQ) of the corresponding assays.

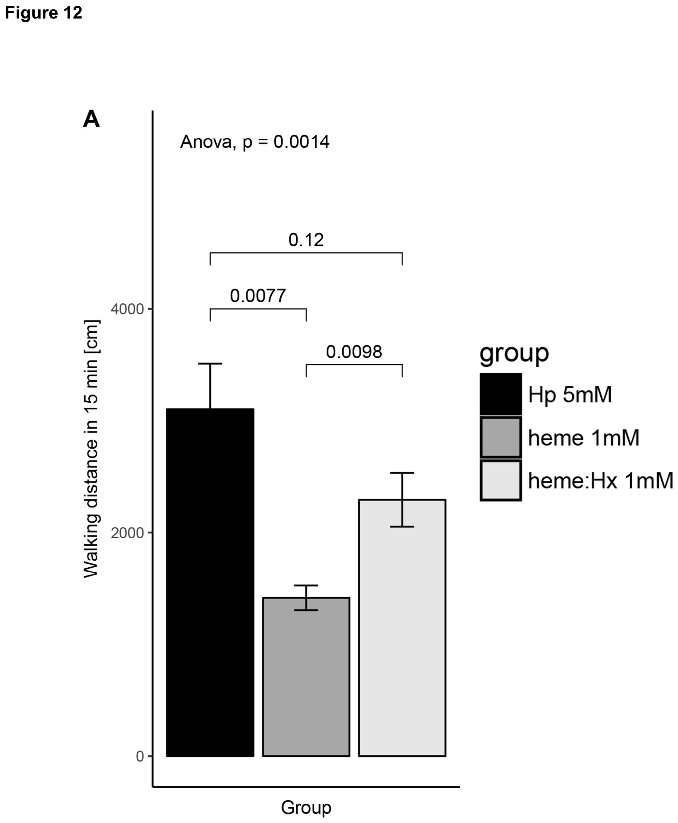

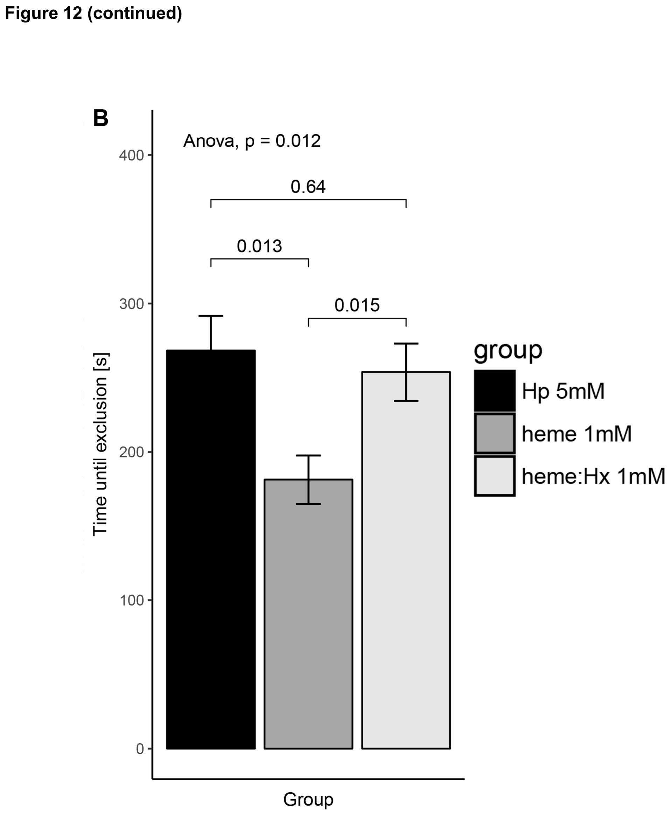

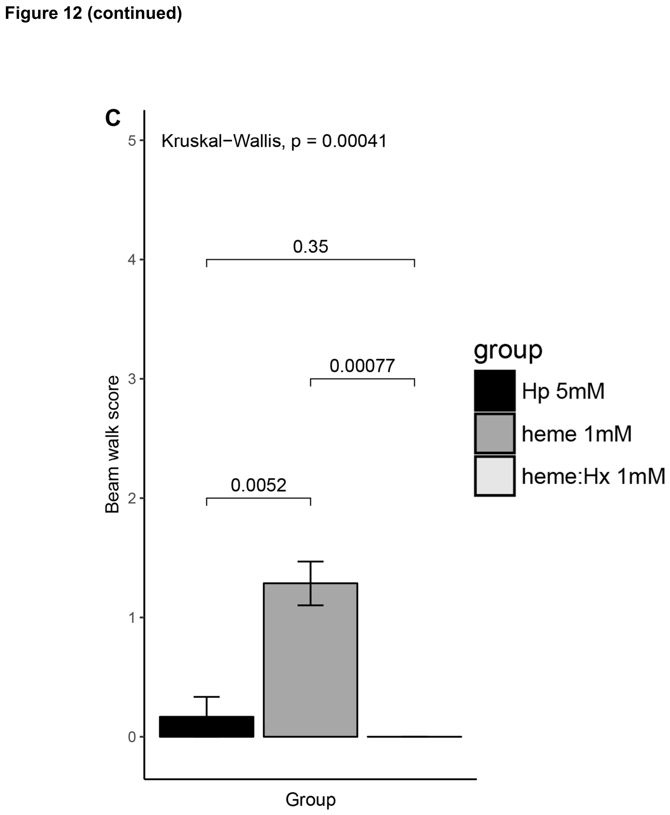

Figure 12 shows neurological function 24 hours after striatal injection of haptoglobin, heme or heme:hemopexin. Scoring of the open-field testing (A), rotarod testing (B) and beamwalk testing (C) after injection of 10 µL haptoglobin (5mM Hp, protein control) (black), heme 1mM (dark gray) or heme:Hx 1mM (light gray), respectively (n=6 per group). Mice after heme, but not heme:Hx injection show impaired neurobehavioral function after 24 hours.

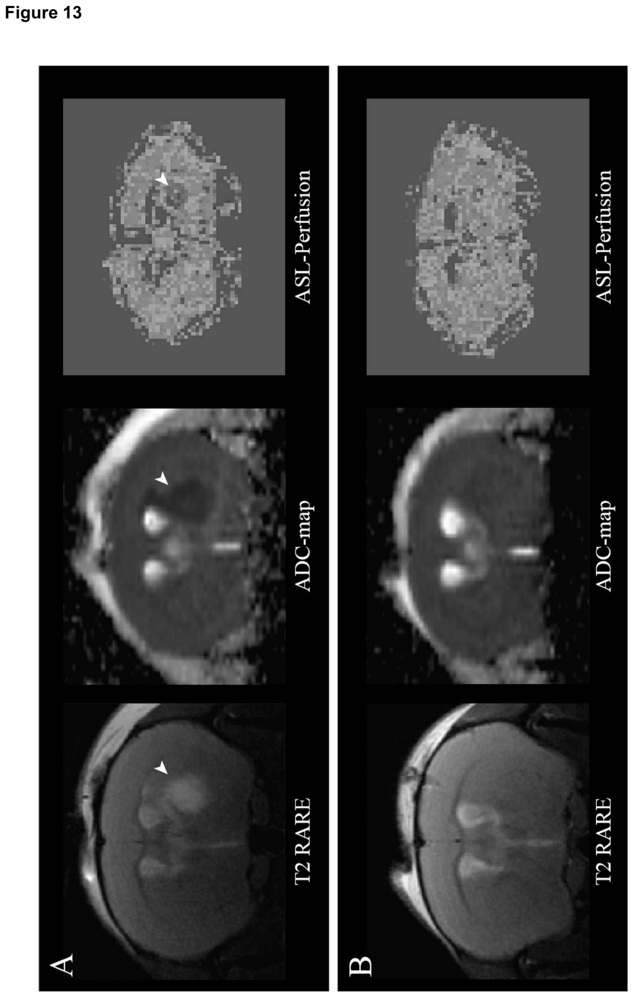

Figure 13 shows radiological changes 24 hours after striatal injection of heme or heme:hemopexin. Illustrative examples for the radiological changes seen 24 hours after striatal injection of heme (A) or heme:Hpx (B), respectively. After injection of heme, perilesional edema is noted on the T2-weighted sequence (left), accompanied by a diffusion restriction in the ADC map (middle) and a focal perfusion deficit on arterial spine labelling (ASL) images (right). After injection of heme:Hpx, only minor radiological changes are seen on all three sequences.

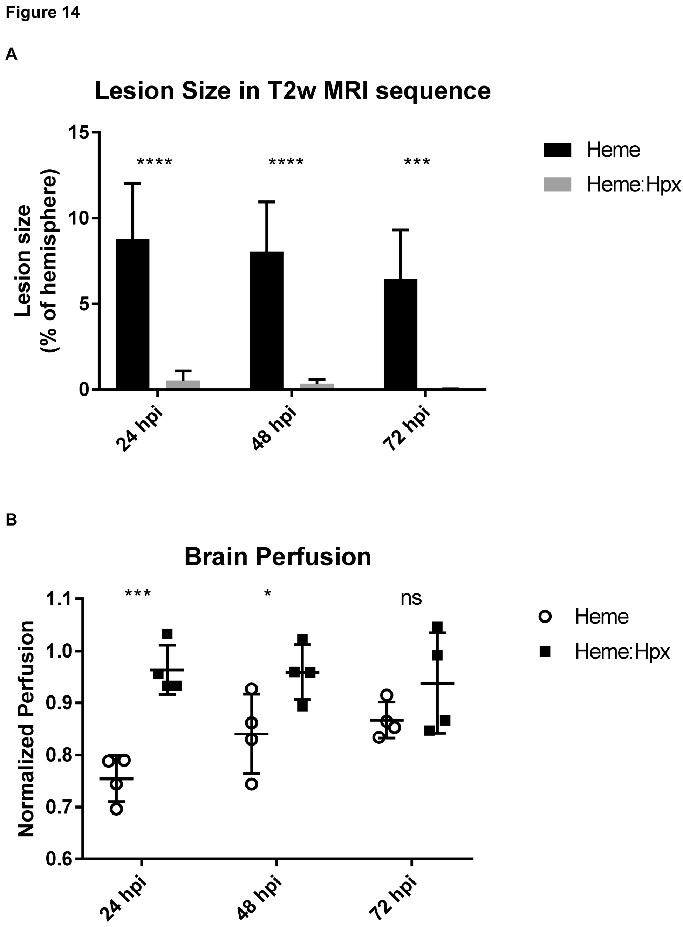

Figure 14 shows quantification of edema size and perfusion 24, 48 and 72 hours after striatal injection of heme or heme:hemopexin. (A.) Semi-automated quantification of the edema size on the T2-weighted images 24, 48 and 72 hours after injection of heme (black) and heme:Hpx (dark gray), respectively. ns: p > 0.05; *: p ≤0.05, **: p ≤ 0.01; ***: p ≤ 0.001, ****: p ≤ 0.0001. (B.) Normalized perfusion on arterial spine labelling images 24, 48 and 72 hours after injection of heme (circles) and heme:Hpx (squares) (n=4 per group). The striatal injection of heme is associated with the formation of a cerebral edema and a regionally decreased perfusion, which is absent after injection of heme:Hpx.

### DETAILED DESCRIPTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

It must be noted that, as used in the subject specification, the singular forms "a", "an" and "the" include plural aspects unless the context clearly dictates otherwise. Thus, for example, reference to "an agent " includes a single resin, as well as two or more agents; reference to "the composition" includes a single composition, as well as two or more compositions; and so forth.

In the absence of any indication to the contrary, reference made to a "%" content throughout this specification is to be taken as meaning % w/w (weight/weight). For example, a solution comprising a haptoglobin content of at least 80% of total protein is taken to mean a composition comprising a haptoglobin content of at least 80% w/w of total protein.

The present invention is predicated, at least in part, on the inventors' surprising finding that hemopexin (Hx) can reduce or otherwise prevent cell-free heme-mediated adverse secondary neurological outcomes, such as cerebral vasospasm, *in vivo.* Thus, in an aspect disclosed herein, there is provided a method of treating or preventing an adverse secondary neurological outcome in a subject following a haemorrhagic stroke accompanied by extravascular erythrolysis and release of cell-free heme and/or cell-free haemoglobin (Hb) into a cerebral spinal fluid (CSF), the method comprising exposing the CSF of a subject in need thereof to a therapeutically effective amount of hemopexin (Hx) and for a period of time sufficient to allow the Hx to form a complex with, and thereby neutralise, the cell-free heme.

### Haemorrhagic stroke

Haemorrhagic stroke, or bleeding, into the CSF compartment, is also referred to interchangeably herein as a brain haemorrhage, a cerebral haemorrhage or an intracranial haemorrhage. It is typically characterised by a ruptured blood vessel in the brain causing localized bleeding. The location of the bleed can vary. For example, haemorrhage into the CSF compartment may result from an intraventricular haemorrhage, an intraparenchymal haemorrhage, and / or a subarachnoid haemorrhage.

Haemorrhagic stroke is made up of a range of pathologies with different natural courses, assessment, and management, as will be familiar to persons skilled in the art. It is generally categorized as *primary* or *secondary,* depending on aetiology.

In an embodiment, the haemorrhagic stroke is an intraventricular haemorrhage (IVH) or a subarachnoid haemorrhage (SAH). In an embodiment, the haemorrhagic stroke is an aneurysmal subarachnoid haemorrhage (aSAH).

Methods of diagnosing a haemorrhagic stroke, and in particular SAH, in a subject will be familiar to persons skilled in the art, illustrative examples of which include cerebral angiography, computerised tomography (CT) and spectrophotometric analysis of oxyHb and bilirubin in the subject's CSF (see, for example, Cruickshank AM., 2001, ACP Best Practice No 166, J. Clin. Path., 54(11):827-830).

It will be understood by persons skilled in the art that the haemorrhagic stroke can be a spontaneous haemorrhage (*e.g*., as a result of a ruptured aneurysm) or a traumatic haemorrhage (e.g., as a result of a trauma to the head). In an embodiment, the haemorrhagic stroke is a spontaneous haemorrhage, also known as a *non-traumatic* haemorrhage. In an embodiment, the haemorrhagic stroke is a traumatic haemorrhage.

The term "cerebrospinal fluid", or CSF, is understood to mean the fluid within the brain ventricles and the cranial and spinal subarachnoid spaces. The brain ventricles, cranial and spinal subarachnoid spaces are collectively referred to herein as the "CSF compartment". Thus, in an embodiment disclosed herein, the method comprises exposing the CSF compartment of the subject in need thereof to a therapeutically effective amount of Hp.

CSF is predominantly, but not exclusively, secreted by the choroid plexuses, which consist of granular meningeal protrusions into the ventricular lumen, the epithelial surface of which is continuous with the ependyma. Studies have also suggested that brain interstitial fluid, ependyma and capillaries may also play a role in CSF secretion. The CSF volume is estimated to be about 150 mL in human adults, with a typically distribution of between 125 mL in cranial and spinal subarachnoid spaces and 25 mL in the ventricles, albeit with marked variation between individuals. CSF secretion in human adults can varies between 400 to 600 mL per day, with about 60-75% of CSF produced by the choroid plexuses of the lateral ventricles and the tela choroidea of the third and fourth ventricles. Choroidal secretion of CSF typically comprises two steps: (i) passive filtration of plasma from choroidal capillaries to the choroidal interstitial compartment according to a pressure gradient and (ii) active transport from the interstitial compartment to the ventricular lumen across the choroidal epithelium, involving carbonic anhydrase and membrane ion carrier proteins. CSF plays an essential role in homeostasis of cerebral interstitial fluid and the neuronal environment by regulation of the electrolyte balance, circulation of active molecules, and elimination of catabolites. CSF transports the choroidal plexus secretion products to their sites of action, thereby modulating the activity of certain regions of the brain by impregnation, while synaptic transmission produces more rapid changes of activities. The wastes of brain metabolism, peroxidation products and glycosylated proteins, accumulate with age-related decreased CSF turnover (Sakka et al., 2011, European Annals of Otorhinolaryngology, Head and Neck Diseases, 128(6):309-316).

### Subarachnoid hemorrhage related secondary brain injury (SAH-SBI)

Besides early brain injury within the first 72 hours, aSAH patient outcomes may be determined by delayed secondary brain injury, which typically occurs between day 4 to 10 after aneurysm rupture. Delayed secondary brain injury is assumed to be multifactorial involving macro- and microvascular dysfunction, neuroinflammation, neuronal apoptosis, and pathological electrical activity of the brain. About two-thirds of patients after aSAH will develop angiographic vasospasms of large cerebral arteries (aVSP). Delayed cerebral ischemia (DCI) with radiologic demarcation of ischemic brain areas and clinically evident delayed ischemic neurologic deficits (DIND) are often found in about one-third of patients after aSAH. The occurrence of at least one of these secondary manifestations defines subarachnoid hemorrhage related secondary brain injury in the studies described herein (SAH-SBI).

### Adverse secondary neurological outcome

Patients who survive a haemorrhagic stroke, such as SAH, are at significant risk of developing one or more adverse secondary neurological outcomes or complications. The term "adverse secondary neurological outcome", as used herein, refers to an adverse neurological event (secondary injury to brain tissue) that follows a haemorrhagic stroke. Secondary injury after haemorrhagic stroke may be caused by a cascade of events initiated by the primary injury (*e.g.*, mass effect and physical disruption), by the physiological response to the haematoma (*e.g.* inflammation), and / or by the release of blood and blood components. Adverse secondary neurological outcomes will be familiar to persons skilled in the art, illustrative examples of which include delayed ischaemic neurological deficit (DIND), delayed cerebral ischaemia (DCI), neurotoxicity, apoptosis, inflammation, nitric oxide depletion, oxidative tissue injury, cerebral vasospasm, cerebral vasoreactivity, oedema and spreading depolarisation (see, for example, Al-Tamimi et al., World Neurosurgery, 73(6):654-667 (2010); Macdonald et al., Neurocrit. Care, 13:416-424 (2010); and Macdonald et al., J. Neurosurg. 99:644-652 (2003)).

The terms "treating", "treatment", "treat" and the like, are used interchangeably herein to mean relieving, minimising, reducing, alleviating, ameliorating or otherwise inhibiting an adverse secondary neurological outcome, including one or more symptoms thereof, as described herein. The terms "treating", "treatment" and the like are also used interchangeably herein to mean preventing an adverse secondary neurological outcome from occurring or delaying the onset or subsequent progression of an adverse secondary neurological outcome in a subject that may be predisposed to, or at risk of, developing an adverse secondary neurological outcome, but has not yet been diagnosed as having it. In that context, the terms "treating", "treatment" and the like are used interchangeably with terms such as "prophylaxis", "prophylactic" and "preventative". It is to be understood, however, that the methods disclosed herein need not completely prevent an adverse secondary neurological outcome from occurring in the subject to be treated. It may be sufficient that the methods disclosed herein merely relieve, reduce, alleviate, ameliorate or otherwise inhibit an adverse secondary neurological outcome in the subject to the extent that there are fewer adverse secondary neurological outcomes and / or less severe adverse secondary neurological outcomes than would otherwise have been observed in the absence of treatment. Thus, the methods described herein may reduce the number and / or severity of adverse secondary neurological outcomes in the subject following haemorrhagic stroke.

It is to be understood that a reference to a subject herein does not imply that the subject has had a haemorrhagic stroke, but also includes a subject that is at risk of a haemorrhagic stroke. In an embodiment, the subject has (*i.e*., is experiencing) a haemorrhagic stroke, or a symptom thereof. In another embodiment, the subject has not had a haemorrhagic stroke at the time of treatment, but is at risk of a haemorrhagic stroke. As an illustrative example, the subject has an aneurysm that has not yet ruptured but is at risk of rupture. In this instance, the subject may undergo surgical intervention to minimise the risk of rupture of the aneurysm (*e.g*., by surgical clipping or endovascular coiling). The methods described herein may therefore suitably be prescribed to the subject as a prophylactic measure to minimise, reduce, abrogate or otherwise inhibit an adverse secondary neurological outcome should the aneurysm rupture prior to, during or subsequent to the surgical intervention. In that context, the methods described herein may be employed as a prophylactic measure prior to, during or subsequent to surgical intervention.

The extent to which the methods disclosed herein provide a subjective, qualitative and / or quantitative reduction in the number and / or severity of adverse secondary neurological outcomes following haemorrhagic stroke may be represented as a percentage reduction, for example, by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% when compared to the number and / or severity of adverse secondary neurological outcomes prior to exposing the CSF to the therapeutically effective amount of Hp.

Suitable methods by which a subjective, qualitative and / or quantitative reduction in the number and / or severity of adverse secondary neurological outcomes can be measured following a haemorrhagic stroke will be familiar to persons skilled in the art and will largely depend on the nature of the adverse secondary neurological outcome to be measured. Illustrative examples are described elsewhere herein.

In an embodiment, the adverse secondary neurological outcome is selected from the group consisting of delayed ischaemic neurological deficit (DIND), delayed cerebral ischaemia (DCI), neurotoxicity, inflammation, nitric oxide depletion, oxidative tissue injury, cerebral vasospasm, reduced cerebrovascular reactivity, oedema and spreading depolarisation.

In an embodiment, the adverse secondary neurological outcome is a delayed ischaemic neurological deficit (DIND). DIND after SAH is a serious and poorly understood syndrome of cerebral ischaemia characterised by increased headache, meningism and / or body temperature, typically followed by a fluctuating decline in consciousness and appearance of focal neurological symptoms. DIND is characteristically defined as deterioration in neurological function seen at least 3 to 4 days post-haemorrhagic ictus. It is also referred to as clinical/symptomatic vasospasm. DIND remains a significant cause of morbidity and mortality in survivors of the initial haemorrhage. The reported prevalence of DIND is about 20% to 35%, although in those with a higher blood load, this may be as high as 40%. DIND has been attributed to cerebral infarcts in approximately 20% of patients and to about 13% of all death and disability after aSAH. Suitable methods of determining DIND will be familiar to persons skilled in the art, illustrative examples of which are described in Dreier et al., Brain, 2006; 129(12): 3224-3237, the contents of which are incorporated herein by reference in their entirety. In an embodiment, DIND is determined by spreading mass depolarization, as evidence, for example, by spreading negative slow voltage variations by electrocorticography. In an embodiment, DIND is associated with a delayed decrease of consciousness by at least two GCS levels and / or a new focal neurological deficit.

In an embodiment, the adverse secondary neurological outcome is a cerebral vasospasm. Cerebral vasospasm, or CV (also referred to as "angiographic cerebral vasospasm"), is one of the most common causes of focal ischaemia after a haemorrhagic stroke and can account for up to about 23% of SAH-related disability and death. CV is typically characterised by narrowing of the blood vessels caused by persistent contraction of blood vessels, in particular of the large capacitance arteries at the base of the brain (*i.e*., the cerebral arteries) following a haemorrhagic stroke into the subarachnoid space. The term "vasospasm" is therefore typically used with reference to angiographically determined arterial narrowing. The persistent contraction of blood vessels reduces perfusion of distal brain regions and increased cerebral vascular resistance. Left untreated, CV can ultimately lead to neurotoxicity (brain cell damage) in the form of cerebral ischaemia and infarction, primarily due to the restricted blood supply to brain tissue. CV can be detected by any suitable means known to persons skilled in the art, illustrative examples of which include digital subtraction angiography (DSA), computed tomography (CT) angiography (CTA), magnetic resonance (MR) angiography (MRA), Transcranial Doppler ultrasonography and catheter (cerebral) angiography (CA). In an embodiment, CV is detected by digital subtraction angiography (DSA). Without being bound by theory or a particular mode of application, vasospasm of the cerebral arteries will typically begin about 3 days after SAH, peak at about 7 to 8 days later and resolve by about 14 days (see, *e.g*., Weir et al., J. Neurosurg., 48:173-178 (1978)), with some degree of angiographic narrowing occurring in at least two-thirds of patients having angiography between 4 and 12 days after SAH.

The incidence of CV depends on the time interval after the SAH. As noted elsewhere herein, peak incidence typically occurs about 7-8 days after SAH (range, 3-12 days). In addition to the time after the SAH, other principal factors that affect the prevalence of vasospasm are the volume, density, temporal persistence and distribution of subarachnoid blood. Prognostic factors for CV may include the amount of subarachnoid blood on CT scan, hypertension, anatomical and systemic factors, clinical grade and whether the patient is receiving antifibrinolytics.

Symptoms of CV typically develop sub-acutely and may fluctuate and can include excess sleepiness, lethargy, stupor, hemiparesis or hemiplegia, aboulia, language disturbances, visual fields deficits, gaze impairment, and cranial nerve palsies. Although some symptoms are localized, they are generally not diagnostic of any specific pathological process. Cerebral angiography is typically employed as the gold standard for visualizing and studying cerebral arteries, although Transcranial Doppler ultrasonography can also be used.

In an embodiment, the adverse secondary neurological outcome is delayed cerebral ischaemia (DCI). DCI typically occurs in around a third of patients with aSAH and causes death or permanent disability in half of these patients (Dorsch and King, Journal of Clinical Neuroscience, 1:19-26 (1994)). DCI is typically defined as radiologically detected infarction of the brain without other identifiable reason (*e.g*. post-surgical intervention) in patients with aSAH.

As reported by Vergouwen et al. (Stroke. 2010;41:2391-2395), uniform definitions of "clinical deterioration caused by delayed cerebral ischemia" and "cerebral infarction" should capture the most relevant elements in terms of morphological and clinical characteristics, without assumptions about its pathogenesis. Because cerebral infarction on CT/MRI is strongly correlated with functional outcome 3 months after SAH, and given its expected high interobserver agreement rate, its ability to detect DCI in sedated and comatose patients, and its objective quantification of the consequences of DCI, cerebral infarction on neuroimaging might be a better outcome measure than clinical deterioration caused by DCI alone. Although previous definitions of DCI often combined clinical features of DCI with either angiography/transcranial Doppler findings or cerebral infarction on neuroimaging or autopsy, the authors suggest that these should be separately reported. They also suggest that clinical deterioration caused by DCI should be not more than a secondary measure of outcome, because of suspected lower interobserver agreement rates. According to Vergouwen *et al.,* the proposed definition of clinical deterioration caused by DCI is: "The occurrence of focal neurological impairment (such as hemiparesis, aphasia, apraxia, hemianopia, or neglect), or a decrease of at least 2 points on the Glasgow Coma Scale (either on the total score or on one of its individual components [eye, motor on either side, verbal]). This should last for at least 1 hour, is not apparent immediately after aneurysm occlusion, and cannot be attributed to other causes by means of clinical assessment, CT or MRI scanning of the brain, and appropriate laboratory studies."

Adverse secondary neurological outcomes following haemorrhagic stroke, including SAH, have also been shown to be associated with inflammation, including immune cell activation and / or infiltration into the CSF compartment and the release of inflammatory cytokines. As discussed by Miller et al. (Biomed Res Int. 2014; 2014: 384342), studies have shown that inflammation is a direct mediator of neurological injury after SAH and a causative factor of post-SAH vasospasm. Key inflammatory molecules implicated in the pathophysiology of SAH will be familiar to persons skilled in the art, illustrative examples of which include selectins (L-selectin and P-selectin), integrins (*e.g*., lymphocyte function-associated antigen 1 (LFA-1) and Mac-1 integrin (CD11b/CD18)), TNFα, monocyte chemoattractant protein 1 (MCP-1), Intercellular Adhesion Molecule 1 (ICAM-1), pro-inflammatory interleukins (*e.g.*, IL-1, IL-6, IL-1B, IL-8) and endothelin 1 (ET-1). In an embodiment disclosed herein, an adverse secondary neurological outcome is associated with differential expression of one or more inflammatory markers selected from the group consisting of a selectin (*e.g.*, L-selectin and P-selectin), an integrin (*e.g.*, lymphocyte function-associated antigen 1 (LFA-1) and Mac-1 integrin (CD11b/CD18)), TNFα, monocyte chemoattractant protein 1 (MCP-1), Intercellular Adhesion Molecule 1 (ICAM-1), a pro-inflammatory interleukin and endothelin 1 (ET-1). In an embodiment, the pro-inflammatory interleukin is selected from the group consisting of IL-1, IL-6, IL-1B and IL-8.

Nissen et al. have previously shown that the serum concentration of P-selectin in patients with DIND is significantly higher when compared to patients without DIND (J Neurol Neurosurg Psychiatry 2001;71:329-333). The authors also showed that the serum concentration of L-selectin in patients with DIND is significantly lower when compared to patients without DIND. Thus, in an embodiment, the extent to which the methods described herein reduce the number and / or severity of adverse secondary neurological outcomes following haemorrhagic stroke is determined by a reduction in the concentration of P-selectin in the serum or CSF of the subject, for example, by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% when compared to the concentration of P-selectin in the subject prior to treatment. In another embodiment, the extent to which the methods described herein reduce the number and / or severity of adverse secondary neurological outcomes following haemorrhagic stroke is determined by an increase in the concentration of L-selectin in the serum or CSF of the subject, for example, by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% when compared to the concentration of L-selectin in the subject prior to treatment. Methods by which the concentration of P-selectin and L-selectin can be measured will be familiar to persons skilled in the art, illustrative examples of which are described in Nissen et al. (J Neurol Neurosurg Psychiatry 2001;71:329-333), the contents of which are incorporated herein by reference in their entirety.

The level of proinflammatory cytokines IL-1B, IL-6, IL-8, TNFα, and MCP-1, as well as endothelin-1, have also been shown to be elevated in patients following SAH (Miller et al. Biomed Res Int. 2014; 2014: 384342). Thus, in an embodiment disclosed herein, the extent to which the methods described herein reduce the number and / or severity of adverse secondary neurological outcomes following haemorrhagic stroke is determined by a reduction in the concentration of a proinflammatory cytokine in the serum or CSF of the subject, for example, by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% when compared to the concentration of the proinflammatory cytokine in the subject prior to treatment, wherein the proinflammatory molecule is selected from the group consisting of IL-1B, IL-6, IL-8, TNFα, MCP-1 and endothelin-1. Methods by which the concentration of inflammatory mediators, as described herein, can be measured will be familiar to persons skilled in the art.

Nitric oxide (NO) depletion in CSF has also been shown to contribute to the pathogenesis of adverse secondary neurological outcomes following haemorrhagic stroke (see Pluta et al. JAMA. 2005;293(12):1477-1484). NO levels are decreased in CSF after SAH due to (1) toxicity of oxyhemoglobin to neurons containing neuronal nitric oxide synthase (NOS) in the adventitia of the artery; (2) endogenous inhibition of endothelial NOS; and (3) scavenging of nitric oxide by oxyhemoglobin released from the subarachnoid clot. Thus, in an embodiment disclosed herein, the extent to which the methods described herein reduce the number and / or severity of adverse secondary neurological outcomes following haemorrhagic stroke is determined by an increase in the concentration of NO in the CSF of the subject, for example, by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% when compared to the concentration of NO in the CSF of the subject prior to treatment. Methods by which the concentration of NO in CSF can be measured will be familiar to persons skilled in the art, an illustrative example of which is described in Pluta et al. (JAMA. 2005;293(12):1477-1484), the contents of which are incorporated herein by reference in their entirety.

In an embodiment, the adverse secondary neurological outcome is oxidative tissue injury. As noted elsewhere herein, the present inventors have shown for the first time that a therapeutically effective amount of Hx can reduce the oxidative potential of cell-free heme in CSF. Thus, in an embodiment, exposing the CSF of a subject in need thereof to the Hx, as described herein, reduces oxidative tissue injury. Suitable methods for determining oxidative tissue injury will be familiar to persons skilled in the art, illustrative examples of which are described in Katerji et al. (2019; Oxid. Med. Cell Longey; 1279250), including measuring levels of malondialdehyde (MDA), an end-product of lipid peroxidation. In an embodiment, exposing the CSF of a subject in need thereof to the Hx, as described herein, reduces lipid peroxidation.

In some embodiments, therapeutic Hx may prevent penetration of cell-free heme from the CSF compartment into the interstitial space of the brain, thereby inhibiting the toxic effects of cell-free heme on the cerebral vasculature and the brain parenchyma. Thus, in an embodiment, the adverse secondary neurological outcome is an adverse secondary neurological outcome within the brain parenchyma.

### Hemopexin

Hemopexin is a 61-kDa plasma β-1B-glycoprotein composed of a single 439 amino acids long peptide chain, which is formed by two four-bladed β-propeller domains, resembling two thick disks that lock together at a 90° angle and are joined by an interdomain linker peptide. The heme, which is released into the blood as the result of intra- and extra-vascular haemolysis, is bound between the two four-bladed β-propeller domains in a pocket formed by the interdomain linker peptide. Residues His213 and His266 coordinate the heme iron atom giving a stable bis-histidyl complex, similar to haemoglobin.

Hemopexin contains about 20% carbohydrates, including sialic acid, mannose, galactose, and glucosamine. Twelve cysteine residues were found in the protein sequence, probably accounting for six disulphide bridges. Hemopexin represents the primary line of defense against heme toxicity thanks to its ability to bind heme with high affinity (K_{d} <1 pM) and to function as a heme specific carrier from the bloodstream to the liver. It binds heme in an equimolar ratio, but there is no evidence that heme is covalently bound to the protein.

In addition to heme binding, hemopexin preparations have also been reported to possess serine protease activity (Lin et. al., 2016; Molecular Medicine 22:22-31) and several other functions, such as exhibition of anti- and pro-inflammatory activities, inhibition of cellular adhesion and binding of certain divalent metal ions.

Whilst endogenous hemopexin can control the adverse effects of free heme under physiological steady-state conditions, it has little effect in maintaining steady-state heme levels under pathophysiogical conditions, such as those associated with haemolysis, where a high level of heme leads to the depletion of endogenous hemopexin, causing heme-mediated oxidative tissue damage.

It is to be understood that naturally-occurring and recombinant forms of Hx are suitable for the methods described herein, as long as they are capable of forming a complex with cell-free heme and thereby neutralise the biological activity of the cell-free heme. Suitable naturally-occurring forms of Hx will be known to persons skilled in the art, illustrative examples of which are described in UniProtKB P02790. Koch et al. (2002, Clin. Chem. 48: 1377-1382) and Takahashi et al. (PNAS, 1985, 82(1):73-77), the entire contents of which are incorporated herein by reference.

In an embodiment, the Hx comprises, consists or consists essentially of plasma derived Hx. In an embodiment, the Hx is a human Hx. Illustrative examples of human Hx will be familiar to persons skille din the art, an illustrative example of which is described in UniProtKB P02790 and reproduced below:
MARVLGAPVA LGLWSLCWSL AIATPLPPTS AHGNVAEGET KPDPDVTERC SDGWSFDATT LDDNGTMLFF KGEFVWKSHK WDRELISERW KNFPSPVDAA FRQGHNSVFL IKGDKVWVYP PEKKEKGYPK LLQDEFPGIP SPLDAAVECH RGECQAEGVL FFQGDREWFW DLATGTMKER SWPAVGNCSS ALRWLGRYYC FQGNQFLRFD PVRGEVPPRY PRDVRDYFMP CPGRGHGHRN GTGHGNSTHH GPEYMRCSPH LVLSALTSDN HGATYAFSGT HYWRLDTSRD GWHSWPIAHQ WPQGPSAVDA AFSWEEKLYL VQGTQVYVFL TKGGYTLVSG YPKRLEKEVG TPHGIILDSV DAAFICPGSS RLHIMAGRRL WWLDLKSGAQ ATWTELPWPH EKVDGALCME KSLGPNSCSA NGPGLYLIHG PNLYCYSDVE KLNAAKALPQ PQNVTSLLGC TH

In an embodiment, the Hx comprises, consists or consists essentially of a sequence of human Hx as described before.

A variety of protocols for the isolation of Hx from a natural source of Hx- (e.g., blood plasma) will be familiar to persons skilled in the art, illustrative examples of which are described in WO 2014/055552 and WO 2019/030262, the entire contents of which are incorporated herein by reference.

It is to be understood that the term "hemopexin" (Hx), as used herein, includes all phenotypes (including all isoforms) of Hx. The Hx may be homogenous (insofar as it consists essentially of an Hx of the same isoform) or heterogeneous (insofar as it comprises a combination of different Hx isoforms, including human and non-human isoforms of Hx). Suitable methods for determining Hx isoforms that are present in an isolate will be familiar to persons skilled in the art, illustrative examples of which include high performance size exclusion chromatography (HPLC-SEC assay) and Hx ELISA.

The Hx may be a naturally-occurring Hx (*e.g.*, plasma derived) or it may be produced as a recombinant protein. In an embodiment, the Hx is plasma derived. In an embodiment, the Hx comprises, consists or consists essentially of recombinant Hx.

Unless stated otherwise, the term Hx, as used herein, includes functional analogues of native or naturally-occurring Hx. The term "functional analogue" is to be understood to mean an agent that shares substantially the same biological activity of naturally-occurring (native) Hx, insofar as that biological activity is at least the ability of the analogue to form a complex with cell-free heme and thereby neutralise its biological activity. By "substantially the same biological activity" typically means the functional analogue has a binding affinity for cell-free heme that is at least 40% (*e.g.,* 40%, 45%, 50%, 55%, 60%, 65%, 70%, 85%, 90%, 95%, 100%, 105%, 100%, 105%, 110%, 115%, 120%, 125%, 130%, 135%, 140%, 145%, 150%, 155%, 160%, 165% and so on) of the binding affinity of naturally-occurring Hx, including naturally-occurring Hx isoforms (*e.g.*, human and/or non-human isoforms of Hx). Suitable methods for determining whether an agent is a functional analogue of Hx will be familiar to persons skilled in the art, illustrative examples of which include are described elsewhere herein (*e.g,* the ability of the functional analogue to reduce cell-free heme-induced cerebral vasospasms).

In an embodiment disclosed herein, the functional analogue of Hx is a functional fragment of native Hx. A functional fragment of native Hx can be any suitable length, as long as the fragment retains the ability to form a complex with cell-free heme and thereby neutralise its biological activity.

In another embodiment, the functional analogue is a peptide that has a different amino acid sequence to a naturally-occurring (native) Hx molecule (*i.e.*, a comparator). The functional analogue may include a molecule that has an amino acid sequence that differs from the amino acid sequence of the alpha and / or beta chains of native Hx by one or more (*e.g.*, 1, 2, 3, 4, 5, 6, 7, 8, 9 or more) amino acid substitutions, wherein said difference does not, or does not completely, abolish the ability of the analogue to form a complex with cell-free heme and thereby neutralise its biological activity. In some embodiments, the functional analogue comprises amino acid substitutions that enhance the ability of the analogue to form a complex with cell-free heme, as compared to native Hx. In an embodiment, the functional analogue has an amino acid sequence that differs from the amino acid sequence of the alpha and / or beta chain of native Hp by one or more conservative amino acid substitutions. As used herein, the term "conservative amino acid substitution" refers to changing amino acid identity at a given position to replace it with an amino acid of approximately equivalent size, charge and/or polarity. Examples of natural conservative substitutions of amino acids include the following 8 substitution groups (designated by the conventional one-letter code): (1) M, I, L, V; (2) F, Y, W; (3) K, R, (4) A, G; (5) S, T; (6) Q, N; (7) E, D; and (8) C, S.

In an embodiment, the functional analogue has at least 85% sequence identity to an amino acid sequence of the alpha and / or beta chain of native Hx. Reference to "at least 85%" includes 85%, 86%, 87%, 88%, 89%, 90%, 91%, 92%, 93%, 94%, 95%, 96%, 97%, 98%, 99% or 100% sequence identity or similarity, for example, after optimal alignment or best fit analysis. Thus, in an embodiment, the sequence has at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% or preferably 100% sequence identity or sequence homology with the sequences identified herein, for example, after optimal alignment or best fit analysis.

The terms "identity", "similarity", "sequence identity", "sequence similarity", "homology", "sequence homology" and the like, as used herein, mean that at any particular amino acid residue position in an aligned sequence, the amino acid residue is identical between the aligned sequences. The term "similarity" or "sequence similarity" as used herein, indicates that, at any particular position in the aligned sequences, the amino acid residue is of a similar type between the sequences. For example, leucine may be substituted for an isoleucine or valine residue. As noted elsewhere herein, this may be referred to as conservative substitution. In an embodiment, an amino acid sequence may be modified by way of conservative substitution of any of the amino acid residues contained therein, such that the modification has no effect on the binding specificity or functional activity of the modified polypeptide when compared to the unmodified (native) Hx polypeptide.

In some embodiments, sequence identity with respect to a peptide sequence relates to the percentage of amino acid residues in the candidate sequence which are identical with the residues of the corresponding peptide sequence after aligning the sequences and introducing gaps, if necessary, to achieve the maximum percentage homology, and not considering any conservative substitutions as part of the sequence identity. Neither N- or C- terminal extensions, nor insertions shall be construed as reducing sequence identity or homology. Methods and computer programs for performing an alignment of two or more amino acid sequences and determining their sequence identity or homology are well known to persons skilled in the art. For example, the percentage of identity or similarity of two amino acid sequences can be readily calculated using algorithms, for example, BLAST, FASTA, or the Smith-Waterman algorithm.

Techniques for determining an amino acid sequence "similarity" are well known to persons skilled in the art. In general, "similarity" means an exact amino acid to amino acid comparison of two or more peptide sequences or at the appropriate place, where amino acids are identical or possess similar chemical and/or physical properties such as charge or hydrophobicity. A so-termed "percent similarity" then can be determined between the compared peptide sequences. In general, "identity" refers to an exact amino acid to amino acid correspondence of two peptide sequences.

Two or more peptide sequences can also be compared by determining their "percent identity". The percent identity of two sequences may be described as the number of exact matches between two aligned sequences divided by the length of the shorter sequence and multiplied by 100. An approximate alignment for nucleic acid sequences is provided by the local homology algorithm of Smith and Waterman, Advances in Applied Mathematics 2:482-489 (1981). This algorithm can be extended to use with peptide sequences using the scoring matrix developed by Dayhoff (Atlas of Protein Sequences and Structure, M. O. Dayhoff ed., 5 suppl. 3:353-358, National Biomedical Research Foundation, Washington, D.C., USA), and normalized by Gribskov (Nucl. Acids Res. 14(6):6745-6763, 1986). Suitable programs for calculating the percent identity or similarity between sequences are generally known in the art.

Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul et al., (1997, Nucl. Acids Res.25:3389. A detailed discussion of sequence analysis can be found in Unit 19.3 of Ausubel et al. ("Current Protocols in Molecular Biology", John Wiley & Sons Inc, 1994-1998, Chapter 15).

In an embodiment, a functional analogue includes amino acid substitutions and/or other modifications relative to native Hx in order to increase the stability of the analogue or to increase the solubility of the analogue.

The functional analogue may be a naturally-occurring compound / peptide or it may be synthetically produced by chemical synthesis using methods known to persons skilled in the art.

The Hx may suitably be produced as a recombinant protein in a microorganism, which can be isolated and, if desired, further purified. Suitable microorganisms for the production of recombinant Hx will be familiar to persons skilled in the art, illustrative examples of which include bacteria, yeast or fungi, eukaryote cells (*e.g.*, mammalian or an insect cells), or in a recombinant virus vector (*e.g*., adenovirus, poxvirus, herpesvirus, Simliki forest virus, baculovirus, bacteriophage, sindbis virus or sendai virus). Suitable bacteria for producing recombinant peptides will be familiar to persons skilled in the art, illustrative examples of which include *E*. *coli, B.subtilis* or any other bacterium that is capable of expressing the peptide sequences. Illustrative examples of suitable yeast types for producing recombinant peptides include *Saccharomyces cerevisiae, Schizosaccharomyces pombe, Candida, Pichia pastoris* or any other yeast capable of expressing peptides. Corresponding methods are well known in the art. Also methods for isolating and purifying recombinantly produced peptide sequences are well known in the art and include, for example, gel filtration, affinity chromatography and ion exchange chromatography.

To facilitate isolation of a recombinant Hx, as described herein, a fusion polypeptide may be made where the peptide sequence of the Hx, or functional analogue thereof, is translationally fused (covalently linked) to a heterologous polypeptide which enables isolation by affinity chromatography. Illustrative examples of suitable heterologous polypeptides are His-Tag (*e.g.* His₆. 6 histidine residues), GST-Tag (Glutathione-S-transferase) *etc.*

For preparing recombinant Hx, phage libraries and/or peptide libraries are also suitable, for instance, produced by means of combinatorial chemistry or obtained by means of high throughput screening techniques for the most varying structures (see, for example, Display: A Laboratory Manual by Carlos F. Barbas (Editor), *et al.;* and Willats WG Phage display: practicalities and prospects. Plant Mol. Biol. 2002 December; 50(6):837-54).

Illustrative examples of recombinant Hx include the peptides with accession no. NP_005134 (as described by Morishita et al. 2018, Clin. Chim. Acta 487, 84-89) and accession no. P00738.

The Hx may be fused, coupled or otherwise attached to one or more heterologous moieties as part of a fusion protein. The one or more heterologous moieties may improve, enhance or otherwise extend the activity or stability of the Hx. In an embodiment, the Hx, as described herein, is suitably attached to a heterologous moiety for extending the half-life of the Hx *in vivo.* Suitable half-life extending heterologous moieties will be familiar to persons skilled in the art, illustrative examples of which include polyethylene glycol (PEGylation), glycosylated PEG, hydroxyl ethyl starch (HESylation), polysialic acids, elastin-like polypeptides, heparosan polymers and hyaluronic acid. Thus, in an embodiment disclosed herein, heterologous moiety is selected from the group consisting of polyethylene glycol (PEGylation), glycosylated PEG, hydroxyl ethyl starch (HESylation), polysialic acids, elastin-like polypeptides, heparosan polymers and hyaluronic acid. In other embodiments, the heterologous moiety may be a heterologous amino acid sequence fused to the Hx.

Alternatively, or in addition, the heterologous moiety may be chemically conjugated to the Hx, for example, a covalent bond. The half-life extending heterologous moiety can be fused, conjugated or otherwise attached to the Hx by any suitable means known to persons skilled in the art, an illustrative example of which is via a chemical linker. The principle of this conjugation technology has been described in an exemplary manner by Conjuchem LLC (see, *e.g*., US patent No. 7,256,253), the entire contents of which are incorporated herein by reference.

In other embodiments, the heterologous moiety is a half-life enhancing protein (HLEP). Suitable half-life enhancing proteins will be familiar to persons skilled in the art, an illustrative example of which includes albumin or fragments thereof. Thus, in an embodiment, the HLEP is an albumin or a fragment thereof. The N-terminus of the albumin or fragment thereof may be fused to the C-terminus of the alpha and / or beta chains of the Hx. Alternatively, or in addition, the C-terminus of the albumin or fragment thereof may be fused to the N-terminus of the alpha and / or beta chains of the Hx. One or more HLEPs may be fused to the N- or C-terminal part(s) of the alpha and / or beta chains of the Hx provided that they do not abolish the binding of the Hx to cell-free heme. It is to be understood, however, that some reduction in the binding of the Hx to cell-free heme may be acceptable, as long as the Hx component of the fusion protein is still capable of forming a complex with, and thereby neutralise, cell-free heme.

The fusion protein may further comprise a chemical bond or a linker sequence positioned between the Hx and the heterologous moiety. The linker sequence may be a peptidic linker consisting of one or more amino acids, in particular of 1 to 50, preferably 1 to 30, preferably 1 to 20, preferably 1 to 15, preferably 1 to 10, preferably 1 to 5 or more preferably 1 to 3 (*e.g.* 1, 2 or 3) amino acids and which may be equal or different from each other. Preferably, the linker sequence is not present at the corresponding position in the wild-type Hx. Preferred amino acids present in said linker sequence include Gly and Ser. In a preferred embodiment, the linker sequence is substantially non-immunogenic to the subject to be treated in accordance with the methods disclosed herein. By substantially non-immunogenic is meant that the linker sequence will not raise a detectable antibody response to the linker sequence in the subject to which it is administered. Preferred linkers may be comprised of alternating glycine and serine residues. Suitable linkers will be familiar to persons skilled in the art, illustrative examples of which are described in WO2007/090584. In an embodiment, the peptidic linker between the Hx and the heterologous moiety comprises, consists or consists essentially of peptide sequences, which serve as natural interdomain linkers in human proteins. Such peptide sequences in their natural environment may be located close to the protein surface and are accessible to the immune system so that one can assume a natural tolerance against this sequence. Illustrative examples are given in WO 2007/090584. Suitable cleavable linker sequences are described, *e.g*., in WO 2013/120939 A1.

Illustrative examples of suitable HLEP sequences are described *infra.* Likewise disclosed herein are fusions to the exact "N-terminal amino acid" or to the exact "C-terminal amino acid" of the respective HLEP, or fusions to the "N-terminal part" or "C-terminal part" of the respective HLEP, which includes N-terminal deletions of one or more amino acids of the HLEP. The fusion protein may comprise more than one HLEP sequence, *e.g*. two or three HLEP sequences. These multiple HLEP sequences may be fused to the C-terminal part of the alpha and / or beta chains of the Hp in tandem, *e.g.* as successive repeats.

In an embodiment, the heterologous moiety is a half-life extending polypeptide. In an embodiment, the half-life extending polypeptide is selected from the group consisting of albumin, a member of the albumin-family or fragments thereof, solvated random chains with large hydrodynamic volume (e.g. XTEN (see Schellenberger et al. 2009; Nature Biotechnol. 27:1186-1190), homo-amino acid repeats (HAP) or proline-alanine-serine repeats (PAS), afamin, alpha-fetoprotein, Vitamin D binding protein, transferrin or variants or fragments thereof, carboxyl-terminal peptide (CTP) of human chorionic gonadotropin-ß subunit, a polypeptide capable of binding to the neonatal Fc receptor (FcRn), in particular an immunoglobulin constant region and portions thereof, *e.g*. the Fc fragment, polypeptides or lipids capable of binding under physiological conditions to albumin, to a member of the albumin-family or to fragments thereof or to an immunoglobulin constant region or portions thereof. The immunoglobulin constant region or portions thereof is preferably an Fc fragment of immunoglobulin G1 (IgG1), an Fc fragment of immunoglobulin G2 (IgG2) or an Fc fragment of immunoglobulin A (IgA). A half-life enhancing polypeptide, as used herein, may be a full-length half-life-enhancing protein or one or more fragments thereof that are capable of stabilizing or prolonging the therapeutic activity or the biological activity of the Hx, in particular of increasing the *in vivo* half-life of the Hp. Such fragments may be of 10 or more amino acids in length or may include at least about 15, preferably at least about 20, preferably at least about 25, preferably at least about 30, preferably at least about 50, or more preferably at least about 100, or more contiguous amino acids from the HLEP sequence, or may include part or all of specific domains of the respective HLEP, as long as the HLEP fragment provides a functional half-life extension of at least 10%, preferably of at least 20%, or more preferably of at least 25%, compared to the respective Hx in the absence of the HLEP. Methods of determining whether a heterologous moiety provides a functional half-life extension to the Hx (*in vivo* or *in vitro*) will be familiar to persons skilled in the art, illustrative examples of which are described elsewhere herein.

The HLEP portion of the fusion protein, as descried herein, may be a variant of a wild type HELP. The term "variant" includes insertions, deletions and / or substitutions, either conservative or non-conservative, where such changes do not substantially alter the ability of the Hx to form a complex with, and thereby neutralise, cell-free heme. The HLEP may suitably be derived from any vertebrate, especially any mammal, for example human, monkey, cow, sheep, or pig. Non-mammalian HLEPs include, but are not limited to, hen and salmon.

The fusion proteins, as described herein, can be created by in-frame joining of at least two DNA sequences encoding the Hx and the heterologous moity, such as a HLEP. Persons skilled in the art will understand that translation of the fusion protein DNA sequence will result in a single protein sequence. As a result of an in-frame insertion of a DNA sequence encoding a peptidic linker according to an embodiment disclosed herein, a fusion protein comprising the Hp, a suitable linker and the heterologous moirty can be obtained.

In an embodiment disclosed herein, the Hx is fused to a heterologous moiety. In an embodiment, the heterologous moiety comprises, consists or consists essentially of a polypeptide selected from the group consisting of albumin or fragments thereof, transferrin or fragments thereof, the C-terminal peptide of human chorionic gonadotropin, an XTEN sequence, homo-amino acid repeats (HAP), proline-alanine-serine repeats (PAS), afamin, alpha-fetoprotein, Vitamin D binding protein, polypeptides capable of binding under physiological conditions to albumin or to immunoglobulin constant regions, polypeptides capable of binding to the neonatal Fc receptor (FcRn), particularly immunoglobulin constant regions and portions thereof, preferably the Fc portion of immunoglobulin, and combinations of any of the foregoing. In another embodiment, the heterologous moiety is selected from the group consisting of hydroxyethyl starch (HES), polyethylene glycol (PEG), polysialic acids (PSAs), elastin-like polypeptides, heparosan polymers, hyaluronic acid and albumin binding ligands, e.g. fatty acid chains, and combinations of any of the foregoing.

The terms, "human serum albumin" (HSA) and "human albumin" (HA) and "albumin" (ALB) are used interchangeably herein. The terms "albumin" and "serum albumin" are broader, and encompass human serum albumin (and fragments and variants thereof), as well as albumin from other species (and fragments and variants thereof).

As used herein, "albumin" refers collectively to albumin polypeptide or amino acid sequence, or an albumin fragment or variant, having one or more functional activities (e.g., biological activities) of albumin. In particular, "albumin" refers to human albumin or fragments thereof, including the mature form of human albumin or albumin from other vertebrates or fragments thereof, or analogs or variants of these molecules or fragments thereof. In some embodiments disclosed herein, the alternative term "FP" is used to identify the HLEP, in particular to define albumin as the HLEP.

The fusion proteins described herein may suitably comprise naturally-occurring polymorphic variants of human albumin and / or fragments of human albumin. Generally speaking, an albumin fragment or variant will be at least 10, preferably at least 40, or most preferably more than 70 amino acids in length.

In an embodiment, the HLEP is an albumin variant with enhanced binding to the FcRn receptor. Such albumin variants may lead to a longer plasma half-life of the Hx or functional analogue thereof compared to the Hx or functional fragment thereof that is fused to a wild-type albumin. The albumin portion of the fusion proteins described herein may suitably comprise at least one subdomain or domain of human albumin or conservative modifications thereof.

In an embodiment, the heterologous moiety is an immunoglobulin molecule or a functional fragment thereof. Immunoglobulin G (IgG) constant regions (Fc) are known in the art to increase the half-life of therapeutic proteins (see, *e.g*., Dumont J A et al. 2006. BioDrugs 20:151-160). The IgG constant region of the heavy chain consists of 3 domains (CH1-CH3) and a hinge region. The immunoglobulin sequence may be derived from any mammal, or from subclasses IgG1, IgG2, IgG3 or IgG4, respectively. IgG and IgG fragments without an antigen-binding domain may also be used as a heterologous moiety, including as a HLEP. The Hp or functional analogue thereof may suitably be connected to the IgG or the IgG fragments via the hinge region of the antibody or a peptidic linker, which may even be cleavable. Several patents and patent applications describe the fusion of therapeutic proteins to immunoglobulin constant regions to enhance the therapeutic proteins' *in vivo* half-lives. For example, US 2004/0087778 and WO 2005/001025 describe fusion proteins of Fc domains or at least portions of immunoglobulin constant regions with biologically active peptides that increase the half-life of the peptide, which otherwise would be quickly eliminated *in vivo.* Fc-IFN-β fusion proteins were described that achieved enhanced biological activity, prolonged circulating half-life and greater solubility (WO 2006/000448 A2). Fc-EPO proteins with a prolonged serum half-life and increased in vivo potency were disclosed (WO 2005/063808 A1) as well as Fc fusions with G-CSF (WO 2003/076567 A2), glucagon-like peptide-1 (WO 2005/000892 A2), clotting factors (WO 2004/101740 A2) and interleukin-10 (U.S. Pat. No. 6,403,077), all with half-life enhancing properties.

Illustrative examples of suitable HLEP which can be used in accordance with the present invention are also described in WO 2013/120939 A1, the contents of which are incorporated herein by reference in their entirety.

The term "therapeutically effective amount", as used herein, means the amount or concentration of Hx in the CSF that is sufficient to allow the Hx to bind to, and form a complex with, cell-free heme present in the CSF and thereby neutralise the otherwise adverse biological effect of the cell-free heme. It would be understood by persons skilled in the art that the therapeutically effective amount of peptide may vary depending upon several factors, illustrative examples of which include whether the Hx is to be administered directly to the subject (*e.g*, intrathecally, intracranially or intracerebroventricularly), the health and physical condition of the subject to be treated, the taxonomic group of subject to be treated, the severity of the haemorrhage (*e.g*., the extent of bleeding), the route of administration, the concentration and / or amount of cell-free heme in the CSF compartment and combinations of any of the foregoing.

The therapeutically effective amount of Hx will typically fall within a relatively broad range that can be determined by persons skilled in the art. Illustrative examples of a suitable therapeutically effective amounts of Hx include from about 2 µM to about 1 mM, preferably from about 2 µM to about 400 µM, preferably from about 5 µM to about 400 µM, preferably from about 5 µM to about 200 µM, or more preferably from about 10 µM to about 200 µM.

In an embodiment, the therapeutically effective amount of Hx is from about 2 µM to about 1 mMIn an embodiment, the therapeutically effective amount of Hx is from about 2 µM to about 400 µM. In an embodiment, the therapeutically effective amount of Hx is from about 5 µM to about 200 µM. In an embodiment, the therapeutically effective amount of Hx is from about 10 µM to about 200 µM.

In an embodiment, the therapeutically effective amount of Hx is at least an equimolar amount to the concentration of cell-free heme in the CSF of the subject following the haemorrhage. In another embodiment, the therapeutically effective amount of Hx is an amount sufficient to complex from about 3 µM to about 1,200 µM cell-free Hb in CSF. In an embodiment, the therapeutically effective amount of Hx is at least four-times the amount of cell-free hemaglobin (Hb) in the CSF of the subject following the haemorrhage. Suitable methods of measuring the concentration of cell-free Hb and heme in CSF will be known to persons skilled in the art, illustrative examples of which are described in Nyakundi et al. (2020, Oxid Med Cell Longev, 2020, 8929020), Righy et al. (2018, Rev Bras Ter Intestiva, 30(1):21-27), Cruickshank AM. (2001, ACP Best Practice No 166, J. Clin. Path., 54(11):827-830), Hugelshofer M. et al. (2018. World Neurosurg.;120:e660-e666) and Hugelshofer M. et al. (J Clin Invest. 2019; 129(12):5219-5235), the contents of which are incorporated herein by reference in their entirety.

It is to be understood that achieving a therapeutically effective amount of Hx, as herein described, may depend on the final volume of the CSF to which the Hx, or functional analogue thereof, is exposed. For example, where the Hx is to administered to an adult human subject (*e.g.,* intrathecally), and taking into account that the average volume of CSF in an adult human subject is about 150 mL, a therapeutically effective amount of about 10 µM Hx can be achieved by administering to the subject a 5mL solution of about 310 µM Hx. In another illustrative example, the methods described herein comprise removing 50mL of CSF from the subject and replacing it with 50mL of artificial CSF comprising about 30 µM Hx, thereby achieving a therapeutically effective amount of about 10 µM Hx in the CSF compartment of the subject.

Dosages of Hx may also be adjusted to provide the optimum therapeutic response. For example, several divided doses may be administered daily, weekly, or other suitable time intervals, or the dosages may be proportionally reduced as indicated by the exigencies of the situation.

The term "exposing", as used herein, means bringing into contact the CSF with the Hx in such a way as to allow the Hx to bind to and form a complex with cell-free heme that is present in the CSF, whereby the formation of heme:Hx complexes substantially neutralises the otherwise adverse biological effect of cell-free heme on brain tissue. By "substantially neutralise" is meant a reduction to the adverse biological effect of cell-free heme on brain tissue, as represented subjectively or qualitatively as a percentage reduction by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% comparted to the biological effect of cell-free heme on brain tissue in the absence of therapeutic Hx, including by at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%. Methods by which a reduction to the adverse biological effect of cell-free Hx can be measured or determined (qualitatively or quantitatively) will be familiar to persons skilled in the art, illustrative examples of which are described elsewhere herein.

As also noted herein, the present inventors have also shown, for the first time, that a therapeutically effective amount of Hx can prevent reduce the oxidative potential of cell-free heme in CSF as assessed by malondialdehyde with the thiobarbituric acid reactive substances (TBARS) assay.

It is to be understood that the manner in which the CSF of a subject in need thereof is exposed to a therapeutically effective amount of Hx, as described herein, may vary, depending, for example, on whether said exposure is to be performed within the CSF compartment of a subject in need thereof (*i.e., in vivo*) or extracorporeally in CSF obtained from the subject (*i.e*, *ex vivo*)*.* Where said exposure is to be performed within the CSF compartment of a subject in need thereof (*i.e., in vivo*)*,* the route of administration of the Hx will be selected to allow the Hx to contact cell-free heme within the CSF compartment. Suitable routes of administration will be familiar to persons skilled in the art, illustrative examples of which include intrathecal, intracranial and intracerebroventricular. In an embodiment, the therapeutically effective amount of Hx is administered via an external ventricular drain that is placed, for example, in the subject after a haemorrhagic stroke to temporarily drain the CSF and decrease intracranial pressure.

In an embodiment, the Hx is administered intracranially to the subject.

In an embodiment, the Hx is administered intrathecally to the subject.

In an embodiment, the Hx is administered intrathecally into the spinal canal.

In an embodiment, the Hx is administered intrathecally into the subarachnoid space.

In an embodiment, the Hx is administered intracerebroventricularly to the subject.

In an embodiment, the method comprises intrathecally administering to the subject the therapeutically effective amount of the Hx. In an embodiment, the method comprises intrathecally administering to the subject the therapeutically effective amount of the Hx into the spinal canal. In an embodiment, the method comprises intrathecally administering to the subject the therapeutically effective amount of the Hx into the subarachnoid space. In an embodiment, the method comprises intracerebroventricularly administering to the subject the therapeutically effective amount of the Hx.

Alternatively, or in addition, the methods described herein comprise removing CSF from a subject in need thereof, exposing the CSF to a therapeutically effective amount of Hx for a period of time sufficient to allow the Hx to form a complex with, and thereby neutralise, the cell-free heme in the CSF, removing the heme:Hx complexes thus formed in the CSF to produce an heme-diminished CSF and administering the heme-diminished CSF to the subject (e.g, intrathecally, or intracerebroventricularly). It will be understood that removing CSF from the CSF compartment will typically not result in a CSF compartment that is entirely free of CSF, noting that at least some CSF will remain in the CSF compartment. Optionally, the CSF compartment can be rinsed with a pharmaceutically acceptable wash solution once CSF has been removed in order to remove at least some of the residual Hx that may be present in the CSF compartment. The wash solution may optionally comprise Hx, to further complex and thereby neutralise at least some of the residual cell-free heme that may be present in the CSF compartment. In an embodiment, the wash solution is an artificial CSF, as described elsewhere herein.

In an embodiment, the method described herein comprises removing a sample of CSF from the CSF compartment of a subject in need thereof, adding Hx to the CSF sample to obtain an Hx-enriched CSF sample, administering the Hx-enriched CSF sample to the CSF compartment of the subject, thereby exposing the CSF compartment to a therapeutically effective amount of the Hx and for a period of time sufficient to allow the Hx to form a complex with, and thereby neutralise, cell-free heme in the CSF of the subject, and optionally, repeating the above steps. Preferably, the amount of Hx that is added to the CSF sample will be determined such that, upon administration to the subject, will provide a therapeutically effective amount of Hx within the CSF of the subject. Hence, the amount of Hx to be added in the CSF sample will depend on the volume of CSF sample that is removed and re-administered to the subject.

In an embodiment, the method comprises:
(i) obtaining sample of CSF from the CSF compartment of the subject following the haemorrhage;
(ii) adding to the CSF sample of step (i) Hx to obtain an Hx-enriched CSF sample;
(iii) administering the Hx-enriched CSF sample to the subject, thereby exposing the CSF compartment of the subject to a therapeutically effective amount of Hx for a period of time sufficient to allow the Hx to form a complex with cell-free heme in the CSF compartment of the subject; and
(iv) optionally repeating steps (i) to (iii).

The volume of CSF that is to be removed and re-administered to the subject will desirably be substantially the same. For example, if a 50mL sample of CSF is removed from the CSF compartment of the subject, the entire 50mL volume of CSF comprising the Hx will be re-administered to the subject. However, it will be understood that the volumes may be dissimilar, as long as any difference in volumes does not give rise to significant adverse clinical outcomes. In some embodiments, the volume of CSF that is re-administered to the subject will be less than the volume of CSF that was removed from the subject. In other embodiments, the volume of CSF that is re-administered to the subject will be greater than the volume of CSF that was removed from the subject, with the addition of Hx, alone or in combination with any other therapeutical agents, making up the extra volume.

In another embodiment, the method described herein comprises removing a volume of CSF from the CSF compartment of a subject in need thereof, replacing the volume of CSF removed from the subject with a volume of artificial CSF comprising a Hx, thereby exposing the CSF of the subject to a therapeutically effective amount of the Hx and for a period of time sufficient to allow the Hx to form a complex with, and thereby neutralise, cell-free heme in the CSF of the subject. Preferably, the amount of Hx in the artificial CSF will be determined such that, upon administration to the subject, will provide a therapeutically effective amount of Hx within the CSF of the subject. Hence, the amount of Hx to be added to the artificial CSF will depend on the volume of artificial CSF that will be administered to the subject.

In an embodiment, the method comprises:
(i) removing a volume of CSF from the CSF compartment of the subject following the haemorrhage;
(ii) providing an artificial CSF comprising Hx;
(iii) administering the artificial CSF of (ii) to the subject, thereby exposing the CSF compartment of the subject to a therapeutically effective amount of Hx for a period of time sufficient to allow the Hx to form a complex with cell-free heme in the CSF compartment of the subject; and
(iv) optionally repeating steps (i) to (iii).

The volume of artificial CSF that is administered to the subject will desirably be substantially the same as the volume of CSF removed from the subject. For example, if a 50mL sample of CSF is removed from the CSF compartment of the subject, a volume of about 50mL of artificial CSF comprising the therapeutically effective amount of Hx will be used to replace the volume of CSF removed. However, it will be understood that the volumes may be dissimilar, as long as any difference in volumes does not give rise to significant adverse clinical outcomes. In some embodiments, the volume of artificial CSF that is administered to the subject will be less than the volume of CSF that was removed from the subject. In other embodiments, the volume of artificial CSF that is administered to the subject will be greater than the volume of CSF that was removed from the subject.

In an embodiment, the method described herein comprises exposing the CSF to the Hx within about 21 days after the haemorrhagic stroke. In another embodiment disclosed herein, the method comprises exposing the CSF to the Hx from about 2 days to about 4 days after the haemorrhagic stroke. In yet another embodiment, the method comprises exposing the CSF to the Hx from about 5 days to about 14 days after the haemorrhagic stroke.

The present inventors have surprisingly shown that exposing cell-free heme within the subarachnoid space to a therapeutically effective amount of Hx for a period of at least about 2 minutes is sufficient to form detectable heme:Hx complexes in the CSF. Thus, in an embodiment, the period of time to which the CSF is exposed to the therapeutically effective amount of Hx is at least about 2 minutes (e.g., 2, 3, 4, 5, 6, 7, 8, 9, 10, 11, 12, 13, 14 minutes, and so on). In an embodiment, the period of time to which the CSF is exposed to the therapeutically effective amount of Hx is at least about 4 minutes. In another embodiment, the period of time to which the CSF is exposed to the therapeutically effective amount of Hx is at least about 5 minutes. In yet another embodiment, the period of time to which the CSF is exposed to the therapeutically effective amount of Hx is at least about 10 minutes. In an embodiment, the period of time to which the CSF is exposed to the therapeutically effective amount of Hx is from about 2 minutes to about 45 minutes, preferably from about 2 minutes to about 20 minutes, or more preferably from about 4 minutes to about 10 minutes.

The term "subject", as used herein, refers to a mammalian subject for whom treatment or prophylaxis is desired. Illustrative examples of suitable subjects include primates, especially humans, companion animals such as cats and dogs and the like, working animals such as horses, donkeys and the like, livestock animals such as sheep, cows, goats, pigs and the like, laboratory test animals such as rabbits, mice, rats, guinea pigs, hamsters and the like and captive wild animals such as those in zoos and wildlife parks, deer, dingoes and the like. In an embodiment, the subject is a human. In a further embodiment, the subject is a paediatric patient aged (i) from birth to about 2 years of age, (ii) from about 2 to about 12 years of age or (iii) from about 12 to about 21 years of age.

In an embodiment, the methods described herein comprise exposing the CSF to the therapeutically effective amount of Hx extracorporeally.

Where the CSF is to be exposed to the Hx extracorporeally (*ex vivo*)*,* the therapeutically effective amount may depend on the volume of CSF to which the Hx will be expose, whether the CSF is exposed to an Hx that is in solution or immobilise on a substrate (*e.g*., for affinity chromatography) and combinations of any of the foregoing. For instance, where the CSF is to be exposed to the Hx for complexation and removal of cell-free heme by affinity chromatography, the amount of Hx that is immobilise on the substrate need not result in complete complexation of cell-free heme during an initial pass, and it may be that multiple passes over the substrate may be required to complex and thereby remove substantially all of the cell-free heme from the CSF.

In an embodiment, the method comprises (i) obtaining a CFS sample from the subject following a haemorrhagic stroke and prior to exposing the CSF to the Hx; (ii) measuring the amount of cell-free Hb or cell-free heme in the CSF sample obtained in step (i); and (iii) determining the at least equimolar amount of Hx based on the concentration of the cell-free Hb or cell-free heme from step (ii). Suitable methods of measuring the amount of cell-free Hb and cell-free heme in a CSF sample will be known to persons skilled in the art, illustrative examples of which are described elsewhere herein.

As noted elsewhere herein, complexation of cell-free heme with the Hx within CSF will neutralise the otherwise adverse biological activity of cell-free heme on brain tissue. Whilst it is generally unnecessary to extract or remove cell-free heme:Hx complexes that have formed in accordance with the methods disclosed herein, it may be desirable in some instances to remove said complexes. For example, where the methods described herein comprise removing CSF from the subject and subsequently exposing the CSF to Hx extracorporeally, it may be desirable to remove cell-free heme:Hx complexes that have formed in the CSF prior to re-administering the CSF to the subject. Thus, in some embodiments, the method comprises removing Hx:cell-free heme complexes formed in the CSF.

In an embodiment, the method comprises:
(i) obtaining CSF from the subject following the haemorrhage;
(ii) exposing the CSF from step (i) to the Hx under conditions to allow the Hx to form a complex with cell-free heme in the CSF;
(iii) extracting the Hx:cell-free heme complexes from the CSF following step (ii) to obtain a heme-diminished CSF that has a lower amount of cell-free heme when compared to the CSF from step (i);
(iv) optionally repeating steps (ii) and (iii) to obtain a heme -diminished CSF that is substantially free of cell-free heme; and
(v) administering the heme-diminished CSF obtained from step (iii) or step (iv) to the CSF compartment of the subject.

As used herein, a "heme-diminished CSF" means CSF from which an amount of cell-free heme has been removed such that the CSF has a lower amount of cell-free heme when compared to the amount of cell-free heme prior to step (iii). It is to be understood that the term "heme-diminished CSF" is not intended to imply that all of the cell-free heme has been removed from the CSF and therefore includes embodiments in which at least some cell-free heme. In an embodiment, the heme-diminished CSF comprises at least about 5%, preferably at least about 10%, preferably at least about 15%, preferably at least about 20%, preferably at least about 25%, preferably at least about 30%, preferably at least about 35%, preferably at least about 40%, preferably at least about 45%, preferably at least about 50%, preferably at least about 55%, preferably at least about 60%, preferably at least about 65%, preferably at least about 70%, preferably at least about 75%, preferably at least about 80%, preferably at least about 85%, preferably at least about 90%, or more preferably at least about 95% less cell-free heme when compared to the amount of cell-free heme in the CSF obtained from the subject prior to extracting the Hx:cell-free heme complexes from the CSF. In an embodiment, the heme-diminished CSF comprises from about 5% to about 10%, preferably from about 10% to about 20%, preferably from about 20% to about 30%, preferably from about 30% to about 40%, preferably from about 40% to about 50%, preferably from about 50% to about 60%, preferably from about 60% to about 70%, preferably from about 70% to about 80%, preferably from about 80% to about 90%, or more preferably from about 90% to about 99% less cell-free heme when compared to the amount of cell-free heme in the CSF obtained from the subject prior to extracting the Hx:cell-free heme complexes from the CSF.

As described elsewhere herein, the heme-diminished CSF may optionally be further treated by repeating steps (ii) and (iii) to remove additional cell-free heme from the CSF, preferably to obtain an heme-diminished CSF that is substantially free of cell-free heme. By "substantially free of cell-free heme" means the Hb-diminished CSF comprises from about 70% to about 80%, preferably from about 80% to about 90%, or more preferably from about 90% to about 99% less cell-free heme when compared to the amount of cell-free heme in the CSF obtained from the subject prior to extracting the Hx:cell-free heme complexes from the CSF.

In an embodiment, the method comprises repeating steps (ii) and (iii) at least once (e.g., 1, 2, 3, 4, 5, 6, 7, 8, 9, 10, 11 times, and so on). In an embodiment, the method comprises repeating steps (ii) and (iii) at least 1 time, preferably 2 times, preferably 3 times, preferably 4 times, preferably 5 times, preferably 6 times, preferably 7 times, preferably 8 times, preferably 9 times, or more preferably 10 times, as required to obtain an Hb-diminished CSF that is substantially free of cell-free heme.

It is to be understood that the number of times that steps (ii) and (iii) need to be repeated to obtain an heme-diminished CSF that is substantially free of cell-free heme may depend on several factors, including (but not limited to) the concentration of the cell-free heme in the CSF from the subject, the concentration of the heme that is employed, the method of extraction, and so forth. In some instances, it may be desirable to perform steps (ii) and (iii) only once, in particular where repeating steps (ii) and (iii) may expose the CSF to contaminants, such as bacteria, yeast, fungus and viruses.

Suitable methods of extracting Hx:cell-free heme complexes from CSF will be known to persons skilled in the art, illustrative examples of which include size exclusion chromatography and / or affinity chromatography. Size exclusion chromatography allows Hx:cell-free heme complexes to be identified and separated from other components in the CSF by virtue of their larger size relative to free heme and Hx. Affinity chromatography allows Hx:cell-free heme complexes to be identified and separated from other components in the CSF by using a binding agent that binds specifically to an heme:Hx complex with negligible binding to free heme. Suitable binding agents, include antibodies or anti-binding fragments thereof, as would be familiar to persons skilled in the art.

In an embodiment, step (ii) comprises passing the CSF from step (i) over a substrate to which the Hx is immobilised. This advantageously allows the cell-free heme in the CSF to bind to the Hx thereby mobilising the cell-free heme to the substrate and allowing the heme-diminished CSF to be conveniently eluted from the substrate. Thus, in an embodiment, the Hx in step (ii) is immobilised on a substrate. Suitable substrates will be familiar to persons skilled in the art, illustrative examples of which include a size exclusion chromatography resin, affinity chromatography resin, a filter or a membrane. In an embodiment, the substrate is selected from ther group consisting of a size exclusion chromatography resin, an affinity chromatography resin, a filter and a membrane.

In an embodiment, step (ii) comprises passing the CSF from step (i) through an affinity chromatography resin under conditions that allow the cell-free heme in the CSF to bind to the resin; wherein step (iii) comprises eluting the CSF from the resin following step (ii); and wherein step (iv) comprising recovering the eluted CSF.

Prior to administering the heme-diminished CSF obtained from step (iii) or step (iv) to the CSF compartment of the subject in step (vi), it may be desirable to add to the heme-diminished CSF a therapeutically effective amount of Hp in order to scavenge residual cell-free Hb that may be present in the subject's CSF compartment and could give rise to an adverse secondary neurological outcome, as described elsewhere herein. Thus, in an embodiment, the method comprises adding to the heme-diminished CSF prior to step (v) a therapeutically effective amount of Hx, as described elsewhere herein.

Persons skilled in the art will understand that once CSF has been removed from the CSF compartment of a subject following a haemorrhagic stroke, there may be residual cell-free Hb remaining in the CSF compartment. It may therefore be desirable to rinse the CSF compartment (once the CSF has been removed in accordance with step (i), above) in order to remove at least some of the residual cell-free Hb and thereby eliminate or otherwise reduce the adverse secondary neurological outcomes to which such residual cell-free heme may otherwise give rise. Thus, in an embodiment, the method further comprises washing the CSF compartment following step (i) with a wash solution.

Suitable wash solutions will be familiar to persons skilled in the art. In an embodiment, the wash solution is an artificial CSF.

Artificial cerebrospinal fluid (aCSF) is typically a fluid that mimics natural CSF, including by salt content. Suitable compositions of aCSF will be familiar to persons skilled in the art, illustrative examples of which are described in US 20060057065 and Matzneller et al. (Pharmacology, 2016; 97(5-6):233-44), the contents of which are incorporated herein by reference in their entirety. The aCSF may comprise NaCl at a similar concentration to that found in natural CSF, as will be familiar to persons skilled in the art, and would typically include concentrations within about 15%, more preferably within about 10% of the concentration of NaCl in natural CSF. The aCSF may comprise NaHCO₃ at a similar concentration to that found in natural CSF, as will be familiar to persons skilled in the art, and would typically include concentrations within about 15%, more preferably within about 10% of the concentration of NaHCO₃ in natural CSF. The aCSF may comprise KCI at a similar concentration to that found in natural CSF, as will be familiar to persons skilled in the art, and would typically include concentrations within about 15%, more preferably within about 10% of the concentration of KCI in natural CSF. The aCSF may comprise NaH₂PO₄ at a similar concentration to that found in natural CSF, as will be familiar to persons skilled in the art, and would typically include concentrations within about 15%, more preferably within about 10% of the concentration of NaH₂PO₄ in natural CSF. The aCSF may comprise MgCl₂ at a similar concentration to that found in natural CSF, as will be familiar to persons skilled in the art, and would typically include concentrations within about 15%, more preferably within about 10% of the concentration of MgCl₂ in natural CSF. The aCSF may comprise glucose at a similar concentration to that found in natural CSF, as will be familiar to persons skilled in the art, and would typically include concentrations within about 15%, more preferably within about 10% of the concentration of glucose in natural CSF. Alternatively, the artificial CSF may omit glucose so as to reduce the likelihood of bacterial growth in any catheter used to administer the aCSF to a subject.

In an embodiment, the artificial CSF / wash solution comprises NaCl, KCI, KH₂PO₄, NaHCO₃, MgCl₆H₂0, CaCl₂H₂O and glucose.

In an embodiment, the wash solution comprises Hx.

In an embodiment, the wash solution comprises from about 2 µM to about 80 mM Hx. In an embodiment, the wash solution comprises from about 2 µM to about 20 mM Hx. In an embodiment, the wash solution comprises from about 100 µM to about 20 mM Hx. In an embodiment, the wash solution comprises from about 2 µM to about 1,200 µM Hx. In an embodiment, the wash solution comprises from about 5 µM to about 200 µM Hx. In an embodiment, the wash solution comprises from about 10 µM to about 120 µM Hx.

In an embodiment, the wash solution comprises at least an equimolar amount of Hx to the concentration of cell-free heme in the CFS of the subject following the haemorrhage. In another embodiment, the wash solution comprises from about 3 µM to about 1,200 µM Hx.

In some embodiments, it may be desirable to forego steps of extracorporeally removing cell-free heme from the CSF of a subject, as described herein, and instead replace the subject's CSF with an artificial CSF, as described elsewhere herein. Thus, in an embodiment, the method comprises:
(i) removing CSF from the subject following the haemorrhage;
(ii) rinsing the CSF compartment of the subject following step (i) with a wash solution comprising a therapeutically effective amount of Hx;
(iii) optionally repeating step (ii); and
(iv) administering to the CSF compartment of the subject, following step (ii) or step (iii), an artificial CSF.

By incorporating a therapeutically effective amount of Hx into the wash solution, residual cell-free heme in the CSF compartment can be complexed with the Hx, thereby neutralising the adverse biological effect of cell-free heme on brain tissue.

In an embodiment, the artificial CSF comprises NaCl, KCI, KH₂PO₄, NaHCO₃, MgCl₆H₂0, CaCl₂H₂O and glucose. In an embodiment, the artificial CSF comprises Hx.

In an embodiment, the artificial CSF comprises from about 2 µM to about 80 mM Hx. In an embodiment, the artificial CSF comprises from about 2 µM to about 20 mM Hx. In an embodiment, the artificial CSF comprises from about 100 µM to about 20 mM Hx. In an embodiment, the artificial CSF comprises from about 2 µM to about 1,200 µM Hx. In an embodiment, the artificial CSF comprises from about 5 µM to about 200 µM Hx. In an embodiment, the artificial CSF comprises from about 10 µM to about 120 µM Hx.

In an embodiment, the artificial CSF comprises at least an equimolar amount of Hx to the concentration of cell-free heme in the CFS of the subject following the haemorrhage. In another embodiment, the artificial CSF comprises from about 3 µM to about 1,300 µM Hx.

### Adjunct therapy

The methods of treating or preventing an adverse secondary neurological outcome in a subject following haemorrhagic stroke, as described herein, may suitably be performed together, either sequentially or in combination (e.g., at the same time), with one or more another treatment strategies designed to reduce, inhibit, prevent or otherwise alleviate one or more adverse secondary neurological outcome in a subject following haemorrhagic stroke. Thus, in an embodiment, the method further comprises administering to the subject a second agent for treating or preventing an adverse secondary neurological outcome following haemorrhagic stroke. Suitable other treatment strategies or second agents for treating or preventing an adverse secondary neurological outcome following haemorrhagic stroke will be familiar to persons skilled in the art, illustrative examples of which include:
(i) *Coagulopathy correction - e.g.,* using vitamin K antagonists (VKAs), novel oral anticoagulants (NOAC, such as dabigatran, rivaroxaban, and apixaban), factor eight inhibitor bypass activity (FEIBA) and activated recombinant factor VII (rFVlla), prothrombin complex concentrate, activated charcoal, antiplatelet therapy (APT), and aspirin monotherapy;
(ii) *Lowering blood pressure - e.g.,* antihypertensive agents, illustrative examples of which include (i) diuretics, such as thiazides, including chlorthalidone, chlorthiazide, dichlorophenamide, hydroflumethiazide, indapamide, and hydrochlorothiazide; loop diuretics, such as bumetanide, ethacrynic acid, furosemide, and torsemide; potassium sparing agents, such as amiloride, and triamterene; and aldosterone antagonists, such as spironolactone, epirenone, and the like; (ii) beta-adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol, and timolol, and the like; (iii) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, and verapamil, and the like; (iv) angiotensin converting enzyme (ACE) inhibitors such as benazepril; captopril; cilazapril; delapril; enalapril; fosinopril; imidapril; losinopril; moexipril; quinapril; quinaprilat; ramipril; perindopril; perindropril; quanipril; spirapril; tenocapril; trandolapril, and zofenopril, and the like; (v) neutral endopeptidase inhibitors such as omapatrilat, cadoxatril and ecadotril, fosidotril, sampatrilat, AVE7688, ER4030, and the like; (vi) endothelin antagonists such as tezosentan, A308165, and YM62899, and the like; (vii) vasodilators such as hydralazine, clonidine, minoxidil, and nicotinyl alcohol, and the like; (viii) angiotensin II receptor antagonists such as candesartan, eprosartan, irbesartan, losartan, pratosartan, tasosartan, telmisartan, valsartan, and EXP-3137, FI6828K, and RNH6270, and the like; (ix) α/β adrenergic blockers as nipradilol, arotinolol and amosulalol, and the like; (x) alpha 1 blockers, such as terazosin, urapidil, prazosin, bunazosin, trimazosin, doxazosin, naftopidil, indoramin, WHIP 164, and XENOIO, and the like; and (xi) -alpha 2 agonists such as lofexidine, tiamenidine, moxonidine, rilmenidine and guanobenz, and the like.
(ii-b) *Vasodilators - e.g.,* hydralazine (apresoline), clonidine (catapres), minoxidil (loniten), nicotinyl alcohol (roniacol), sydnone and sodium nitroprusside.
(iii) *Management of Seizures, Glucose and Temperature - e.g.,* antiepileptic drugs, insulin infusions to control blood glucose levels, maintenance of normo-thermia and therapeutic cooling;
(iv) *Surgical treatment - e.g.,* hematoma evacuation (surgical clot removal), decompressive craniectomy (DC), minimally invasive surgery (MIS; such as needle aspiration of basal ganglia haemorrhages), MIS with recombinant tissue-type plasminogen activator (rtPA);
(v) *Timing of Surgery - e.g.,* from 4 to 96 hours after symptom onset;
(vi) *Thrombin Inhibition - e.g.,* hirudin, argatroban, serine protease inhibitors (e.g., nafamostat mesilate);
(vii) *Prevention of Heme and Iron Toxicity - e.g.,* non-specific heme oxygenase (HO) inhibitors such as tin-mesoporphyrin, iron chelators such as deferoxamine;
(viii) *PPARg antagonists and agonists - e.g.,* rosiglitazone, 15d-PGJ2 and pioglitazone;
(ix) *Inhibition of microglial activation - e.g.,* tuftsin fragment 1-3 (a microglia/macrophage inhibitory factor) or minocycline (a tetracycline-class antibiotic);
*(x) Upregulation of NF-Erythroid-2-Related Factor 2 (Nrf2);*
(xi) *Cyclo-Oxygenase (COX) Inhibition - e.g.,* celecoxib (a selective COX-2 inhibitor);
(xii) *Matrix Metalloproteinases;*
(xiii) *TNF-α modulators - e.g.,* adenosine receptor agonists such as CGS 21680, TNF-α-specific antisense oligodeoxynucleotides such as ORF4-PE;
(xiv) *Raising blood pressure - e.g.,* catecholamines; and
*(xv) Inhibitors of TLR4 signalling - e.g.,* antibody Mts510 and TAK-242 (a cyclohexene derivative);

In an embodiment, the second agent is a vasodilator. Suitable vasodilators will be familiar to persons skilled in the art, illustrative examples of which include sydnone and sodium nitroprusside. Thus, in an embodiment disclosed herein, the second agent is selected from the group consisting of a sydnone and sodium nitroprusside.

The second agent may include haptoglobin (Hp). As described elsewhere herein, the present inventors have demonstrated that exogenous haptoglobin can be used to provide anti-vasospastic and anti-oxidative effects within the clinically relevant CSF-Hb concentration range and thereby hypothesise that therapeutic combinations of haptoglobin and hemopexin in the context of aSAH will likely provide synergistic protection against the vasospastic and oxidative effets of cell-free Hb and cell-free heme. Thus, in an embodiment, the methods described herein further comprise exposing the CSF of the subject to a therapeutically effective amount of haptoglobin (Hp) and for a period of time sufficient to allow the Hp to form a complex with, and thereby neutralise, cell-free Hb.

As noted elsewhere herein, the present inventors have found that Hp can reduce or otherwise prevent cell-free Hb-mediated CV *in vivo.* It is to be understood that the extent to which the methods disclosed herein reduce or otherwise prevent heme-mediated CV may depend on several factors, such as the degree of vasoconstriction (vessel narrowing) that is induced by cell-free Hb following a haemorrhagic stroke, the concentration of cell-free Hb in the subject's CSF following a haemorrhagic stroke, the time period to which the CSF is exposed to the Hp and the presence or absence of any persistence bleeding. Means of assessing whether the methods disclosed herein have reduced or otherwise prevented CV in the subject will be familiar to persons skilled in the art, illustrative examples of which include digital subtraction angiography (DSA), computed tomography (CT) angiography (CTA), magnetic resonance (MR) angiography (MRA) and catheter angiography (CA).

In an embodiment, exposing the CSF of a subject in need thereof to the Hp, as described herein, restores the average diameter of the lumen of a constricted cerebral blood vessel by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, or more preferably from about 40% to about 50% following a 60 minute period of exposure, as determined by DSA.

In an embodiment, exposing the CSF of a subject in need thereof to the Hp, as described herein, restores the average diameter of the lumen of a constricted anterior cerebral artery by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, or more preferably from about 40% to about 50% following a 60 minute period of exposure, as determined by DSA.

In an embodiment, exposing the CSF of a subject in need thereof to the Hp, as described herein, restores the average diameter of the lumen of a constricted internal carotid artery by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, or more preferably from about 40% to about 50% following a 60 minute period of exposure, as determined by DSA.

In an embodiment, exposing the CSF of a subject in need thereof to the Hp, as described herein, restores the average diameter of the lumen of a constricted medial cerebral artery by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, or more preferably from about 40% to about 50% following a 60 minute period of exposure, as determined by DSA.

In an embodiment, exposing the CSF of a subject in need thereof to the Hp, as described herein, restores the average diameter of the lumen of a constricted basilar artery by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, or more preferably from about 40% to about 50% following a 60 minute period of exposure, as determined by DSA.

In an embodiment, exposing the CSF of a subject in need thereof to the Hp, as described herein, increases the average lumen area of a constricted small parenchymal vessel (e.g., a cerebral arteriole) by at least 10%, preferably by at least 20%, preferably by at least 25%, preferably by at least 30%, preferably by at least 35%, preferably by at least 40%, preferably by at least 45%, preferably by at least 55%, preferably by at least 60%, preferably by at least 65%, preferably by at least 70%, preferably by at least 75%, or more preferably by at least 80% following a 60 minute period of exposure, as determined by *in vivo* perfusion imaging (e.g. MRI perfusion, CT perfusion). In an embodiment, the small parenchymal vessel is a cerebral arteriole. In an embodiment, exposing the CSF of a subject in need thereof to the Hp, as described herein, restores the cerebral microperfusion by prevention of small parenchymal vessel constriction by at least 10%, preferably by at least 20%, preferably by at least 25%, preferably by at least 30%, preferably by at least 35%, preferably by at least 40%, preferably by at least 45%, preferably by at least 55%, preferably by at least 60%, preferably by at least 65%, preferably by at least 70%, preferably by at least 75%, or more preferably by at least 80% following a 60 minute period of exposure, as determined by *in vivo* perfusion imaging (*e.g*. MRI perfusion, CT perfusion). In an embodiment, the small parenchymal vessel is a cerebral arteriole.

As noted elsewhere herein, the present inventors have shown for the first time that a therapeutically effective amount of Hp can reduce the oxidative potential of cell-free Hb in CSF. Thus, in an embodiment, exposing the CSF of a subject in need thereof to the Hp, as described herein, reduces oxidative tissue injury. Suitable methods for determining oxidative tissue injury will be familiar to persons skilled in the art, illustrative examples of which are described in Katerji et al. (2019; Oxid. Med. Cell Longey; 1279250), including measuring levels of malondialdehyde (MDA), an end-product of lipid peroxidation. Thus, in an embodiment, exposing the CSF of a subject in need thereof to the Hp, as described herein, reduces lipid peroxidation.

### Haptoglobin

Haptoglobin (Hp) has a tetrameric structure comprising two alpha and two beta chains, linked by disulphide linkages. The beta chain (245 amino acids) has a mass of about 40 kDa (of which approximately 30% w/w is carbohydrate) and is shared by all phenotypes. The alpha chain exists in at least two forms: alpha1 (83 amino acids, 9 kDa) and alpha2 (142 amino acids, 17.3 kDa). Therefore, Hp occurs as three phenotypes, referred to as Hp1-1, Hp2-1 and Hp2-2. Hp1-1 contains two alpha1 chains, Hp2-2 contains two alpha2 chains, and Hp2-1 contains one alpha1 and one alpha2 chain. Hp 1-1 has a molecular mass of 100 kDa, or 165 kDa when complexed with Hb. Hp1-1 exists as a single isoform, and is also referred to as Hp dimer. Hp2-1 has an average molecular mass of 220 kDa and forms liner polymers. Hp2-2 has an average molecular mass of 400 kDa and forms cyclic polymers. Each different polymeric form is a different isoform. A PCR methodology has been devised (Koch et al. 2002, Clin. Chem. 48: 1377-1382) for studying Hp polymorphism.

Two major alleles, Hp1 and Hp2, exist for the Hp gene found on chromosome 16. The two alleles are responsible for three different possible genotypes with structural polymorphism: homozygous (1-1 or 2-2) and heterozygous 2-1. In Western populations, it is estimated that the distribution of Hp 1-1 is ~16%, Hp 2-1 is ~48%, and Hp 2-2 is ~36%. Hp is cleaved into two subunits α and β chains, joined by a disulphide bond. Both alleles share the same β chain. The β chain is responsible for binding the Hb, thus both genotypes have similar Hb binding affinity.

It is to be understood that naturally-occurring and recombinant forms of Hp are suitable for the methods described herein, as long as they are capable of forming a complex with cell-free Hb and thereby neutralise the biological activity of the cell-free Hb. Suitable naturally-occurring forms of Hp will be known to persons skilled in the art, illustrative examples of which are described in Koch et al. (2002, Clin. Chem. 48: 1377-1382) and Kasvosve et al. (2010, Chapter 2 - Haptoglobin Polymorphism and Infection; Advances in Clinical Chemistry, 50:23-46), the entire contents of which are incorporated herein by reference.

In an embodiment, the Hp comprises, consists or consists essentially of plasma derived Hp. The Hp is preferably a human Hp. A variety of protocols for the isolation of Hp from a natural source of Hp- (*e.g*., blood plasma) will be familiar to persons skilled in the art, illustrative examples of which are described in US patent nos. 4,061,735 and 4,137,307 (to Funakoshi *et al*.) and US patent publication no. 20140094411 (to Brinkman), the entire contents of which are incorporated herein by reference. Other suitable methods for isolating Hp from a natural source of Hp are described in Katnik & Jadach (1993, Arch. Immunol. Ther. Exp. (Warz) 41: 303-308), Tseng et al. (2004, Protein Expr Purif 33: 265-273), Katnik et al. (1995, Eur J. Clin. Chem. Clin. Biochem. 33:727-732), Yang and Mao (1999, J. Chromatogr. B. Biomed. Sci. Appl., 731: 395-402), and Basler & Burrel (1983 Inflammation 7(4): 387-400).

It is to be understood that the term "haptoglobin", as used herein, includes all phenotypes (including all isoforms) of Hp. The Hp may be homogenous (insofar as it consists essentially of an Hp of the same isoform) or heterogeneous (insofar as it comprises a combination of different Hp isoforms, including Hp1-1, Hp1-2 and Hp2-2). It is to be understood that the composition of the Hp will ultimately depend on the phenotypes of the source. For example, if pooled plasma samples are used to extract / purify the Hp, more than one isoform of Hp will likely be isolated. Suitable methods for determining Hp isoforms that are present in an isolate will be familiar to persons skilled in the art, illustrative examples of which are discussed in Shih et al. (2014, Hematology, 89(4):443-447), the entire contents of which are incorporated herein by reference. Other suitable methods of determining Hp isoforms that are present in an isolate include high performance size exclusion chromatography (HPLC-SEC assay), Hp ELISA, and turbimetric readings, that different isoforms of Hp will typically give differing signals in the different assays.

In an embodiment, the Hp is selected from the group consisting of an Hp1-1 homodimer, an Hp1-2 multimer, an Hp2-2 multimer and a combination of any of the foregoing. In a preferred embodiment, the Hp comprises, consists or consists essentially of an Hp1-1 homodimer. The Hp may be a naturally-occurring Hp (*e.g*., plasma derived) or it may be produced as a recombinant protein, illustrative examples of which are described elsewhere herein. In an embodiment, the plasma derived Hp comprises, consists or consists essentially of Hp1-1 homodimer. In an embodiment, the Hp comprises, consists or consists essentially of recombinant Hp.

Unless stated otherwise, the term Hp, as used herein, includes functional analogues of native or naturally-occurring Hp. The term "functional analogue" is to be understood to mean an agent that shares substantially the same biological activity of naturally-occurring (native) Hp, insofar as that biological activity is at least the ability of the analogue to form a complex with cell-free Hb and thereby neutralise its biological activity. By "substantially the same biological activity" typically means the functional analogue has a binding affinity for cell-free Hb that is at least 40% (*e.g.,* 40%, 45%, 50%, 55%, 60%, 65%, 70%, 85%, 90%, 95%, 100%, 105%, 100%, 105%, 110%, 115%, 120%, 125%, 130%, 135%, 140%, 145%, 150%, 155%, 160%, 165% and so on) of the binding affinity of naturally-occurring Hp, including naturally-occurring Hp isoforms (*e.g.*, Hp1-1, Hp1-2 and Hp2-2). Suitable methods for determining whether an agent is a functional analogue of Hp will be familiar to persons skilled in the art, illustrative examples of which include are described elsewhere herein (*e.g*, the ability of the functional analogue to reduce cell-free Hb-induced cerebral vasospasms).

In an embodiment disclosed herein, the functional analogue of Hp is a functional fragment of native Hp. A functional fragment of native Hp can be any suitable length, as long as the fragment retains the ability to form a complex with cell-free Hb and thereby neutralise its biological activity.

In another embodiment, the functional analogue is a peptide that has a different amino acid sequence to a naturally-occurring (native) Hp molecule (*i.e.,* a comparator). The functional analogue may include a molecule that has an amino acid sequence that differs from the amino acid sequence of the alpha and / or beta chains of native Hp by one or more (*e.g.,* 1, 2, 3, 4, 5, 6, 7, 8, 9 or more) amino acid substitutions, wherein said difference does not, or does not completely, abolish the ability of the analogue to form a complex with cell-free Hb and thereby neutralise its biological activity. In some embodiments, the functional analogue comprises amino acid substitutions that enhance the ability of the analogue to form a complex with cell-free Hb, as compared to native Hp. In an embodiment, the functional analogue has an amino acid sequence that differs from the amino acid sequence of the alpha and / or beta chain of native Hp by one or more conservative amino acid substitutions. As used herein, the term "conservative amino acid substitution" refers to changing amino acid identity at a given position to replace it with an amino acid of approximately equivalent size, charge and/or polarity.

Examples of natural conservative substitutions of amino acids include the following 8 substitution groups (designated by the conventional one-letter code): (1) M, I, L, V; (2) F, Y, W; (3) K, R, (4) A, G; (5) S, T; (6) Q, N; (7) E, D; and (8) C, S.

In an embodiment, the functional analogue has at least 85% sequence identity to an amino acid sequence of the alpha and / or beta chain of native Hp. Reference to "at least 85%" includes 85%, 86%, 87%, 88%, 89%, 90%, 91%, 92%, 93%, 94%, 95%, 96%, 97%, 98%, 99% or 100% sequence identity or similarity, for example, after optimal alignment or best fit analysis. Thus, in an embodiment, the sequence has at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% or preferably 100% sequence identity or sequence homology with the sequences identified herein, for example, after optimal alignment or best fit analysis.

The terms "identity", "similarity", "sequence identity", "sequence similarity", "homology", "sequence homology" and the like, as used herein, mean that at any particular amino acid residue position in an aligned sequence, the amino acid residue is identical between the aligned sequences, as described elsewhere herein. The term "similarity" or "sequence similarity" as used herein, indicates that, at any particular position in the aligned sequences, the amino acid residue is of a similar type between the sequences, as described elsewhere herein. In an embodiment, an amino acid sequence may be modified by way of conservative substitution of any of the amino acid residues contained therein, such that the modification has no effect on the binding specificity or functional activity of the modified polypeptide when compared to the unmodified (native) Hp polypeptide.

In an embodiment, a functional analogue includes amino acid substitutions and/or other modifications relative to native Hp in order to increase the stability of the analogue or to increase the solubility of the analogue. The functional analogue may be a naturally-occurring compound / peptide or it may be synthetically produced by chemical synthesis using methods known to persons skilled in the art.

The Hp may suitably be produced as a recombinant protein in a microorganism, which can be isolated and, if desired, further purified. Suitable microorganisms for the production of recombinant Hp will be familiar to persons skilled in the art, illustrative examples of which include bacteria, yeast or fungi, eukaryote cells (*e.g*., mammalian or an insect cells), or in a recombinant virus vector (*e.g*., adenovirus, poxvirus, herpesvirus, Simliki forest virus, baculovirus, bacteriophage, sindbis virus or sendai virus). Suitable bacteria for producing recombinant peptides will be familiar to persons skilled in the art, illustrative examples of which include *E*. *coli, B.subtilis* or any other bacterium that is capable of expressing the peptide sequences. Illustrative examples of suitable yeast types for producing recombinant peptides include *Saccharomyces cerevisiae, Schizosaccharomyces pombe, Candida, Pichia pastoris* or any other yeast capable of expressing peptides. Corresponding methods are well known in the art. Also methods for isolating and purifying recombinantly produced peptide sequences are well known in the art and include, for example, gel filtration, affinity chromatography and ion exchange chromatography.

To facilitate isolation of a recombinant Hp, as described herein, a fusion polypeptide may be made where the peptide sequence of the Hp, or functional analogue thereof, is translationally fused (covalently linked) to a heterologous polypeptide which enables isolation by affinity chromatography. Illustrative examples of suitable heterologous polypeptides are His-Tag (*e.g*. His₆. 6 histidine residues), GST-Tag (Glutathione-S-transferase) *etc.*

For preparing recombinant Hp, phage libraries and/or peptide libraries are also suitable, for instance, produced by means of combinatorial chemistry or obtained by means of high throughput screening techniques for the most varying structures (see, for example, Display: A Laboratory Manual by Carlos F. Barbas (Editor), *et al.;* and Willats WG Phage display: practicalities and prospects. Plant Mol. Biol. 2002 December; 50(6):837-54).

Illustrative examples of recombinant Hp include the peptides with accession no. NP_005134 (as described by Morishita et al. 2018, Clin. Chim. Acta 487, 84-89) and accession no. P00738.

The Hp may be fused, coupled or otherwise attached to one or more heterologous moieties as part of a fusion protein, including those described elsewhere herein in the context of Hx.

In the context of Hp, the term "therapeutically effective amount", as used herein, means the amount or concentration of Hp that, when administered to a subject in need thereof, is sufficient to allow the Hp to bind to, and form a complex with, cell-free Hb present in the CSF of the subject and thereby neutralise the otherwise adverse biological effect of the cell-free Hb. It would be understood by persons skilled in the art that the therapeutically effective amount of peptide may vary depending upon several factors, illustrative examples of which include whether the Hp is to be administered directly to the subject (*e.g,* intrathecally, intracranially or intracerebroventricularly), the health and physical condition of the subject to be treated, the taxonomic group of subject to be treated, the severity of the haemorrhage (*e.g*., the extent of bleeding), the route of administration, the concentration and / or amount of cell-free Hb in the CSF compartment and combinations of any of the foregoing.

The therapeutically effective amount of Hp will typically fall within a relatively broad range that can be determined by persons skilled in the art. Illustrative examples of a suitable therapeutically effective amounts of Hp include from about 2 µM to about 20 mM, preferably from about 2 µM to about 5 mM, preferably from about 100 µM to about 5 mM, preferably from about 2 µM to about 300 µM, preferably from about 5 µM to about 100 µM, preferably from about 5 µM to about 50 µM, or more preferably from about 10 µM to about 30 µM.

In an embodiment, the therapeutically effective amount of Hp is from about 2 µM to about 20 mM. In an embodiment, the therapeutically effective amount of Hp is from about 2 µM to about 5 mM. In an embodiment, the therapeutically effective amount of Hp is from about 100 µM to about 5 mM. In an embodiment, the therapeutically effective amount of Hp is from about 2 µM to about 300 µM. In an embodiment, the therapeutically effective amount of Hp is from about 5 µM to about 50 µM. In an embodiment, the therapeutically effective amount of Hp is from about 10 µM to about 30 µM.

In an embodiment, the therapeutically effective amount of Hp is at least an equimolar amount to the concentration of cell-free Hb in the CFS of the subject following the haemorrhage. In another embodiment, the therapeutically effective amount of Hp is an amount sufficient to complex from about 3 µM to about 300 µM cell-free Hb in CSF. Suitable methods of measuring the concentration of cell-free Hb in CSF will be known to persons skilled in the art, illustrative examples of which are described in Cruickshank AM., 2001, ACP Best Practice No 166, J. Clin. Path., 54(11):827-830) and Hugelshofer M. et al., 2018. World Neurosurg.;120:e660-e666), the contents of which are incorporated herein by reference in their entirety.

It is to be understood that achieving a therapeutically effective amount of Hp, as herein described, may depend on the final volume of the CSF to which the Hp, or functional analogue thereof, is exposed. For example, where the Hp is to administered to an adult human subject (*e.g*., intrathecally), and taking into account that the average volume of CSF in an adult human subject is about 150 mL, a therapeutically effective amount of about 10 µM Hp can be achieved by administering to the subject a 5mL solution of about 310 µM Hp. In another illustrative example, the methods described herein comprise removing 50mL of CSF from the subject and replacing it with 50mL of artificial CSF comprising about 30 µM Hp, thereby achieving a therapeutically effective amount of about 10 µM Hp in the CSF compartment of the subject.

Dosages of Hp may also be adjusted to provide the optimum therapeutic response. For example, several divided doses may be administered daily, weekly, or other suitable time intervals, or the dosages may be proportionally reduced as indicated by the exigencies of the situation.

It is to be understood that, in the context of Hp, the term "therapeutically effective amount" does not imply an amount or concentration of Hp that, when administered to a subject in need thereof, will complex with and neutralise all cell-free Hb that is present in the CSF of the subject. In some embodiments, it is sufficient that the therapeutically effective amount of Hp, when administered to a subject in need thereof, will complex with and neutralise a sufficient amount of the cell-free Hb that is present in the CSF of the subject so as to minimise, abrogate or otherwise reduce the adverse secondary neurological outcomes in the subject that are mediated by cell-free Hb following a haemorrhagic stroke into the CSF of the subject. The reduction in the concentration cell-free Hb in the CSF of the subject following treatment, as described herein, that would be required to minimise, abrogate or otherwise reduce an adverse secondary neurological outcome following a haemorrhagic stroke into the CSF of the subject provide will be familiar to persons skille din the art. In an embodiment, the therapeutically effective amount of Hp is sufficient to reduce the amount of cell-free Hb in the CSF of the subject during treatment to a level of about 8 µM or less, preferably about 7 µM or less, preferably about 6 µM or less, preferably about 5 µM or less, preferably about 4 µM or less, preferably about 3 µM or less, preferably about 2 µM or less or more preferably about 1 µM or less. In an embodiment, the therapeutically effective amount of Hp is sufficient to reduce the amount of cell-free Hb in the CSF of the subject during treatment to a level of about 7 µM or less. In another embodiment, the therapeutically effective amount of Hp is sufficient to reduce the amount of cell-free Hb in the CSF of the subject during treatment to a level of about 6 µM or less.

In the context of Hp, the term "exposing", as used herein, means bringing into contact the CSF with the Hp in such a way as to allow the Hp to bind to and form a complex with cell-free Hb (the predominant form of which will be oxyHb) that is present in the CSF, whereby the formation of Hb:Hp complexes substantially neutralises the otherwise adverse biological effect of cell-free Hb on brain tissue. By "substantially neutralise" is meant a reduction to the adverse biological effect of cell-free Hb on brain tissue, as represented subjectively or qualitatively as a percentage reduction by at least 10%, preferably from about 10% to about 20%, preferably from about 15% to about 25%, preferably from about 20% to about 30%, preferably from about 25% to about 35%, preferably from about 30% to about 40%, preferably from about 35% to about 45%, preferably from about 40% to about 50%, preferably from about 45% to about 55%, preferably from about 50% to about 60%, preferably from about 55% to about 65%, preferably from about 60% to about 70%, preferably from about 65% to about 75%, preferably from about 70% to about 80%, preferably from about 75% to about 85%, preferably from about 80% to about 90%, preferably from about 85% to about 95%, or most preferably from about 90% to 100% comparted to the biological effect of cell-free Hb on brain tissue in the absence of therapeutic Hp, including by at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%. Methods by which a reduction to the adverse biological effect of cell-free Hb can be measured or determined (qualitatively or quantitatively) will be familiar to persons skilled in the art, illustrative examples of which are described elsewhere herein.

It is to be understood that the manner in which the CSF of a subject in need thereof is exposed to a therapeutically effective amount of Hp, as described herein, may vary, depending, for example, on whether said exposure is to be performed within the CSF compartment of a subject in need thereof (*i.e., in vivo*) or extracorporeally in CSF obtained from the subject (*i.e, ex vivo*)*.* Where said exposure is to be performed within the CSF compartment of a subject in need thereof (*i.e., in vivo*)*,* the route of administration of the Hp will be selected to allow the Hp to contact cell-free Hb within the CSF compartment. Suitable routes of administration will be familiar to persons skilled in the art, illustrative examples of which include intrathecal, intracranial and intracerebroventricular. In an embodiment, the therapeutically effective amount of Hp is administered via an external ventricular drain that is placed, for example, in the subject after a haemorrhagic stroke to temporarily drain the CSF and decrease intracranial pressure.

In an embodiment, the method comprises intracranially administering to the subject the therapeutically effective amount of the Hp.

In an embodiment, the method comprises intrathecally administering to the subject the therapeutically effective amount of the Hp. In an embodiment, the method comprises intrathecally administering to the subject the therapeutically effective amount of the Hp into the spinal canal. In an embodiment, the method comprises intrathecally administering to the subject the therapeutically effective amount of the Hp into the subarachnoid space. In an embodiment, the method comprises intracerebroventricularly administering to the subject the therapeutically effective amount of the Hp.

Alternatively, or in addition, the methods described herein comprise removing CSF from a subject in need thereof, exposing the CSF to a therapeutically effective amount of Hp for a period of time sufficient to allow the Hp to form a complex with, and thereby neutralise, the cell-free Hb in the CSF, removing the Hb:Hp complexes thus formed in the CSF to produce an Hb-diminished CSF and administering the Hb-diminished CSF to the subject (*e.g,* intrathecally, or intracerebroventricularly). It will be understood that removing CSF from the CSF compartment will typically not result in a CSF compartment that is entirely free of CSF, noting that at least some CSF will remain in the CSF compartment. Optionally, the CSF compartment can be rinsed with a pharmaceutically acceptable wash solution once CSF has been removed in order to remove at least some of the residual Hb that may be present in the CSF compartment. The wash solution may optionally comprise Hp, to further complex and thereby neutralise at least some of the residual cell-free Hb that may be present in the CSF compartment. In an embodiment, the wash solution is an artificial CSF, as described elsewhere herein.

In an embodiment, the method described herein comprises removing a sample of CSF from the CSF compartment of a subject in need thereof, adding Hp to the CSF sample to obtain an Hp-enriched CSF sample, administering the Hp-enriched CSF sample to the CSF compartment of the subject, thereby exposing the CSF compartment to a therapeutically effective amount of the Hp and for a period of time sufficient to allow the Hp to form a complex with, and thereby neutralise, cell-free Hb in the CSF of the subject, and optionally, repeating the above steps. Preferably, the amount of Hp that is added to the CSF sample will be determined such that, upon administration to the subject, will provide a therapeutically effective amount of Hp within the CSF of the subject. Hence, the amount of Hp to be added in the CSF sample will depend on the volume of CSF sample that is removed and re-administered to the subject.

In an embodiment, the method further comprises:
(i) obtaining sample of CSF from the CSF compartment of the subject following the haemorrhage;
(ii) adding to the CSF sample of step (i) Hp to obtain an Hp-enriched CSF sample;
(iii) administering the Hp-enriched CSF sample to the subject, thereby exposing the CSF compartment of the subject to a therapeutically effective amount of Hp for a period of time sufficient to allow the Hp to form a complex with cell-free Hb in the CSF compartment of the subject; and
(iv) optionally repeating steps (i) to (iii).

The volume of CSF that is to be removed and re-administered to the subject will desirably be substantially the same. For example, if a 50mL sample of CSF is removed from the CSF compartment of the subject, the entire 50mL volume of CSF comprising the Hp will be re-administered to the subject. However, it will be understood that the volumes may be dissimilar, as long as any difference in volumes does not give rise to significant adverse clinical outcomes. In some embodiments, the volume of CSF that is re-administered to the subject will be less than the volume of CSF that was removed from the subject. In other embodiments, the volume of CSF that is re-administered to the subject will be greater than the volume of CSF that was removed from the subject, with the addition of Hp, alone or in combination with any other therapeutical agents, making up the extra volume.

In another embodiment, the method described herein comprises removing a volume of CSF from the CSF compartment of a subject in need thereof, replacing the volume of CSF removed from the subject with a volume of artificial CSF comprising a Hp, thereby exposing the CSF of the subject to a therapeutically effective amount of the Hp and for a period of time sufficient to allow the Hp to form a complex with, and thereby neutralise, cell-free Hb in the CSF of the subject. Preferably, the amount of Hp in the artificial CSF will be determined such that, upon administration to the subject, will provide a therapeutically effective amount of Hp within the CSF of the subject. Hence, the amount of Hp to be added to the artificial CSF will depend on the volume of artificial CSF that will be administered to the subject.

In an embodiment, the method further comprises:
(i) removing a volume of CSF from the CSF compartment of the subject following the haemorrhage;
(ii) providing an artificial CSF comprising Hp;
(iii) administering the artificial CSF of (ii) to the subject, thereby exposing the CSF compartment of the subject to a therapeutically effective amount of Hp for a period of time sufficient to allow the Hp to form a complex with cell-free Hb in the CSF compartment of the subject; and
(iv) optionally repeating steps (i) to (iii).

The volume of artificial CSF that is administered to the subject will desirably be substantially the same as the volume of CSF removed from the subject. For example, if a 50mL sample of CSF is removed from the CSF compartment of the subject, a volume of about 50mL of artificial CSF comprising the therapeutically effective amount of Hp will be used to replace the volume of CSF removed. However, it will be understood that the volumes may be dissimilar, as long as any difference in volumes does not give rise to significant adverse clinical outcomes. In some embodiments, the volume of artificial CSF that is administered to the subject will be less than the volume of CSF that was removed from the subject. In other embodiments, the volume of artificial CSF that is administered to the subject will be greater than the volume of CSF that was removed from the subject.

In an embodiment, the method described herein comprises exposing the CSF to the Hp within about 21 days after the haemorrhagic stroke. In another embodiment disclosed herein, the method comprises exposing the CSF to the Hp from about 2 days to about 4 days after the haemorrhagic stroke. In yet another embodiment, the method comprises exposing the CSF to the Hp from about 5 days to about 14 days after the haemorrhagic stroke.

In an embodiment, the period of time to which the CSF is exposed to the therapeutically effective amount of Hp is at least about 2 minutes (*e.g.,* 2, 3, 4, 5, 6, 7, 8, 9, 10, 11, 12, 13, 14 minutes, and so on). In an embodiment, the period of time to which the CSF is exposed to the therapeutically effective amount of Hp is at least about 4 minutes. In another embodiment, the period of time to which the CSF is exposed to the therapeutically effective amount of Hp is at least about 5 minutes. In yet another embodiment, the period of time to which the CSF is exposed to the therapeutically effective amount of Hp is at least about 10 minutes. In an embodiment, the period of time to which the CSF is exposed to the therapeutically effective amount of Hp is from about 2 minutes to about 45 minutes, preferably from about 2 minutes to about 20 minutes, or more preferably from about 4 minutes to about 10 minutes.

In an embodiment, the methods described herein further comprise exposing the CSF to the therapeutically effective amount of Hp extracorporeally.

Where the CSF is to be exposed to the Hp extracorporeally (*ex vivo*)*,* the therapeutically effective amount may depend on the volume of CSF to which the Hp will be expose, whether the CSF is exposed to an Hp that is in solution or immobilise on a substrate (*e.g.,* for affinity chromatography) and combinations of any of the foregoing. For instance, where the CSF is to be exposed to the Hp for complexation and removal of cell-free Hb by affinity chromatography, the amount of Hp that is immobilise on the substrate need not result in complete complexation of cell-free Hb during an initial pass, and it may be that multiple passes over the substrate may be required to complex and thereby remove substantially all of the cell-free Hb from the CSF.

In an embodiment, the method further comprises (i) obtaining a CFS sample from the subject following a haemorrhagic stroke and prior to exposing the CSF to the Hp; (ii) measuring the amount of cell-free Hb in the CSF sample obtained in step (i); and (iii) determining the at least equimolar amount of Hp based on the concentration of cell-free Hb from step (ii). Suitable methods of measuring the amount of cell-free Hb in a CSF sample will be known to persons skilled in the art, an illustrative example of which is described by Oh et al. (2016, Redox Biology, 9: 167-177), the contents of which are incorporated herein by reference in their entirety.

As noted elsewhere herein, complexation of cell-free Hb with the Hb within CSF will neutralise the otherwise adverse biological activity of cell-free Hb on brain tissue. Whilst it is generally unnecessary to extract or remove cell-free Hb:Hp complexes that have formed in accordance with the methods disclosed herein, it may be desirable in some instances to remove said complexes. For example, where the methods described herein comprise removing CSF from the subject and subsequently exposing the CSF to Hp extracorporeally, it may be desirable to remove cell-free Hb:Hp complexes that have formed in the CSF prior to re-administering the CSF to the subject. Thus, in some embodiments, the method comprises removing Hp:cell-free Hb complexes formed in the CSF.

In an embodiment, the method further comprises:
(i) obtaining CSF from the subject following the haemorrhage;
(ii) exposing the CSF from step (i) to the Hp under conditions to allow the Hp to form a complex with cell-free Hb in the CSF;
(iii) extracting the Hp:cell-free Hb complexes from the CSF following step (ii) to obtain an Hb-diminished CSF that has a lower amount of cell-free Hb when compared to the CSF from step (i);
(iv) optionally repeating steps (ii) and (iii) to obtain an Hb-diminished CSF that is substantially free of cell-free Hb; and
(v) administering the Hb-diminished CSF obtained from step (iii) or step (iv) to the CSF compartment of the subject.

As used herein, an "Hb-diminished CSF" means CSF from which an amount of cell-free Hb has been removed such that the CSF has a lower amount of cell-free Hb when compared to the amount of cell-free Hb prior to step (iii). It is to be understood that the term "Hb-diminished CSF" is not intended to imply that all of the cell-free Hb has been removed from the CSF and therefore includes embodiments in which at least some cell-free Hb. In an embodiment, the Hb-diminished CSF comprises at least about 5%, preferably at least about 10%, preferably at least about 15%, preferably at least about 20%, preferably at least about 25%, preferably at least about 30%, preferably at least about 35%, preferably at least about 40%, preferably at least about 45%, preferably at least about 50%, preferably at least about 55%, preferably at least about 60%, preferably at least about 65%, preferably at least about 70%, preferably at least about 75%, preferably at least about 80%, preferably at least about 85%, preferably at least about 90%, or more preferably at least about 95% less cell-free Hb when compared to the amount of cell-free Hb in the CSF obtained from the subject. In an embodiment, the Hb-diminished CSF comprises from about 5% to about 10%, preferably from about 10% to about 20%, preferably from about 20% to about 30%, preferably from about 30% to about 40%, preferably from about 40% to about 50%, preferably from about 50% to about 60%, preferably from about 60% to about 70%, preferably from about 70% to about 80%, preferably from about 80% to about 90%, or more preferably from about 90% to about 99% less cell-free Hb when compared to the amount of cell-free Hb in the CSF obtained from the subject.

As described elsewhere herein, the Hb-diminished CSF may optionally be further treated by repeating steps (ii) and (iii) to remove additional cell-free Hb from the CSF, preferably to obtain an Hb-diminished CSF that is substantially free of cell-free Hb. By "substantially free of cell-free Hb" means the Hb-diminished CSF comprises from about 70% to about 80%, preferably from about 80% to about 90%, or more preferably from about 90% to about 99% less cell-free Hb when compared to the amount of cell-free Hb in the CSF obtained from the subject.

In an embodiment, the method comprises repeating steps (ii) and (iii) at least once (*e.g.,* 1, 2, 3, 4, 5, 6, 7, 8, 9, 10, 11 times, and so on). In an embodiment, the method comprises repeating steps (ii) and (iii) at least 1 time, preferably 2 times, preferably 3 times, preferably 4 times, preferably 5 times, preferably 6 times, preferably 7 times, preferably 8 times, preferably 9 times, or more preferably 10 times, as required to obtain an Hb-diminished CSF that is substantially free of cell-free Hb.

It is to be understood that the number of times that steps (ii) and (iii) need to be repeated to obtain an Hb-diminished CSF that is substantially free of cell-free Hb may depend on several factors, including (but not limited to) the concentration of the cell-free Hb in the CSF from the subject, the concentration of the Hb that is employed, the method of extraction, and so forth. In some instances, it may be desirable to perform steps (ii) and (iii) only once, in particular where repeating steps (ii) and (iii) may expose the CSF to contaminants, such as bacteria, yeast, fungus and viruses.

Suitable methods of extracting Hp:cell-free Hb complexes from CSF will be known to persons skilled in the art, illustrative examples of which include size exclusion chromatography and / or affinity chromatography. Size exclusion chromatography allows Hp:cell-free Hb complexes to be identified and separated from other components in the CSF by virtue of their larger size relative to free Hb and Hp. Affinity chromatography allows Hp:cell-free Hb complexes to be identified and separated from other components in the CSF by using a binding agent that binds specifically to an Hb:Hp complex with negligible binding to free Hb. Suitable binding agents, include antibodies or anti-binding fragments thereof, as would be familiar to persons skilled in the art.

In an embodiment, step (ii) comprises passing the CSF from step (i) over a substrate to which the Hp is immobilised. This advantageously allows the cell-free Hb in the CSF to bind to the Hp thereby mobilising the cell-free Hb to the substrate and allowing the Hb-diminished CSF to be conveniently eluted from the substrate. Thus, in an embodiment, the Hp in step (ii) is immobilised on a substrate. Suitable substrates will be familiar to persons skilled in the art, illustrative examples of which include a size exclusion chromatography resin and affinity chromatography resin. In an embodiment, the substrate is an affinity chromatography resin.

In an embodiment, step (ii) comprises passing the CSF from step (i) through an affinity chromatography resin under conditions that allow the cell-free Hb in the CSF to bind to the resin; wherein step (iii) comprises eluting the CSF from the resin following step (ii); and wherein step (iv) comprising recovering the eluted CSF.

Prior to administering the Hb-diminished CSF obtained from step (iii) or step (iv) to the CSF compartment of the subject in step (vi), it may be desirable to add to the Hb-diminished CSF a therapeutically effective amount of Hp in order to scavenge residual cell-free Hb that may be present in the subject's CSF compartment and could give rise to an adverse secondary neurological outcome, as described elsewhere herein. Thus, in an embodiment, the method comprises adding to the Hb-diminished CSF prior to step (v) a therapeutically effective amount of Hp, as described elsewhere herein.

Persons skilled in the art will understand that once CSF has been removed from the CSF compartment of a subject following a haemorrhagic stroke, there may be residual cell-free Hb remaining in the CSF compartment. It may therefore be desirable to rinse the CSF compartment (once the CSF has been removed in accordance with step (i), above) in order to remove at least some of the residual cell-free Hb and thereby eliminate or otherwise reduce the adverse secondary neurological outcomes to which such residual cell-free Hb may otherwise give rise. Thus, in an embodiment, the method further comprises washing the CSF compartment following step (i) with a wash solution.

Suitable wash solutions will be familiar to persons skilled in the art. In an embodiment, the wash solution is an artificial CSF, as described elsewhere herein.

In an embodiment, the wash solution further comprises Hp. In an embodiment, the wash solution comprises from about 2 µM to about 20 mM Hp. In an embodiment, the wash solution comprises from about 2 µM to about 5 mM Hp. In an embodiment, the wash solution comprises from about 100 µM to about 5 mM Hp. In an embodiment, the wash solution comprises from about 2 µM to about 300 µM Hp. In an embodiment, the wash solution comprises from about 5 µM to about 50 µM Hp. In an embodiment, the wash solution comprises from about 10 µM to about 30 µM Hp.

In an embodiment, the wash solution comprises at least an equimolar amount of Hp to the concentration of cell-free Hb in the CFS of the subject following the haemorrhage. In another embodiment, the wash solution comprises from about 3 µM to about 300 µM Hp.

In some embodiments, it may be desirable to forego steps of extracorporeally removing cell-free Hb from the CSF of a subject, as described herein, and instead replace the subject's CSF with an artificial CSF, as described elsewhere herein. Thus, in an embodiment, the method further comprises:
(i) removing CSF from the subject following the haemorrhage;
(ii) rinsing the CSF compartment of the subject following step (i) with a wash solution comprising a therapeutically effective amount of Hp;
(iii) optionally repeating step (ii); and
(iv) administering to the CSF compartment of the subject, following step (ii) or step (iii), an artificial CSF.

By incorporating a therapeutically effective amount of Hp into the wash solution, residual cell-free Hb in the CSF compartment can be complexed with the Hp, thereby neutralising the adverse biological effect of cell-free Hb on brain tissue.

### Artificial CSF

In another aspect disclosed herein, there is provided an artificial cerebral spinal fluid (CSF) comprising Hx, as herein described.

Artificial cerebrospinal fluid (aCSF) is typically a fluid that mimics natural CSF, including by salt content. Suitable compositions of aCSF will be familiar to persons skilled in the art, illustrative examples of which are described in US 20060057065 and Matzneller et al. (Pharmacology, 2016; 97(5-6):233-44, the contents of which are incorporated herein by reference in their entirety). The aCSF may comprise NaCl at a similar concentration to that found in natural CSF, as will be familiar to persons skilled in the art, and would typically include concentrations within about 15%, more preferably within about 10% of the concentration of NaCl in natural CSF. The aCSF may comprise NaHCO₃ at a similar concentration to that found in natural CSF, as will be familiar to persons skilled in the art, and would typically include concentrations within about 15%, more preferably within about 10% of the concentration of NaHCO₃ in natural CSF. The aCSF may comprise KCI at a similar concentration to that found in natural CSF, as will be familiar to persons skilled in the art, and would typically include concentrations within about 15%, more preferably within about 10% of the concentration of KCI in natural CSF. The aCSF may comprise NaH₂PO₄ at a similar concentration to that found in natural CSF, as will be familiar to persons skilled in the art, and would typically include concentrations within about 15%, more preferably within about 10% of the concentration of NaH₂PO₄ in natural CSF. The aCSF may comprise MgCl₂ at a similar concentration to that found in natural CSF, as will be familiar to persons skilled in the art, and would typically include concentrations within about 15%, more preferably within about 10% of the concentration of MgCl₂ in natural CSF. The aCSF may comprise glucose at a similar concentration to that found in natural CSF, as will be familiar to persons skilled in the art, and would typically include concentrations within about 15%, more preferably within about 10% of the concentration of glucose in natural CSF. Alternatively, the artificial CSF may omit glucose so as to reduce the likelihood of bacterial growth in any catheter used to administer the aCSF to a subject.

In an embodiment, the artificial CSF comprises from about 2 µM to about 80 mM Hx. In an embodiment, the artificial CSF comprises from about 2 µM to about 20 mM Hx. In an embodiment, the artificial CSF comprises from about 100 µM to about 20 mM Hx. In an embodiment, the artificial CSF comprises from about 2 µM to about 1,200 µM Hx. In an embodiment, the artificial CSF comprises from about 5 µM to about 200 µM Hx. In an embodiment, the artificial CSF comprises from about 10 µM to about 120 µM Hx.

In an embodiment, the artificial CSF comprises at least an equimolar amount of Hx to the concentration of cell-free heme in the CFS of the subject following the haemorrhage. In another embodiment, the artificial CSF comprises from about 3 µM to about 1,200 µM Hx.

In an embodiment, the artificial CSF further comprises Hp. In an embodiment, the artificial CSF comprises from about 2 µM to about 20 mM Hp. In an embodiment, the artificial CSF comprises from about 2 µM to about 5 mM Hp. In an embodiment, the artificial CSF comprises from about 100 µM to about 5 mM Hp. In an embodiment, the artificial CSF comprises from about 2 µM to about 300 µM Hp. In an embodiment, the artificial CSF comprises from about 5 µM to about 50 µM Hp. In an embodiment, the artificial CSF comprises from about 10 µM to about 30 µM Hp.

In an embodiment, the artificial CSF comprises at least an equimolar amount of Hp to the concentration of cell-free Hb in the CFS of the subject following the haemorrhage. In another embodiment, the artificial CSF comprises from about 3 µM to about 300 µM Hp.

In an embodiment, the artificial CSF comprises from about 2 µM to about 80 mM Hx and from about 2 µM to about 20 mM Hp. In an embodiment, the artificial CSF comprises from about 2 µM to about 20 mM Hx and from about 2 µM to about 5 mM Hp. In an embodiment, the artificial CSF comprises from about 100 µM to about 20 mM Hx and from about 2 µM to about 5 mM Hp. In an embodiment, the artificial CSF comprises from about 2 µM to about 1,200 µM Hx and from about 2 µM to about 300 mM Hp. In an embodiment, the artificial CSF comprises from about 5 µM to about 200 µM Hx and from about 2 µM to about 50 mM Hp. In an embodiment, the artificial CSF comprises from about 10 µM to about 120 µM Hx and from about 2 µM to about 30 mM Hp.

### Pharmaceutical compositions

In another aspect disclosed herein, there is provided a pharmaceutical composition for treating or preventing an adverse secondary neurological outcome in a subject following a haemorrhagic stroke in accordance with the methods described herein, the composition comprising a therapeutically effective amount of Hx, as described herein, and a pharmaceutically acceptable carrier.

In another aspect disclosed herein, there is provided a pharmaceutical composition for use in treating or preventing an adverse secondary neurological outcome in a subject following a haemorrhagic stroke in accordance with the methods described herein, the composition comprising a therapeutically effective amount of Hx, as described herein, and a pharmaceutically acceptable carrier.

In an embodiment, the composition comprises from about 2 µM to about 80 mM Hx. In an embodiment, the composition comprises from about 2 µM to about 20 mM Hx. In an embodiment, the composition comprises from about 100 µM to about 20 mM Hx, or a functional analogue thereof. In an embodiment, the composition comprises from about 2 µM to about 1,200 µM Hx. In an embodiment, the composition comprises from about 5 µM to about 200 µM Hx. In an embodiment, the composition comprises from about 10 µM to about 120 µM Hx.

In an embodiment, the composition comprises at least an equimolar amount of Hx to the concentration of cell-free heme in the CFS of the subject following the haemorrhage. In another embodiment, the composition comprises from about 3 µM to about 1,200 µM Hx.

In an embodiment, the composition further comprises a therapeutically effective amount of Hp, as described herein.

In an embodiment, the composition comprises from about 2 µM to about 20 mM Hp. In an embodiment, the composition comprises from about 2 µM to about 5 mM Hp. In an embodiment, the composition comprises from about 100 µM to about 5 mM Hp. In an embodiment, the composition comprises from about 2 µM to about 300 µM Hp. In an embodiment, the composition comprises from about 5 µM to about 50 µM Hp. In an embodiment, the composition comprises from about 10 µM to about 30 µM Hp.

In an embodiment, the composition comprises at least an equimolar amount of Hp to the concentration of cell-free Hb in the CFS of the subject following the haemorrhage. In another embodiment, the composition comprises from about 3 µM to about 300 µM Hp.

In an embodiment, the composition comprises from about 2 µM to about 80 mM Hx and from about 2 µM to about 20 mM Hp. In an embodiment, the composition comprises from about 2 µM to about 20 mM Hx and from about 2 µM to about 5 mM Hp. In an embodiment, the composition comprises from about 100 µM to about 20 mM Hx and from about 2 µM to about 5 mM Hp. In an embodiment, the composition comprises from about 2 µM to about 1,200 µM Hx and from about 2 µM to about 300 mM Hp. In an embodiment, the composition comprises from about 5 µM to about 200 µM Hx and from about 2 µM to about 50 mM Hp. In an embodiment, the composition comprises from about 10 µM to about 120 µM Hx and from about 2 µM to about 30 mM Hp.

In another aspect disclosed herein, there is provided use of a therapeutically effective amount of Hx, as described herein, in the manufacture of a medicament for treating or preventing an adverse secondary neurological outcome in a subject following a haemorrhagic stroke in accordance with the methods described herein.

In an embodiment, the pharmaceutical compositions disclosed herein are formulated for intrathecal administration. Suitable intrathecal delivery systems will be familiar to persons skilled in the art, illustrative examples of which are described by Kilburn et al. (2013, Intrathecal Administration. In: Rudek M., Chau C., Figg W., McLeod H. (eds) Handbook of Anticancer Pharmacokinetics and Pharmacodynamics. Cancer Drug Discovery and Development. Springer, New York, NY), the contents of which are incorporated herein by reference in their entirety.

In another embodiment, the pharmaceutical compositions disclosed herein are formulated for intracranial administration. Suitable intrathecal delivery systems will be familiar to persons skilled in the art, illustrative examples of which are described by Upadhyay et al. (2014, PNAS, 111(45):16071-16076), the contents of which are incorporated herein by reference in their entirety.

In another embodiment, the pharmaceutical compositions disclosed herein are formulated for intracerebroventricular administration. Suitable intrathecal delivery systems will be familiar to persons skilled in the art, illustrative examples of which are described by Cook et al. (2009, Pharmacotherapy. 29(7):832-845), the contents of which are incorporated herein by reference in their entirety.

Suitable pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

### Kits

In another aspect disclosed herein, there is provided a kit comprising the artificial CSF, as described herein, or the pharmaceutical composition, as described herein. The active agents, as herein described (including Hx and, optionally, Hp) may be presented in the form of a kit of components adapted for allowing concurrent, separate or sequential administration of the active agents. Each carrier, diluent, adjuvant and/or excipient must be "pharmaceutically acceptable" insofar as it is compatible with the other ingredients of the composition and physiologically tolerated by the subject. The compositions may conveniently be presented in unit dosage form and may be prepared by methods well known in the art of pharmacy. Such methods include the step of bringing into association the active ingredient with the carrier, which constitutes one or more accessory ingredients. In general, the compositions are prepared by uniformly and intimately bringing into association the active ingredient with liquid carriers, diluents, adjuvants and/or excipients or finely divided solid carriers or both, and then if necessary shaping the product.

### Methods of diagnosis

As described elsewhere herein, the present inventors have surprisingly found that the concentration of ventricular CSF-Hb can be used to determine whether a subject is at risk of developing an adverse secondary neurological outcome following a haemorrhagic stroke.

Thus, in another aspect disclosed herein, there is provided a method of determining whether a subject is at risk of developing an adverse secondary neurological outcome following a haemorrhagic stroke accompanied by extravascular erythrolysis and release of cell-free heme and/or cell-free haemoglobin (Hb) into a cerebral spinal fluid (CSF), the method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).

Suitable methods of measuring the amount of cell-free Hb in the CSF sample will be known to persons skilled in the art, illustrative examples of which are described elsewhere herein.

In an embodiment, the "reference value" is a value that is representative of an amount of cell-free Hb in the CSF of a healthy subject, or population of healthy subjects. In this context, an amount of cell-free Hb in a CSF sample from a test subject that is greater than the reference value is indicative that the subject is at risk of developing an adverse secondary neurological outcome following a haemorrhagic stroke. Conversely, an amount of cell-free Hb in a CSF sample from a test subject that is equal to or lower than the reference value is indicative that the subject is not at risk of developing an adverse secondary neurological outcome following a haemorrhagic stroke.

In an embodiment, the "reference value" is a value that is representative of an amount of cell-free Hb in the CSF of a subject, or a population of subjects, who have had an adverse secondary neurological outcome following a haemorrhagic stroke. In this context, an amount of cell-free Hb in a CSF sample from a test subject that is equivalent to or greater than the reference value is indicative that the subject is at risk of developing an adverse secondary neurological outcome following a haemorrhagic stroke. Conversely, an amount of cell-free Hb in a CSF sample from a test subject that is lower than the reference value is indicative that the subject may not be at risk of developing an adverse secondary neurological outcome following a haemorrhagic stroke.

In an embodiment, the reference value is substantially zero, meaning there is no detectable physiological cell-free Hb in the CSF. In another embodiment, the reference value is representative of a level of cell-free Hb in the CSF of one or more patients with haemorrhagic stroke but no adverse neurological outcome. In this context, it is to be understood that, in some instances, there might be a threshold level of cell-free Hb in CSF that can be tolerated without an adverse neurological outcome.

In an embodiment, the subject is determined to be at risk of developing adverse secondary neurological outcome where the amount of cell-free Hb in the CSF sample determined in step (ii) is at least about 7 µM.

In an embodiment, the adverse secondary neurological outcome is angiographic vasospasm and the subject is determined to be at risk of developing angiographic vasospasm where the amount of cell-free Hb in the CSF sample determined in step (ii) is at least about 3 µM.

In an embodiment, the adverse secondary neurological outcome is delayed cerebral ischemia and the subject is determined to be at risk of developing delayed cerebral ischemia where the amount of cell-free Hb in the CSF sample determined in step (ii) is at least about 5 µM.

In an embodiment, the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.

By "at least about 7 µM" includes at least about 7 µM, at least about 8 µM, at least about 9 µM, at least about 10 µM, at least about 15 µM, at least about 20 µM, at least about 25 µM, at least about 30 µM, at least about 35 µM, at least about 40 µM, at least about 45 µM, at least about 50 µM, and so on. In an embodiment, the subject is determined to be at risk of developing an adverse secondary neurological outcome following a haemorrhagic stroke where the amount of cell-free Hb in the CSF sample determined in step (ii) is at least about 7 µM, preferably at least about 8 µM, preferably at least about 9 µM, preferably at least about 10 µM, preferably at least about 15 µM, preferably at least about 20 µM, preferably at least about 25 µM, preferably at least about 30 µM, preferably at least about 35 µM, preferably at least about 40 µM, preferably at least about 45 µM, or more preferably at least about 50 µM.

Similarly, by "at least about 3 µM" includes at least about 3 µM, at least about 4 µM, at least about 5 µM, at least about 6 µM, at least about 7 µM, at least about 8 µM, at least about 9 µM, at least about 10 µM, at least about 15 µM, at least about 20 µM, at least about 25 µM, at least about 30 µM, at least about 35 µM, at least about 40 µM, at least about 45 µM, at least about 50 µM, and so on. In an embodiment, the subject is determined to be at risk of developing an adverse secondary neurological outcome following a haemorrhagic stroke where the amount of cell-free Hb in the CSF sample determined in step (ii) is at least about 3 µM, at least about 4 µM, at least about 5 µM, at least about 6 µM, at least about 7 µM, preferably at least about 8 µM, preferably at least about 9 µM, preferably at least about 10 µM, preferably at least about 15 µM, preferably at least about 20 µM, preferably at least about 25 µM, preferably at least about 30 µM, preferably at least about 35 µM, preferably at least about 40 µM, preferably at least about 45 µM, or more preferably at least about 50 µM.

The methods of determining risk of developing an adverse secondary neurological outcome following a haemorrhagic stroke, as described herein, advantageously allow a subject to be stratified to an appropriate treatment regimen, having regard to their determined risk. For example, if a subject is determined to be at risk of developing an adverse secondary neurological outcome following a haemorrhagic stroke, the subject can be stratified to the methods of treatment described herein. Conversely, if a subject is determined not to be at risk of developing an adverse secondary neurological outcome following a haemorrhagic stroke, the subject can be stratified to either no treatment, or an alternative and less invasive treatment regimen.

Methods of determining risk may suitably comprise using receiver operating characteristic (ROC) curves (*e.g.,* ROCR package (v1.0-11) (Sing *et al.,* 2005), plotROC package (v2.2.1) (Sachs, 2017)), the corresponding bootstrap area under the curves (AUC)(pROC package (v1.16.2) (Robin *et al.,* 2011)) and / or the optimal Youden index (ROCit package (v1.1.1) (Md Riaz Ahmed, 2019)). Illustrative examples of methods of determining risk of developing an adverse secondary neurological outcome following a haemorrhagic stroke are also described elsewhere herein.

In an embodiment, the method further comprises treating the subject determined to be at risk of an adverse secondary neurological outcome, wherein said treatment comprises exposing the CSF of the subject to (i) a therapeutically effective amount of hemopexin (Hx) and for a period of time sufficient to allow the Hx to form a complex with, and thereby neutralise, the cell-free heme, as described herein; and / or (ii) a therapeutically effective amount of haptroglobin (Hp) and for a period of time sufficient to allow the Hp to form a complex with, and thereby neutralise, the cell-free Hb, as described herein.

In another aspect disclosed herein, there is provided a method of stratifying a subject to a treatment for an adverse secondary neurological outcome in a subject following a haemorrhagic stroke accompanied by extravascular erythrolysis and release of cell-free heme and/or cell-free haemoglobin (Hb) into a cerebral spinal fluid (CSF), wherein the method comprises (a) determining whether a subject is at risk of developing an adverse secondary neurological outcome, as herein described, and (b) treating the subject determined to be at risk of an adverse secondary neurological outcome in accordance with the methods described herein.

Those skilled in the art will appreciate that the invention described herein is susceptible to variations and modifications other than those specifically described. It is to be understood that the invention includes all such variations and modifications which fall within the spirit and scope. The invention also includes all of the steps, features, compositions and compounds referred to or indicated in this specification, individually or collectively, and any and all combinations of any two or more of said steps or features.

Certain embodiments of the invention will now be described with reference to the following examples which are intended for the purpose of illustration only and are not intended to limit the scope of the generality hereinbefore described.

### EXAMPLES

### Example 1 : Ventricular CSF Hb as a biomarker of SAH-SBI

This study received ethics approval before commencement. Written informed consent was obtained from all patients or their legal representatives. All experiments were performed in conformity with the biosafety regulations and in appropriate working conditions. The results are reported in accordance with the STROBE statement (von Elm *et al.,* 2007).

### Study population

Clinical data and CSF samples were obtained from a prospective consecutive patient cohort with aSAH admitted to the neurointensive care unit of an academic tertiary care center over a 3 year period (April 2017 to March 2020). The eligibility criteria comprised: (i) age > 18 years; (ii) radiological diagnosis of a subarachnoid hemorrhage; (iii) exclusion of non-aneurysmal subarachnoid hemorrhage (*e.g.,* trauma, perimesencephalic subarachnoid hemorrhage); (iv) insertion of an external ventricular drain (EVD) as part of standard of care.

### Clinical data acquisition

Patient data collection was performed independent and blinded to CSF sampling. Baseline features were assessed at the time of diagnosis, including demographic characteristics (age, gender), clinical scores and measures (WFNS grade, Hunt & Hess grade, initial glascow coma scale (GCS)) and radiological assessments (aneurysm location, maximal aneurysm diameter, hematoma volume (see below), presence of intraventricular hemorrhage (IVH), Fisher grading, BNI grading) (Figure 7A). Clinical data was collected prospectively during the 14-day high-risk phase for SAH-SBI based on a standardized multimodal monitoring at the neurointensive care unit and as part of the 3-months follow-up (Figure 7A). General patient management conformed to current international guidelines (Diringer *et al.,* 2011; Connolly *et al.,* 2012). The presence of DIND, DCI or aVSP was assessed on a daily basis. DIND was defined as a new focal neurological deficit or a decrease in GCS of at least 2 points for at least 2 hours not attributable to any other identifiable cause such as hydrocephalus, epileptic seizures or systemic factors. In case the patient could not be clinically assessed (*e.g.,* sedation), clinical data was classified as non-available (NA). DCI was defined as a new ischemia or new infarction on CT/CTP or MRI not attributable to surgical intervention. The definition of aVSP comprised a new narrowing of cerebral arteries based on a digital subtraction angiography (DSA), computed 6 tomography angiography (CTA) of magnetic resonance angiography (MRA). In the absence of an appropriate imaging procedure on the respective day, this was noted as NA. SAH-SBI was defined as the composite outcome of DIND, DCI and aVSP. In addition, flow velocity over both middle, anterior and posterior cerebral arteries was assessed daily using TCD.

Complications (meningoencephalitis, cerebral salt wasting, diabetes insipidus, pneumonia, Takotsubo cardiomyopathy, non-convulsive status epilepticus, chronic hydrocephalus, death) and functional status (Glasgow Outcome Scale Extended (GOSE) and modified Rankin Scale (mRS)) were evaluated up to the 3-months follow-up.

### Subarachnoid hematoma segmentation and volumetric analysis

Based on the initial CT scan, the volume of the subarachnoid hematoma was manually delineated using 3DSlicer (Kikinis *et al.,* 2014). This was performed in 46 of the 47 subjects, as one had an initial MRI only and was therefore excluded from this analysis. Preprocessing prior to delineation consisted of brain extraction. The biggest cavity from a 200 HU thresholded skull mask was extracted (Weidert *et al.,* 2020). Consequently, the brain mask was multiplied with the original CT scan and median filtered with a 5x5x3 kernel (Figure 7B). The volume of the hemorrhage was determined as the multiplication of the voxel size by the number of segmented voxels (Figure 2C).

### CSF sampling

CSF samples were collected daily from the EVD, starting after aneurysm repair. A sampling duration between day 1 (day after the bleeding) and day 14 was intended. CSF collection was performed blinded to clinical data. Immediately after sample collection, the CSF was centrifuged at 1500 G for 15 minutes (Capricorn CEP 2000 Benchtop centrifuge, Capricorn labs, UK) and the supernatant was stored at -80°C for further analysis.

### Spectrophotometry of CSF Hb and heme metabolites

Absorption spectra in the visual range between 350 and 650 nm of all CSF samples were measured on a Shimadzu UV-1800 spectrophotometer (Shimadzu, Japan). Quantification of the different Hb species and their metabolites (oxyHb, metHb, bilirubin, biliverdin) was performed using spectral deconvolution. Therefore, extinction curves of the individual substances with known concentrations were fitted to the extinction curve of the CSF using a non-negative least squares algorithm as described previously (Deuel *et al.,* 2015).

### LC-MSMS CSF proteome analysis

Using a label-free proteomics quantification approach, the CSF proteome of 18 patients was characterized at five sequential time points along the observation period (0, 0.5, 1, 1.5 and 2 weeks after aSAH) (Figure 7C). The samples were processed in two batches in order to improve generalizability, which were combined for analysis after normalization (85 samples in total - 48 in batch 1, 37 in batch 2). Hb, which is abundantly present in the erythrolysed CSF, was selectively removed prior to the analysis using a haptoglobin affinity column to avoid ion-suppression and increase the sensitivity (Hugelshofer *et al.,* 2019).

Following digestion of the Hb-depleted CSF samples with trypsin, mass spectrometry analysis was performed on a Q Exactive HF mass spectrometer (Thermo Scientific) equipped with a Digital PicoView source (New Objective) and coupled to an M-Class UPLC (Waters).

### A. Sample preparation

Total protein concentration was estimated using the Qubit^{®} Protein Assay Kit (Life Technologies, Zurich, Switzerland). The samples were then prepared by using a commercial iST Kit (PreOmics Phoenix, Germany) with an updated version of the protocol. Briefly, 20 µg of protein was solubilized in 'Lyse' buffer, boiled at 95 °C for 10 minutes and processed with HIFU for 30 s, setting the ultrasonic amplitude to 85%. Afterwards, the samples were transferred to the cartridge and digested by adding 50 µl of the 'Digest' solution. After 60 minutes of incubation at 37 °C, the digestion was stopped with 100 µl of Stop solution. The solutions in the cartridge were removed by centrifugation at 3800 g, while the peptides were retained by the iST-filter. Finally, the peptides were washed, eluted, dried and resolubilized in 20 µL of buffer (3% acetonitrile, 0.1% formic acid) buffer for LC-MS analysis.

### B. Liquid chromatography-mass spectrometry analysis

Mass spectrometry analysis was performed on an Orbitrap Fusion Lumos (Thermo Scientific) equipped with a Digital PicoView source (New Objective) and coupled to a M-Class UPLC (Waters). Solvent composition at the two channels was 0.1% formic acid for channel A and 0.1% formic acid, 99.9% acetonitrile for channel B. For each sample 1 µL of diluted peptides were loaded on a commercial MZ Symmetry C18 Trap Column (100Å, 5 µm, 180 µm x 20 mm, Waters) followed by nanoEase MZ C18 HSS T3 Column (100Å, 1.8 µm, 75 µm x 250 mm, Waters). The peptides were eluted at a flow rate of 300 nL/min by a gradient from 5 to 22% B in 77 min, 32% B in 10 min and 95% B for 10 min. Samples were acquired in a randomized order. The mass spectrometer was operated in data-dependent mode (DDA) acquiring a full-scan MS spectra (300-1'500 m/z) at a resolution of 120'000 at 200 m/z after accumulation to a target value of 500'000. Data-dependent MS/MS were recorded in the linear ion trap using quadrupole isolation with a window of 0.8 Da and HCD fragmentation with 35% fragmentation energy. The ion trap was operated in rapid scan mode with a target value of 10'000 and a maximum injection time of 50 ms. Only precursors with intensity above 5'000 were selected for MS/MS and the maximum cycle time was set to 3 s. Charge state screening was enabled. Singly, unassigned, and charge states higher than seven were rejected. Precursor masses previously selected for MS/MS measurement were excluded from further selection for 20 s, and the exclusion window was set at 10 ppm. The samples were acquired using internal lock mass calibration on m/z 371.1012 and 445.1200. The mass spectrometry proteomics data were handled using the local laboratory information management system (LIMS) (Türker *et al.,* 2010).

### C. Protein identification and label free protein quantification

The acquired raw MS data were processed by MaxQuant (version 1.6.2.3), followed by protein identification using the integrated Andromeda search engine (Cox and Mann, 2008). Spectra were searched against a Swissprot Homo sapiens reference proteome (taxonomy 9606, version from 2019-07-09), concatenated to its reversed decoyed fasta database and common protein contaminants. Carbamidomethylation of cysteine was set as fixed modification, while methionine oxidation and N-terminal protein acetylation were set as variables. Enzyme specificity was set to trypsin/P allowing a minimal peptide length of 7 amino acids and a maximum of two missed-cleavages. MaxQuant Orbitrap default search settings were used. The maximum false discovery rate (FDR) was set to 0.01 for peptides and 0.05 for proteins. Label free quantification was enabled and a 2 minutes window for match between runs was applied. In the MaxQuant experimental design template, each file is kept separate in the experimental design to obtain individual quantitative values. The MaxQuant results were loaded into Scaffold (Proteome Software Inc.) to validate the peptide and protein identifications. Only proteins identified with at least 2 peptides were considered for follow up analysis.

The individual protein intensities of protein were normalized to the mean intensity of the respective protein at baseline (week 0) and log2 transformed. The overall fold change was calculated as the sum of the log-ratios for each protein over the whole time course. Further, for each time point, normalized protein intensities were compared to baseline (week 0) using a Wilcoxon rank-sum test and p-values were combined over all time points using the metap package (Dewey, 2020). The temporal changes in the CSF proteome were clustered using k-means analysis, whereby the optimal number of clusters was determined visually with an elbow diagram (Figure 7D) using the factoextra package (v1.0.7) (Kassambara and Mundt, 2020).

### Hemoglobin, haptoglobin, hemopexin and rLP

Hb used in *ex vivo* experiments was purified from expired human blood concentrates as previously described (Elmer *et al.,* 2009). Hb concentrations were determined by spectral deconvolution as described above and are given as molar concentrations of total heme (1M Hb tetramer is equivalent to 4M heme). Hx was used in equimolar concentration to the concentration of heme measured in the test sample. For all Hb used in these studies, the fraction of ferrous oxyHb (HbFe 2+O2) was always greater than 98% as determined by spectrophotometry. Purified haptoglobin (phenotype 1-1) and hemopexin from human plasma was obtained from CSL Behring.

### Neurovascular functions

The vascular function assay (Figure 7E) was performed using fresh porcine basilar arteries obtained from a local abattoir (n = 12). Vessels were pre stretched to reach 8 the optimal passive pretension as previously published (Hugelshofer *et al.,* 2020). Then, 10 µM prostaglandin F2α (PGF2α; Sigma, Buchs, Switzerland) was used as a pre-contracting agent. In order to determine the maximum possible tension in absence of endogenous NO, L-N5-(1-Iminoethyl)ornithine hydrochloride (L-NIO; Sigma-Aldrich, Saint Louis, US-MO) at a concentration of 10 µM was added at the end of the experiment. The recorded tension was normalized to the individual NO reserve capacity of the respective vessel, i.e. the baseline tension after addition of PGF2α (0%) and the tension after addition of L-NIO at the end of the experiment (100%) (Figure 7F). Damaged vessels with a continuous loss in tension over the duration of the experiment, resulting in a PGF2α max / L-NIO max ratio below 0.7, were excluded from the analysis (Figure 7F). A first series of experiments was conducted to evaluate the vasoconstrictive potential of the patient CSF in the high-risk phase for SAH-SBI after aSAH and to assess the specific role of oxyHb in this process. For this purpose patient CSF from the high-risk phase (between day 4 and 14) was selectively depleted from Hb using a haptoglobin affinity column. In the initial phase of the experiment, the vessels were immersed in the Hb-depleted CSF. Subsequently, Hb was added to the CSF in the precise amount that had been depleted previously to determine its specific impact on vascular tension. In a second series of experiments, the influence of an increasing exposure to Hb on the vascular tension was assessed. Half-log10 Hb dilution series (10⁻⁴ M through 10⁻⁸ M) in Krebs-Henseleit-Buffer and subsequent immersion of the vessels with increasing Hb concentrations were conducted. For the evaluation of the haptoglobin effect, a third series of experiments was performed with identical Hb dose escalation, except for an equimolar addition of haptoglobin.

### TBARS

The oxidative potential of CSF samples was quantified by measuring the formation of malondialdehyde (MDA) as the final product of lipid peroxidation after incubation with a well-defined reconstituted lipoprotein (rLP, obtained from CSL Behring, Bern, Switzerland) (Figure 7G). Using a 96-well plate, 25 µL CSF were mixed with 5 µL rLP (3 mg/mL) and incubated at 37°C for 24 hours. Subsequently, the concentration of MDA was measured using a thiobarbituric acid reactive substance (TBARS) assay. In short 125 µL of 750 mM trichloroacetic acid in 1 M HCI was added to the samples, followed by short vortexing and subsequent addition of 100 µL 25 mM 2-thiobarbituric acid in 1 M NaOH.

After an incubation period at 80° for 60 min, the TBARS in the supernatant were quantified. For absolute quantification of MDA, the absorption at 600 nm was subtracted from the absorption at 532 nm and the concentration was calculated with the molar extinction 9 coefficient of 0.156 mM- 1c m- 1. To achieve a more sensitive but relative quantification, the fluorescence emission was measured at 550 nm, using 510 nm as excitation wavelength.

### Statistical analysis

Statistical analysis was performed using R 3.6.3 (R Core Team, 2020). Categorical data is provided as absolute numbers (n) and proportions (%), continuous data as mean and standard deviation (SD). For factorial experiments, we performed a repeated measure ANOVA using the ez package (v4.4-0) (Lawrence, 2016). The mixed linear model analysis was performed with the Ime4 package (v1.1-23) (Bates *et al.,* 2015). For gene set enrichment analysis we used gene set enrichment analysis, GSEA software from the broad institute (Subramanian et *al.,* 2005), and the hallmark gene sets of the Molecular Signature Database (MSigDB) (Liberzon *et al.,* 2015). As input, we used a ranked list of the identified proteins, where the rank score was calculated as the product of -log10(p-value) and the sum(log-ratio) of the protein. The associations between baseline measures (aneurysm location, aneurysm size, hematoma volume, presence of IVH) and CSF-Hb, as well as between CSF-Hb and SAH-SBI were analyzed using a generalized additive models (GAM) with spline-fit for the day after aSAH and random effect for patient ID using the mgcv package (v1.8-31) (Wood, 2017). The sensitivity and specificity of CSF oxyHb for SAH-SBI was analyzed using receiver operating characteristic (ROC) curves (ROCR package (v1.0-11) (Sing *et al.,* 2005), plotROC package (v2.2.1) (Sachs, 2017)), the corresponding bootstrap area under the curves (AUC)(pROC package (v1.16.2) (Robin *et al.,* 2011)) and the optimal Youden index (ROCit package (v1.1.1) (Md Riaz Ahmed, 2019)). TCD flow velocities were first used categorically, according to their clinical assessment (pathological or non-pathological), which served to determine sensitivity and specificity. Second, as *TCD scaled,* they were used continuously for pathophysiological association with the measured CSF Hb, each value being scaled to the mean flow over the corresponding vessel (anterior, middle or posterior cerebral artery) of the cohort. The *max TCD scaled* represented the maximum scaled value measured daily. Due to the exploratory character of the study, no level of statistical significance was defined, instead the results were interpreted based on the level of evidence: p < 0.001: very strong evidence; p < 0.01: strong evidence; p < 0.05 evidence; p < 0.1 weak evidence; p > 0.1: no evidence (Bland, 2015).

### Results

### A. Study population

### Out of 52 consecutively screened patients fulfilling the eligibility criteria, 47 were included.

Five patients or their legal representatives refused informed consent. Table 1 summarizes the baseline features of the patient cohort.

**Table 1**

| | | | | |
|---|---|---|---|---|
| **n** | | 47 | **WFNS grade** [n (%)] | |
| **Age** [mean (SD)] | | 60.1 (12.9) | 1 | 5 (10.6) |
| **Male gender** [n (%)] | | 19 (40.4) | 2 | 12 (25.5) |
| **Aneurysm location** [n (%)] | | | 3 | 2 (4.3) |
| | Anterior communicating artery | 19 (40.4) | 4 | 10 (21.3) |
| | Basilar artery | 6 (12.8) | 5 | 18 (38.3) |
| | Internal carotid artery | 1 (2.1) | **Hunt &Hess grade** [n (%)] | |
| | Middle cerebral artery | 8 (17.0) | 1 | 1 (2.1) |
| | Posterior communicating artery | 6 (12.8) | 2 | 13 (27.7) |
| | Pericallosal artery | 2 (4.3) | 3 | 7 (14.9) |
| | Posterior Inferior cerebellar artery | 4 (8.5) | 4 | 10 (21.3) |
| | Vertebral artery | 1 (2.1) | 5 | 16 (34.0) |
| **Aneurysm size*** [mean (SD)] | | 6.9 (4.4) | **BNI grade** [n (%)] | |
| **Blood volume**** [mean (SD)] | | 66.8 (40.6) | 2 | 5 (10.6) |
| **Intraventricular hemorrhage** [n (%)] | | 20 (42.6) | 3 | 7 (14.9) |
| **Initial GCS** [mean (SD)] | | 8.9 (4.8) | 4 | 13 (27.7) |
| **Treatment** [n (%)] | | | 5 | 22 (46.8) |
| | Bypass | 2 (4.3) | **Fisher grade** [n (%)] | |
| | Clipping | 14 (29.8) | 1 | 1 (2.1) |
| | Coillng | 31 (66.0) | 3 | 26 (55.3) |
| | | | 4 | 20 (42.6) |

### B. Spectrophotometry of CSF-Hb and heme-metabolites

Spectrophotometry with spectral deconvolution was used to quantify cell-free oxyHb (ferrous HbFe 2+) and its downstream metabolites biliverdin and bilirubin, as well as the primary Hb oxidation product metHb (ferric HbFe 3+). Individual temporal profiles and peak concentrations of oxyHb (0.6 µM to 242.2 µM) were highly variable (Figure 8). In most patients oxyHb remained very low during the first 2 to 3 days after aSAH, followed by a significant increase, plateauing between days 9 and 12, and decreasing after that (Figure 1A). Bilirubin was elevated already on day 1, exhibiting the most significant increase within the first 3 to 5 days before it reached a plateau (Figure 1A). This time course reflects phagocytosis and heme-metabolism by macrophages in the subarachnoid space. The intermediate metabolite biliverdin begins to rise markedly after day 4 and reaches its peak on day 12 (Figure 1A). Also, metHb followed a delayed increase with a peak 11 days after aSAH consistent with slow autoxidation of cell-free oxyHb in the CSF (Figure 1A).

### C. LC-MSMS CSF proteome analysis

Quantitative LC-MS/MS analysis was performed on 85 CSF samples from 18 patients collected at 5 time-points post bleeding. Figure 1B illustrates the temporal course of 757 CSF proteins that were assigned to four groups by k-means clustering. Figure 1C shows a volcano plot of the normalized signal intensities summed across all samples per patient providing an overall view on protein accumulation or depletion within the two week period after bleeding. Cluster 1 (green) comprised proteins that remained unchanged over time. Cluster 4 (yellow) represents proteins with a decreasing intensity over time. These were mainly plasma proteins that entered the subarachnoid space by the initial bleeding and were subsequently cleared from the CSF. The gene set enrichment analysis (GSEA) of the ranked proteins using the hallmark gene sets of the Molecular Signature Database (MSigDB) (Liberzon *et al.,* 2015), assigned the top negative enrichment to coagulation (ES = -0.66, FDR = 0.007, Figure 1D). Figure 1E displays the temporal courses of four selected plasma proteins (HP, HPR, HPX and ALB). Cluster 2 (violet) contained proteins with a 12 pronounced accumulation over time, and cluster 3 (blue) proteins with a moderate increase.

The GSEA identified glycolysis (ES = 0.78, FDR = 0.016, Figure 1F) and heme metabolism (ES = 0.74, FDR = 0.096, Figure 1H) as the top enriched hallmarks, representing lysis of red blood cells (RBC) with release of cytoplasmic proteins and breakdown of Hb by macrophages, respectively. Figure 1G displays the time course of the RBC proteins carboanhydrase 1 (CA1) and 2 (CA2), catalase (CAT), and aldolase A (ALDOA). Figure 1I shows the macrophage proteins CD163, CSF1R, CD14, and TIMP-1. Accumulation of these proteins points to a pronounced macrophage influx into the subarachnoid space within the first days after hemorrhage. Collectively, the proteome dynamics in patient CSF after aSAH reinforced that erythrolysis as well as an adaptive macrophage response are the two dominant processes occurring in the CSF space after aSAH.

### D. Determinants of CSF-Hb levels

A generalized additive model (GAM) was applied with spline-fit for the day after aSAH and a random effect for the patient ID to estimate determinants of CSF-Hb concentrations. The partial dependence plots are given in Figure 2B. The size of the aneurysm did not seem to be associated with the course of CSF-Hb. Among the different aneurysm locations, only middle cerebral artery aneurysms had an association with Hb levels, which was negative (p = 0.0018). The volume of the hematoma demonstrated a strong association with CSF-Hb levels. The day after aSAH has a negative association in the first 3-5 days, followed by a weakly positive association in the subsequent days. This association most likely reflects the delayed onset of erythrolysis and CSF-Hb generation in the subarachnoid space. The presence of IVH was associated with higher levels of CSF-Hb (p < 0.0001). With IVH, CSF-Hb showed an early increase to a plateau, whereas in the absence of IVH the increase was more delayed and gradual but leveling at comparable peak concentrations around day 12 (Figure 2C). This is most likely a CSF compartment effect (Figure 2D).

### E. Increased CSF-Hb is associated with secondary brain injury

The cohort outcome parameters representative of SAH-SBI (patient-wise and day-wise), rescue therapies, complications, mortality, and functional status at 3-months follow-up are summarized in Table 3. The CSF-Hb levels stratified by SAH-SBI are shown in Figure 3A and Figure 9. Based on a GAM with spline-fit for the day after aSAH and a random effect for patient ID, we found very strong evidence (p = 0.00045) for an association between the occurrence of SAH-SBI and the CSF-Hb concentration. Figure 3B gives the partial dependence of SAH-SBI on CSF-Hb and the day post SAH. To estimate the power for daily CSF-Hb as a monitoring marker for SAH-SBI, the respective receiver operating characteristic (ROC) curves and corresponding areas under the ROC curve (AUC) were calculated for DIND, DCI, aVSP (Figure 9A-C, Table 2) and the composite outcome SAH-SBI (Figure 3C, Table 2). The high diagnostic accuracy remained unaltered in a recalculated model with data limited to the high-risk period (day 4-14) avoiding a potential bias introduced by the generally low Hb levels in the first days after bleeding (Figure 9D). Bilirubin, biliverdin and methemoglobin had a lower diagnostic accuracy than oxyHb (Figure 9E-G). Computation of the optimal Youden index yielded a CSF-Hb value of 7.1 µM for SAH-SBI (Figure 3C). For the individual outcomes aVSP, DCI and DIND, the optimal Youden indices were 3.4, 5.5, and 7.1 µM, respectively (Figure 9A-C).

**Table 2**

| **Parameter/Score** | **AUC (95% CI) or Sensitivity / Specificity** | | | |
|---|---|---|---|---|
| **Current study** | **SAH-SBI** | **DIND** | **DCI** | **aVSP** |
| **Hunt and Hess score** | **0.59 (0.53-0.65)** | **0.64 (0.57-0.7)** | **0.5 (0.4-0.8)** | **0.49 (0.39-0.59)** |
| **WFNS score** | **0.59 (0.53-0.65)** | **0.64 (0.57-0.7)** | **0.5 (0.4-0.8)** | **0.49 (0.39-0.59)** |
| **modified Fisher score** | **0.59 (0.53-0.65)** | **0.64 (0.57-0.7)** | **0.5 (0.4-0.6)** | **0.49 (0.39-0.59)** |
| **BNI** | **0.59 (0.53-0.65)** | **0.64 (0.57-0.7)** | **0.5 (0.4-0.6)** | **0.49 (0.39-0.59)** |
| **TCD** | **0.28 (0.17, 0.42) / 0.97 (0.88, 1.00)** | **0.32 (0.18, 0.48) / 0.96 (0.87, 1.00)** | **0.15 (0.03, 0.38) / 0.93 (0.76, 0.99)** | **0.17 (0.05, 0.37) / 0.96 (0.78, 1.00)** |
| **CSF-Hb** | **0.89 (0.85-0.92)** | **0.89 (0.84-0.92)** | **0.88 (0.81-0.93)** | **0.89 (0.82-0.93)** |

| **Published literature** | | | | |
|---|---|---|---|---|
| **Hunt and Hess score (Hunt and Hess, 1968)** | **0.67** | | | |
| **WFNS score (Teasdale *et al*., 1988)** | **0.67** | | | |
| **modified Fisher score (Frontera *et al.*, 2006)** | **0.61** | | | |
| **BNI (Wilson *et al*., 2012)** | **0.62** | | | |
| **VASOGRADE score (Dengler *et al*., 2018)** | **0.67** | | | |
| **HAIR score (Dengler *et al*., 2018)** | **0.74** | | | |
| **(Lee *et al*., 2018)** | **0.73** | | | |
| **EDGI (Fang *et al.,* 2019)** | **0.78** | | | |
| **(Roederer *et al*., 2014)** | **0.71** | | | |
| **TCD (Sloan *et al*., 1989)** | **0.59 / 1.0** | | | |
| **Blood tissue kallikrein (Bian *et al*., 2020)** | **0.81** | | | |
| **Blood calprotectin (S100A8/A9) (Wang *et al*., 2020)** | **0.78** | | | |
| **Blood IL-6 (Murol *et al*., 2013)** | **0.68** | | | |
| **Blood white blood cell count (Al-Mufti *et al*., 2019)** | **0.63** | | | |
| **CSF tenascin-C (Suzuki *et al*., 2011)** | **0.81 / 0.79** | | | |
| **CS neuropeptide Y (Schebesch *et al.*, 2013)** | **0.82 / 0.72** | | | |

**Table 3**

| **Total n** | **Patient-wise (%) 47 patients** | | **Day-wise (%) 415 samples** | |
|---|---|---|---|---|
| **ICV-rtPA** | 5 (10.6) | | - | |
| **Spasmolysis** | 7 (14.9) | | - | |
| **Meningoencephalitis** | 1 (2.1) | | - | |
| **Cerebral salt wasting** | 19 (40.4) | | - | |
| **Diabetes insipidus** | 9 (19.1) | | - | |
| **Pneumonia** | 34 (72.3) | | - | |
| **TakoTsubo cardiomyopathy** | 6 (12.8) | | - | |
| **NCSE** | 4 (10.3) | | - | |
| **Chronic hydrocephalus** | 24 (53.3) | | - | |
| **Death** | 14 (30.4) | | - | |

| | | *absent* | *present* | *NA* |
|---|---|---|---|---|
| **aVSP** | 23 (48.9) | 61 (14.7) | 66 (15.9) | 288 (69.4) |
| **DCI** | 21 (44.7) | 75 (18.1) | 54 (13.0) | 286 (68.9) |
| **DIND** | 30 (63.8) | 149 (35.9) | 110 (26.5) | 156 (37.6) |
| **SAH-SBI** | 31 (66.0) | 174 (41.9) | 138 (33.3) | 103 (24.8) |

| **GOSE (at 3 months)** | | | | |
|---|---|---|---|---|
| 1 | 14 (30.4) | | - | |
| 2 | 1 (2.2) | | - | |
| 3 | 10 (21.7) | | - | |
| 4 | 4 (8.7) | | - | |
| 5 | 7 (15.2) | | - | |
| 6 | 2 (4.3) | | - | |
| 7 | 3 (6.5) | | - | |
| 8 | 5 (10.9) | | - | |

| **mRS (at 3 months)** | | | | |
|---|---|---|---|---|
| 0 | 1 (2.2) | | - | |
| 1 | 6 (13.0) | | - | |
| 2 | 8 (17.4) | | - | |
| 3 | 6 (13.0) | | - | |
| 4 | 5 (10.9) | | - | |
| 5 | 6 (13.0) | | - | |

Given their frequent use in clinical practice (Diringer *et al.,* 2011; Connolly *et al.,* 2012), the diagnostic accuracy of clinical and radiological scores was assessed, as well as daily TCD measurements in the patient cohort. Clinical and radiological scores demonstrated poor predictive qualities (Table 2, Figure 9H-K). TCD measurements demonstrated a high specificity for SAH-SBI, aVSP, DCI and DIND. The sensitivity of TCD, however, was minimal (Table 2).

High CSF-Hb levels were also associated with poor clinical outcome expressed by the GOSE score (p = 0.0056, Figure 3D) and the mRS score (p = 0.0016, Figure 9L) at 3 months follow-up.

### F. Basilar artery vasoconstriction and lipid peroxidation occur in the concentration range of patient CSF-Hb

The data suggest that cell-free Hb is an upstream mediator of SAH-SBI. To enhance the plausibility of this association, *ex vivo* vasoconstriction and lipid peroxidation was examined with Hb exposures matching the range of CSF-Hb concentrations in the patient cohort. A new vascular function model with porcine basilar arteries that were precontracted with PGF2α was established and dilated by an intrinsic endothelial NOS (eNOS) response at the end of experiment. This setup allowed an investigation into the effect of Hb on the endogenous NO reserve capacity. No supplemental NO donor was used.

Figure 4A demonstrates the sigmoid dose-response curve with a steep increase of vascular tension between 10^{-6.5} and 10⁻⁶ M oxyHb, and reaching maximum contraction at 10⁻⁵ M oxyHb. An almost identical curve was obtained when we plotted the max-scaled patient TCD values against categories of CSF-Hb (Figure 4B). The CSF-Hb categories were selected to match the concentration-steps used in the ex vivo vasoconstriction assay. Patients with aVSP (red dots) mostly had CSF-Hb of 10 -5 M or more. We have also measured the generation of TBARS in a mixture of oxyHb with a reconstituted lipoprotein containing unsaturated phosphatidylcholine, which is considered the main physiological lipid substrate for Hb peroxidation reactions *in vivo* (Deuel *et al.,* 2015). The dose-response experiment shown in Figure 4C demonstrates that the steepest increase in MDA formation also occurred between 10 ^{-5.5} and 10 ⁻⁵ M oxyHb across a range of different reaction incubation times. Figure 4D plots the CSF-Hb concentrations of our patients on the identical x-axes as for the plotted values of the above described parameters. It demonstrates that the calculated cut-off concentration for CSF-Hb (optimal Youden Index), which discriminates between patients with SAH-SBI and without SAH-SBI, coincides with close-to-maximal basilar artery vasoconstriction, TCD values and generation of TBARS.

Collectively, the vascular function and lipid peroxidation studies indicate critical inflection points overlapping with the relevant CSF-Hb concentrations in patients with SAH-SBI.

G. Depletion and neutralization of CSF-Hb mitigates pathological vasoconstriction and lipid peroxidation.

To mechanistically link CSF-Hb in patients with SAH-SBI associated pathophysiologies, a series of Hb depletion and neutralization experiments were performed. Porcine basilar artery segments that were immersed in CSF, which was depleted of CSF-Hb by a haptoglobin column (Figure 5A), had a significantly lower contractile force than the segments immersed in the same CSF sample that was replenished with highly purified Hb to the original CSF-Hb concentration (Figure 5B). This experimental set-up controls for the potential nonspecific removal of vasoactive substances by the haptoglobin column. Also, we repeated a Hb dose response experiment to confirm the anti-vasospastic effect of soluble haptoglobin 1-1 (Hp1-1). Across the Hb concentration range, Hp 1-1 significantly attenuated the contractile force of basilar arteries (repeated measures ANOVA, p < 0.001) (Figure 5C).

The oxidative potential of CSF samples from patients after aSAH was also quantified. Compared to baseline CSF (day 0), variably increased MDA products was found after exposure of lipoprotein to CSF samples that were collected during the high risk phase (week 0.5 - 2; Figure 5D). Addition of Hp1-1 at equimolar concentrations to CSF-Hb effectively attenuated MDA formation. Noteably, the addition of hemopexin at an equimolar concentration showed an even stronger attenuation of MDA formation.

### Example 2: Quantitative assessment of hemoglobin and heme scavenger proteins in patient CSF samples

### A. Quantification of human Hp, Hpx and Albumin in CSF samples

Ten microliters of CSF sample were placed into a clean Eppendorf tube followed by the addition of 80 µL MeOH to precipitate the protein. The methanol was removed after centrifugation and the pellet was air-dried and afterwards re-suspended in 50 mM NH₄HCO₃/0.16% ProteaseMAX containing heavy-isoptope labeled peptides, which are specific for human haptoglobin, hemopexin and albumin and are used as internal standard. After incubation at 56°C/550 rpm for 45 min the samples were reduced by adding 0.5 M DTT (56°C/550 rpm for 20 min). The samples were then alkylated by addition of 0.5 M IAA and incubation for 20 min at RT protected from light. Tryptic digestion was carried out at 37°C/550 rpm and stopped after 3 h by addition of formic acid. After centrifugation the samples were separated immediately on a C18 column (AdvanceBio Peptide Mapping, 2.1 x 150 mm). The measurements were conducted using an Agilent 1290 Infinity II - 6550 iFunnel QTOF LC-MS system.

Data was analyzed by calculating the peak area of the analyte and the internal standard using Agilent MassHunter Quant software. A standard curve was created for each protein in Agilent MassHunter Quant by plotting the average response ratio of analyte to internal standard against concentration for each standard sample. The analyte concentration in the CSF samples was back calculated using the standard curve equation.

### B. Determination of free and Haptoglobin bound hemoglobin in CSF

Hp-bound and unbound fractions of Hb (cell free Hb) were determined by SEC-high-performance liquid chromatography (SEC-HPLC) using an Ultimate 3000SD HPLC attached to a LPG-3400SD quaternary pump and a photodiode array detector (DAD) (ThermoFisher). CSF samples and Hb standards were separated on a Diol-120 (3 µm, 300 X 8.0 mm) column (YMC CO Ltd.) with PBS, pH 7.4 (Bichsel) as the mobile phase at a flow rate of 1 mL/min. For all samples two wavelengths were recorded (λ = 280 nm and λ = 414 nm). Bound and unbound Hb in CSF was determined by calculating the peak area of both peaks (7-8 min retention time for Hb:Hp, depending on Hp phenotype, 10 min retention time for cell free Hb). Values from CSF samples were interpolated by generating a standard curve based on peak area and plotted against the concentrations.

### C. Determination of heme bound hemoproteins in CSF

Heme bound hemoproteins were determined by SEC-high-performance liquid chromatography (SEC-HPLC) using an Ultimate 3000SD HPLC attached to a LPG-3400SD quaternary pump and a photodiode array detector (DAD) (ThermoFisher). CSF samples and heme standards were separated on a Diol-120 (3 µm, 300 X 8.0 mm) column (YMC CO Ltd.) with PBS, pH 7.4 (Bichsel) as the mobile phase at a flow rate of 1 mL/min. For all samples two wavelengths were recorded (λ = 280 nm and λ = 414 nm). Hemoproteins in CSF was determined by calculating the peak area of both peaks (9 min retention time for heme bound hemoproteins, 10 min retention time for cell free Hb). Values from CSF samples were interpolated by generating a standard curve based on peak area and plotted against the concentrations.

### D. Detection of hemopexin:heme complexes in human CSF samples

50 microliters of CSF sample were placed into a clean Eppendorf tube followed by the addition of 150 µL Buffer A (Multiple Affinity Removal Systems, Agilent). In a first chromatography step high abundant human proteins were depleted and carried out according to the manufacturer's protocol on an Ultimate 3000SD HPLC attached to two LPG-3400SD quaternary pumps and a photodiode array detector (DAD) (ThermoFisher). Briefly, the diluted plasma sample was injected onto a multi affinity removal column depleting human albumin and IgG (Human IgG/HSA, 4.6 × 50 mm, Agilent) and separated with Buffer A (Multiple Affinity Removal Systems, Agilent) as the mobile phase at a flow rate of 0.25 mL/min. Depleted CSF was collected into a fresh HPLC vial and re-injected and separated on a Diol-300 (3 µm, 300 X 8.0 mm) column (YMC CO Ltd.) with PBS, pH 7.4 (Bichsel) as the mobile phase at a flow rate of 1 mL/min. For all samples two wavelengths were recorded (λ = 280 nm and λ = 414 nm). The amount of Hemopexin:heme complexes was determined by calculating the peak area of the complex (9 min retention time). Values from depleted CSF samples were interpolated by generating a standard curve based on peak area and plotted against the concentrations.

### Results

In CSF samples from 28 aSAH patients collected over a time period of 14 days, cell free hemoglobin was assessed (Figure 11). Although the methodology to quantify cell free Hb in CSF samples differed from the method applied within the same cohort as described in Example 1, above, the results are in perfect agreement (correlation coefficient (r) = 0.96). Again, the data demonstrated that cell free Hb levels increased over time and peaked around day 10 (1.5 week). In contrast and due to the infiltration of blood into the CSF, haptoglobin and hemopexin concentrations were increased at the very early timepoints compared to the healthy controls. Both scavenger proteins showed an inverse kinetic to hemoglobin levels, suggesting depletion due to the ongoing hemolysis. Albumin concentration remained comparable throughout the time course but significantly increased if compared to healthy control CSF samples.

In a next step, the scavenger proteins haptoglobin and hemopexin were investigated in terms of their presence as complex either with hemoglobin or heme. Overall and as expected based on the kinetic of both proteins, the values are very low and the majority of the data points were interpolated outside of the standard curve. Together with the cell free Hb data it let suggest that at early time points, where both proteins were clearly detected within patient CSF, no target is yet present and at late time points where Hb levels are increased, both scavengers are already depleted and cleared from the CSF. Hence, these data suggest that the natural Hb detoxification pathway is fully saturated and scavenger proteins are depleted. Albumin, an interim heme storage protein, was found to be elevated throughout the study duration and additional investigation of hemoproteins, heme bound proteins other than cell free Hb or Hp bound Hb, revealed a similar pattern as total albumin in CSF. These data suggest that the majority of detected hemoproteins is actually heme bound to albumin, since heme bound to hemopexin was identified as very low or even absent. In contrast to hemopexin, heme bound to albumin is still able to participate in iron mediated toxicity and therefore considered as one of the major contributors of secondary brain injuries in aSAH patients. These data support the use of hemopexin to further reduce heme-mediated oxidative damage in aSAH patients.

### Example 3 : Heme toxicity/hemopexin protection

### A. Striatal injection model

Wild-type male C57BL/6J mice (10-12 weeks) were obtained from Charles River (Sulzfeld, Germany) and maintained at the animal facility of the University of Zurich (LASC) and treated in accordance with the guidelines of the Swiss Federal Veterinary Office.

Mice were anesthetized with isoflurane (4-5% for induction, 1-2% for maintenance; Baxter Healthcare Co. Deerfield, IL, USA) evaporated in 100% oxygen. Throughout the surgical procedure, body temperature was monitored with a rectal probe and maintained constant with the use of an electronic thermostat-controlled warming blanket. Anesthetized mice were placed in a motorized stereotaxic frame (Stoelting, Dublin, Ireland) and a 1 cm long midline skin incision was made. After removal of the soft tissue covering the skull, the frame referenced to bregma and a small burrhole was drilled above the entry point for the target. Then a 33G needle was slowly advanced (0.1 mm/s) into the striatum (bregma coordinates: 2 mm ML, 0.5 mm AP, 3.5 mm DV) followed by injection of 10µL heme-albumin (1mM), haptoglobin (5 mM) (protein control group) or heme-hemopexin (1mM) with an infusion rate of 100 nl/s. The needle was left in place for 5 minutes, then removed slowly and the skin incision was closed with a suture. After the surgical procedure, the animals were placed in a heated wake up box and monitored until they were fully recovered and could be transferred back to their homecage.

### B. Behavioural Testing

The open field exploration tests were conducted in four identical square arenas (40×40×35 cm high) made of opaque acrylic glass, located in a testing room under diffused lighting (approximately 35 Ix as measured in the center of the arenas). A digital camera is mounted directly above the four arenas. Images are captured at a rate of 5 Hz and transmitted to a PC running the Ethovision (Noldus, The Netherlands) tracking system. For measuring basal locomotor activity, the animals are gently placed in the center of the arena and allowed to explore for 15 minutes. The total distance moved during 10 minutes (in cm), beginning after a 5 minutes acclimatization phase was measured.

For the rotarod behavioural testing the mice are placed on a rotating cylinder and therefore forced to walk. This allows to assess the forced locomotor activity. The rotation of the cylinder is accelerated from 5 revolutions (beginning) up to 40 revolutions per minute (end) over a time period of 300 seconds. The time until the mice fall off the cylinder is measured.

For discrete neurological deficits, mice were subjected to a beam walk test. There the mice have to cross an elevated beam to reach a safe place (small shelter). The beam has a length of 100cm and is 13mm wide. During the assessment, the motor function was scored by two investigators blinded to treatment.

### C. MRI protocol

Mice were anesthetized with isoflurane (4-5% for induction, 1-2% for maintenance; Baxter Healthcare Co. Deerfield, IL, USA) evaporated in 100% oxygen. Throughout the imaging procedure, body temperature was monitored with a rectal probe and maintained constant with the use of a warming blanket and respiratory rate was recorded by plethysmography. Mice were placed into a 7T small animal MRI scanner (Brucker, Germany). T2, ASL and DWI images were recorded according to a standardized protocol.

### Results

As shown in Figure 12, heme impaired neurological function in mice 24 hours after striatal injection, as evidenced by reduced open-field testing (Figure 12A), rotarod testing (Figure 12B) and beamwalk testing (Figure 12C). By contrast, mice that received striatal injection of heme:Hx complexes showed no impairement in neurobehavioral activity at the same time point (24 hours). The impaired neurological function was associated with radiological changes in mice that received a striatal injection of heme, included perilesional edema, a diffusion restriction in the ADC map and a focal perfusion deficit on arterial spine labelling (ASL) images (Figure 13). In mice that received a striatal injection of heme:Hpx, only minor radiological changes were seen. As shown in Figure 14, quantification of the radiological changes confirmed that striatal injection of heme was associated with the formation of cerebral edema and regionally decreased perfusion, which was absent in mice that were injected with heme:Hpx.

### Discussion

The present inventors have identified ventricular CSF-Hb as a biomarker for SAH-SBI that surprisingly exceeds the diagnostic accuracy of other established methods. Within the clinically relevant concentration range, CSF-Hb induced vasoconstriction and oxidized unsaturated lipids *ex vivo,* suggesting that it plays a role as an upstream mediatior of adverse secondary neurological outcomes following haemorrhagic stroke accompanied by extravascular erythrolysis and release of cell-free heme and/or cell-free haemoglobin (Hb) into a cerebral spinal fluid (CSF). The strong association of high CSF-Hb with adverse clinical outcomes, the underlying pathophysiological rationale, and the favorable effects of hemopexin and haptoglobin, position CSF-Hb as a suitable biomarker and target in patients at risk for SAH-SBI.

Besides its high diagnostic accuracy for adverse secondary neurological outcomes following a haemorrhagic stroke, such as DIND, DCI, and aVSP, the additional advantages of CSF-Hb consist in its simple and reliable analytics. Precise CSF-Hb values can be determined on the ICU in a simple two-step procedure consisting of a centrifugation step to remove cells and debris from CSF and subsequent spectrophotometry with automated spectral deconvolution. Furthermore, the physical law underlying spectrophotometry allows absolute quantification of CSF-Hb without a parallel measurement of standard curves or calibration samples. Collectively, this significantly reduces analytical cost and turnaround-time and may enhance widespread applicability and acceptance.

The present inventors have also shown that CSF-Hb is the major vasoconstrictor in patient CSF. Furthermore, the active dynamic range of Hb concentrations in the ex vivo vasospasm model overlaps critical inflection points that determine TCD flow velocities in large cerebral arteries and the presence or absence of SAH-SBD in our patient cohort. Within the same clinically relevant concentration range, CSF-Hb was found to be was highly active as a lipid oxidant, suggesting that oxidative Hb toxicity contributes to non-ischemic neuronal damage. Lipid oxidation activity in patient CSF was attributed, at least in part, to the downstream Hb metabolite, heme. Its selective neutralization with hemopexin virtually blocked the formation of MDA, while haptoglobin resulted in partial inhibition.

The data disclosed herein demonstrate that CSF-Hb is a suitable drug-target to prevent and treat SAH-SBI. This is supported by the strong association of CSF-Hb with adverse clinical outcomes, the rational pathophysiology, and the favorable functional effects of CSF-Hb neutralization with hemopexin and haptoglobin in the clinically relevant toxicity range. To date, the only drug reducing the occurrence of SAH-SBI after aSAH is the calcium channel blocker nimodipine (*Class I, Level A*)*.* While nimodipine has a positive effect on clinical outcomes, it does not prevent the occurrence of aVSP. Previous studies on the preventive effect of various drugs, CSF drainage, subarachnoid lavage, lamina terminalis fenestration or head shaking have not yet shown a significant effect on the risk of SAH-SBI. In already symptomatic patients, therapeutic options are limited to non-causal rescue therapies to improve regional cerebral blood flow.

In the CSF analyses described herein, endogenous haptoglobin was shown to be cleared from the CSF within the first days after aSAH, before relevant generation of cell-free Hb. Thus, the protective function of endogenous haptoglobin appeared neglectable, whereas exogenous haptoglobin provides anti-vasospastic and anti-oxidative effects within the clinically relevant CSF-Hb concentration range. Unexpectedly, the heme-scavenger protein hemopexin surpassed the antioxidant function of haptoglobin, suggesting that a fraction of heme may have already been released from oxidized or degraded CSF-Hb, forming a pool of oxidative free heme that remains associated with low-affinity heme-binding proteins. These data suggest that therapeutic combinations of haptoglobin and hemopexin in the context of aSAH are expected to provide synergistic protection against adverse secondary neurological outcomes following a haemorrhagic stroke.

The present inventors have also shown that hemopexin abrogates the heme-mediated impairement of neurological function following striatal injection. This effect correlated with radiological changes, noting that mice that received a striatal injection of heme showed perilesional edema, diffusion restriction in the ADC map and focal perfusion deficit on arterial spine labelling (ASL) images, whereas in mice that received a striatal injection of the heme:Hpx complex, these adverse radiological changes were largely absent.

The following items are embodiments of the invention:
1. A method of treating or preventing an adverse secondary neurological outcome in a subject following a haemorrhagic stroke accompanied by extravascular erythrolysis and release of cell-free heme and/or cell-free haemoglobin (Hb) into a cerebral spinal fluid (CSF), the method comprising exposing the CSF of a subject in need thereof to a therapeutically effective amount of hemopexin (Hx) and for a period of time sufficient to allow the Hx to form a complex with, and thereby neutralise, the cell-free heme.
2. The method of item 1, wherein the haemorrhagic stroke is a spontaneous haemorrhage or a traumatic haemorrhage.
3. The method of item 1 or item 2, wherein the haemorrhagic stroke is an intraventricular haemorrhage or a subarachnoid haemorrhage.
4. The method of item 3, wherein the subarachnoid haemorrhage is an aneurysmal subarachnoid haemorrhage.
5. The method of any one of items 1 to 4, wherein the adverse secondary neurological outcome is selected from the group consisting of a delayed ischaemic neurological deficit (DIND), delayed cerebral ischaemia (DCI), neurotoxicity, inflammation, nitric oxide depletion, oxidative tissue injury, cerebral vasospasm, cerebral vasoreactivity and oedema.
6. The method of item 5, wherein the adverse secondary neurological outcome is a cerebral vasospasm.
7. The method of item 5, wherein the adverse secondary neurological outcome is oxidative tissue damage.
8. The method of item 5, wherein the adverse secondary neurological outcome is a delayed ischaemic neurological deficit.
9. The method of item 5, wherein the adverse secondary neurological outcome is delayed cerebral ischaemia.
10. The method of any one of items 1 to 9, wherein the adverse secondary neurological outcome is an adverse secondary neurological outcome within the brain parenchyma.
11. The method of any one of items 1 to 10, comprising exposing the CSF to the Hx within about 21 days after onset of the haemorrhage.
12. The method of item 11, comprising exposing the CSF to the Hx from about 2 days to about 14 days after onset of the haemorrhage.
13. The method of any one of items 1 to 12, wherein the Hx is administered intracranially to the subject.
14. The method of any one of items 1 to 12, wherein the Hx is administered intrathecally to the subject.
15. The method of item 14, wherein the Hx is administered intrathecally into the spinal canal.
16. The method of item 14, wherein the Hx is administered intrathecally into the subarachnoid space.
17. The method of any one of items 1 to 12, wherein the Hx is administered intracerebroventricularly to the subject.
18. The method of item 17, wherein the Hx is administered intracerebroventricularly to the subject via an external ventricular drain.
19. The method of any one of items 13 to 18, wherein the period of time to which the CSF is exposed to the therapeutically effective amount of Hx is at least about 2 minutes.
20. The method of any one of items 1 to 19, wherein the therapeutically effective amount of Hx is at least an equimolar amount to the concentration of cell-free heme, or of cell-free Hb expressed as heme equivalent, as measured in the CSF of the subject following the haemorrhage.
21. The method of item 20, further comprising (i) obtaining a CSF sample from the subject following the haemorrhage and prior to exposing the CSF to the Hx; (ii) measuring the amount of cell-free Hb or cell-free heme in the CSF sample obtained in step (i); and (iii) determining the at least equimolar amount of Hx based on the concentration of cell-free Hb expressed as heme equivalent or cell-free heme from step (ii).
22. The method of any one of items 1 to 21, wherein the therapeutically effective amount of Hx is from about 2 µM to about 1 mM.
23. The method of item 22, wherein the therapeutically effective amount of Hx is from about 2 µM to about 400 µM.
24. The method of item 22, wherein the therapeutically effective amount of Hx is from about 5 µM to about 200 µM.
25. The method of item 22, wherein the therapeutically effective amount of Hx is from about 10 µM to about 200 µM.
26. The method of any one of items 1 to 25, further comprising removing Hx:cell-free heme complexes formed in the CSF.
27. The method of any one of items 1 to 12, comprising exposing the CSF to the therapeutically effective amount of Hx extracorporeally.
28. The method of item 27, comprising:
   (i) obtaining sample of CSF from the CSF compartment of the subject following the haemorrhage;
   (ii) adding to the CSF sample of step (i) Hx to obtain an Hx-enriched CSF sample;
   (iii) administering the Hx-enriched CSF sample to the subject, thereby exposing the CSF compartment of the subject to a therapeutically effective amount of Hx for a period of time sufficient to allow the Hx to form a complex with cell-free heme in the CSF compartment of the subject; and
   (iv) optionally repeating steps (i) to (iii).
29. The method of item 27, comprising:
   (i) removing a volume of CSF from the CSF compartment of the subject following the haemorrhage;
   (ii) providing an artificial CSF comprising Hx;
   (iii) administering the artificial CSF of (ii) to the subject, thereby exposing the CSF compartment of the subject to a therapeutically effective amount of Hx for a period of time sufficient to allow the Hx to form a complex with cell-free heme in the CSF compartment of the subject; and
   (iv) optionally repeating steps (i) to (iii).
30. The method of item 27, comprising:
   (i) obtaining CSF from the subject following the haemorrhage;
   (ii) exposing the CSF from step (i) to the Hx under conditions to allow the Hx, or the functional analogue thereof, to form a complex with cell-free heme in the CSF;
   (iii) extracting the Hx:cell-free heme complexes from the CSF following step (ii) to obtain a heme-diminished CSF that has a lower amount of cell-free heme when compared to the CSF from step (i);
   (iv) optionally repeating steps (ii) and (iii) to obtain an heme-diminished CSF that is substantially free of cell-free heme; and
   (vi) administering the heme-diminished CSF obtained from step (iii) or step (iv) to the CSF compartment of the subject.
31. The method of item 30, further comprising adding to the heme-diminished CSF prior to step (vi) a therapeutically effective amount of Hx.
32. The method of item 30 or item 31, wherein the Hx is immobilised on a substrate.
33. The method of item 32, wherein the substrate with immobilised Hx is selected from the group consisting of a size exclusion chromatography resin, an affinity chromatography resin, a filter and a membrane.
34. The method of item 33, wherein step (ii) comprises passing the CSF from step (i) through an affinity chromatography resin under conditions that allow the cell-free heme in the CSF to bind to the resin; wherein step (iii) comprises eluting the CSF from the resin following step (ii); and wherein step (iv) comprising recovering the eluted CSF.
35. The method of any one of items 30 to 34, optionally comprising washing the CSF compartment following step (i) with a wash solution.
36. The method of item 35, wherein the wash solution is an artificial CSF.
37. The method of item 29 or item 36, wherein the artificial CSF comprises NaCl, KCI, KH₂PO₄, NaHCO₃, MgCl₆H₂O, CaCl₂H₂O and glucose.
38. The method of any one of items 35 to 37, wherein the wash solution comprises Hx.
39. The method of item 38, wherein the wash solution comprises from about 2 µM to about 80 mM Hx.
40. The method of item 39, wherein the wash solution comprises from about 2 µM to about 1,200 µM Hx.
41. The method of any one of items 1 to 40, comprising:
   (i) removing CSF from the subject following the haemorrhage;
   (ii) rinsing the CSF compartment of the subject following step (i) with a wash solution comprising a therapeutically effective amount of Hx;
   (iii) optionally repeating step (ii); and
   (iv) administering to the CSF compartment of the subject, following step (ii) or step (iii), an artificial CSF.
42. The method of item 41, wherein the artificial CSF comprises NaCl, KCI, KH₂PO₄, NaHCO₃, MgCl₆H₂O, CaCl₂H₂O and glucose.
43. The method of item 41 or item 42, wherein the artificial CSF comprises Hx.
44. The method of item 43, wherein the artificial CSF comprises from about 2 µM to about 80 mM Hx.
45. The method of item 44, wherein the artificial CSF comprises from about 2 µM to about 1,200 µM Hx.
46. The method of any one of items 1 to 45, wherein the Hx is a recombinant protein.
47. The method of any one of items 1 to 45, wherein the Hx is plasma derived.
48. The method of any one of items 1 to 47, further comprising exposing the CSF of the subject to a therapeutically effective amount of haptoglobin (Hp) and for a period of time sufficient to allow the Hp to form a complex with, and thereby neutralise, cell-free Hb.
49. The method of item 48, comprising exposing the CSF to the Hp within about 21 days after onset of the haemorrhage.
50. The method of item 48, comprising exposing the CSF to the Hp from about 2 days to about 14 days after onset of the haemorrhage.
51. The method of any one of items 48 to 50, wherein the Hp is administered intracranially to the subject.
52. The method of any one of items 48 to 50, wherein the Hp is administered intrathecally to the subject.
53. The method of item 52, wherein the Hp is administered intrathecally into the spinal canal.
54. The method of item 52, wherein the Hp is administered intrathecally into the subarachnoid space.
55. The method of any one of items 48 to 50, wherein the Hp is administered intracerebroventricularly to the subject.
56. The method of any one of items 48 to 55, wherein the period of time to which the CSF is exposed to the Hp is at least about 2 minutes.
57. The method of any one of items 48 to 56, wherein the therapeutically effective amount of Hp is at least an equimolar amount to the concentration of cell-free Hb in the CSF of the subject following the haemorrhage.
58. The method of item 57, further comprising (i) obtaining a CSF sample from the subject following the haemorrhage and prior to exposing the CSF to the Hp; (ii) measuring the amount of cell-free Hb in the CSF sample obtained in step (i); and (iii) determining the at least equimolar amount of Hp based on the concentration of cell-free Hb from step (ii).
59. The method of any one of items 48 to 58, wherein the therapeutically effective amount of Hp is from about 2 µM to about 20 mM.
60. The method of item 59, wherein the therapeutically effective amount of Hp is from about 2 µM to about 300 µM.
61. The method of item 59, wherein the therapeutically effective amount of Hp is from about 5 µM to about 50 µM.
62. The method of item 59, wherein the therapeutically effective amount of Hp is from about 10 µM to about 30 µM.
63. The method of any one of items 48 to 62, further comprising removing Hp:cell-free Hb complexes formed in the CSF.
64. The method of any one of items 48 to 50, comprising exposing the CSF to the therapeutically effective amount of Hp extracorporeally.
65. The method of item 64, comprising:
   (j) obtaining sample of CSF from the CSF compartment of the subject following the haemorrhage;
   (ii) adding to the CSF sample of step (i) Hp to obtain an Hp-enriched CSF sample;
   (iii) administering the Hp-enriched CSF sample to the subject, thereby exposing the CSF compartment of the subject to a therapeutically effective amount of Hp for a period of time sufficient to allow the Hp to form a complex with cell-free Hb in the CSF compartment of the subject; and
   (iv) optionally repeating steps (i) to (iii).
66. The method of item 64, comprising:
   (j) removing a volume of CSF from the CSF compartment of the subject following the haemorrhage;
   (ii) providing an artificial CSF comprising Hp;
   (iii) administering the artificial CSF of (ii) to the subject, thereby exposing the CSF compartment of the subject to a therapeutically effective amount of Hp for a period of time sufficient to allow the Hp to form a complex with cell-free Hb in the CSF compartment of the subject; and
   (iv) optionally repeating steps (i) to (iii).
67. The method of item 64, comprising:
   (j) obtaining CSF from the subject following the haemorrhage;
   (ii) exposing the CSF from step (i) to the Hp under conditions to allow the Hp, or the functional analogue thereof, to form a complex with cell-free Hb in the CSF;
   (iii) extracting the Hp:cell-free Hb complexes from the CSF following step (ii) to obtain an Hb-diminished CSF that has a lower amount of cell-free Hb when compared to the CSF from step (i);
   (iv) optionally repeating steps (ii) and (iii) to obtain an Hb-diminished CSF that is substantially free of cell-free Hb; and
   (vi) administering the Hb-diminished CSF obtained from step (iii) or step (iv) to the CSF compartment of the subject.
68. The method of item 67, further comprising adding to the Hb-diminished CSF prior to step (vi) a therapeutically effective amount of Hp.
69. The method of item 67 or item 68, wherein the Hp is immobilised on a substrate.
70. The method of item 69, wherein the substrate is an affinity chromatography resin.
71. The method of item 70, wherein step (ii) comprises passing the CSF from step (i) through an affinity chromatography resin under conditions that allow the cell-free Hb in the CSF to bind to the resin; wherein step (iii) comprises eluting the CSF from the resin following step (ii); and wherein step (iv) comprising recovering the eluted CSF.
72. The method of any one of items 65 to 71, optionally comprising washing the CSF compartment following step (i) with a wash solution.
73. The method of item 72, wherein the wash solution is an artificial CSF.
74. The method of item 66 or item 73, wherein the artificial CSF comprises NaCl, KCl, KH₂PO₄, NaHCO₃, MgCl₆H₂0, CaCl₂H₂O and glucose.
75. The method of any one of items 72 to 74, wherein the wash solution comprises Hp.
76. The method of item 75, wherein the wash solution comprises from about 2 µM to about 20 mM Hp.
77. The method of item 76, wherein the wash solution comprises from about 2 µM to about 300 µM Hp.
78. The method of any one of items 48 to 77, comprising:
   (i) removing the CSF from the subject following the haemorrhage;
   (ii) rinsing the CSF compartment of the subject following step (i) with a wash solution comprising a therapeutically effective amount of Hp;
   (iii) optionally repeating step (ii); and
   (iv) administering to the CSF compartment of the subject, following step (ii) or step (iii), an artificial CSF.
79. The method of item 78, wherein the artificial CSF comprises NaCl, KCI, KH₂PO₄, NaHCO₃, MgCl₆H₂0, CaCl₂H₂O and glucose.
80. The method of item 78 or item 79, wherein the artificial CSF comprises Hp.
81. The method of item 80, wherein the artificial CSF comprises from about 2 µM to about 20 mM Hp.
82. The method of item 81, wherein the artificial CSF comprises from about 2 µM to about 300 µM Hp.
83. The method of any one of items 48 to 82, wherein the Hp is selected from the group consisting of an Hp1-1 homodimer, an Hp1-2 multimer, an Hp2-2 multimer and a combination of any of the foregoing.
84. The method of item 83, wherein the Hp comprises an Hp1-1 homodimer.
85. The method of any one of items 48 to 84, wherein the Hp is a recombinant protein.
86. The method of any one of items 48 to 84, wherein the Hp is plasma derived.
87. The method of any one of items 48 to 86, wherein the therapeutically effective amount of Hp is sufficient to reduce the amount of cell-free Hb in the CSF of the subject during treatment to a level of about 8 µM or less.
88. The method of item 87, wherein the therapeutically effective amount of Hp is sufficient to reduce the amount of cell-free Hb in the CSF of the subject during treatment to a level of about 7 µM or less.
89. The method of item 87, wherein the therapeutically effective amount of Hp is sufficient to reduce the amount of cell-free Hb in the CSF of the subject during treatment to a level of about 6 µM or less.
90. The method of any one of items 1 to 89, further comprising administering to the subject a second agent for treating or preventing an adverse secondary neurological outcome following a haemorrhagic stroke.
91. The method of item 90, wherein the second agent is a vasodilator.
92. The method of item 90 or item 91, wherein the second agent is selected from the group consisting of a sydnone and sodium nitroprusside.
93. A pharmaceutical composition for use in treating or preventing an adverse secondary neurological outcome in a subject following a haemorrhagic stroke in accordance with the method of any one of items 1 to 92, the composition comprising a therapeutically effective amount of Hx, and a pharmaceutically acceptable carrier.
94. A pharmaceutical composition for use in treating or preventing an adverse secondary neurological outcome in a subject following a haemorrhagic stroke in accordance with the method of any one of items 48 to 89, the composition comprising a therapeutically effective amount of Hp, and a pharmaceutically acceptable carrier.
95. The composition for use of item 94, wherein the therapeutically effective amount of Hp is sufficient to reduce the amount of cell-free Hb in the CSF of the subject during treatment to a level of about 8 µM or less.
96. The composition for use of item 95, wherein the therapeutically effective amount of Hp is sufficient to reduce the amount of cell-free Hb in the CSF of the subject during treatment to a level of about 7 µM or less.
97. The composition for use of item 95, wherein the therapeutically effective amount of Hp is sufficient to reduce the amount of cell-free Hb in the CSF of the subject during treatment to a level of about 6 µM or less.
98. Use of a therapeutically effective amount of hemopexin (Hx) in the manufacture of a medicament for treating or preventing an adverse secondary neurological outcome in a subject following a haemorrhagic stroke in accordance with the method of any one of items 1 to 92.
99. Use of a therapeutically effective amount of haptoglobin (Hp) in the manufacture of a medicament for treating or preventing an adverse secondary neurological outcome in a subject following a haemorrhagic stroke in accordance with the method of any one of items 48 to 88.
100. The use of item 99, wherein the therapeutically effective amount of Hp is sufficient to reduce the amount of cell-free Hb in the CSF of the subject during treatment to a level of about 8 µM or less.
101. The use of item 100, wherein the therapeutically effective amount of Hp is sufficient to reduce the amount of cell-free Hb in the CSF of the subject during treatment to a level of about 7 µM or less.
102. The use of item 100, wherein the therapeutically effective amount of Hp is sufficient to reduce the amount of cell-free Hb in the CSF of the subject during treatment to a level of about 6 µM or less.
103. A therapeutically effective amount of hemopexin (Hx) for use in the treatment or prevention of an adverse secondary neurological outcome in a subject following a haemorrhagic stroke in accordance with the method of any one of items 1 to 92.
104. A therapeutically effective amount of haptoglobin (Hp) for use in the treatment or prevention of an adverse secondary neurological outcome in a subject following a haemorrhagic stroke in accordance with the method of any one of items 48 to 89.
105. The therapeutically effective amount of Hp for use of item 104, wherein the therapeutically effective amount of Hp is sufficient to reduce the amount of cell-free Hb in the CSF of the subject during treatment to a level of about 8 µM or less.
106. The therapeutically effective amount of Hp for use of item 105, wherein the therapeutically effective amount of Hp is sufficient to reduce the amount of cell-free Hb in the CSF of the subject during treatment to a level of about 7 µM or less.
107. The therapeutically effective amount of Hp for use of item 105, wherein the therapeutically effective amount of Hp is sufficient to reduce the amount of cell-free Hb in the CSF of the subject during treatment to a level of about 6 µM or less.
108. A method of determining whether a subject is at risk of developing an adverse secondary neurological outcome following a haemorrhagic stroke accompanied by extravascular erythrolysis and release of cell-free heme and/or cell-free haemoglobin (Hb) into a cerebral spinal fluid (CSF), the method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).
109. The method of item 108, wherein the subject is determined to be at risk of developing adverse secondary neurological outcome where the amount of cell-free Hb in the CSF sample determined in step (ii) is at least about 7 µM.
110. The method of item 108, wherein the adverse secondary neurological outcome is angiographic vasospasm and the subject is determined to be at risk of developing angiographic vasospasm where the amount of cell-free Hb in the CSF sample determined in step (ii) is at least about 7 µM.
111. The method of item 108, wherein the adverse secondary neurological outcome is angiographic vasospasm and the subject is determined to be at risk of developing angiographic vasospasm where the amount of cell-free Hb in the CSF sample determined in step (ii) is at least about 7 µM.
112. The method of item 108, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.
113. The method of item 108, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.
114. The method of any one of items 108 to 113, further comprising treating the subject, determined to be at risk of developing an adverse secondary neurological outcome following a haemorrhagic stroke, in accordance with the method of any one of items 1 to 92.
115. The method of any one of items 1 to 92, wherein the subject is determined to be at risk of developing an adverse secondary neurological outcome following a haemorrhagic stroke accompanied by extravascular erythrolysis and release of cell-free heme and/or cell-free haemoglobin (Hb) into a cerebral spinal fluid (CSF) by a method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).
116. The method of item 115, wherein the subject is determined to be at risk of developing adverse secondary neurological outcome where the amount of cell-free Hb in the CSF sample determined in step (ii) is at least about 7 µM.
117. The method of item 115, wherein the adverse secondary neurological outcome is angiographic vasospasm and the subject is determined to be at risk of developing angiographic vasospasm where the amount of cell-free Hb in the CSF sample determined in step (ii) is at least about 7 µM.
118. The method of item 115, wherein the adverse secondary neurological outcome is angiographic vasospasm and the subject is determined to be at risk of developing angiographic vasospasm where the amount of cell-free Hb in the CSF sample determined in step (ii) is at least about 7 µM.
119. The method of item 115, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.
120. The method of item 115, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.
121. The composition for use of any one of items 93 to 97 wherein the subject is determined to be at risk of developing an adverse secondary neurological outcome following a haemorrhagic stroke accompanied by extravascular erythrolysis and release of cell-free heme and/or cell-free haemoglobin (Hb) into a cerebral spinal fluid (CSF) by a method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).
122. The composition for use of item 121, wherein the subject is determined to be at risk of developing adverse secondary neurological outcome where the amount of cell-free Hb in the CSF sample determined in step (ii) is at least about 7 µM.
123. The composition for use of item 121, wherein the adverse secondary neurological outcome is angiographic vasospasm and the subject is determined to be at risk of developing angiographic vasospasm where the amount of cell-free Hb in the CSF sample determined in step (ii) is at least about 7 µM.
124. The composition for use of item 121, wherein the adverse secondary neurological outcome is angiographic vasospasm and the subject is determined to be at risk of developing angiographic vasospasm where the amount of cell-free Hb in the CSF sample determined in step (ii) is at least about 7 µM.
125. The composition for use of item 121, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.
126. The composition for use of item 121, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.
127. The use of any one of items 98 to 102, wherein the subject is determined to be at risk of developing an adverse secondary neurological outcome following a haemorrhagic stroke accompanied by extravascular erythrolysis and release of cell-free heme and/or cell-free haemoglobin (Hb) into a cerebral spinal fluid (CSF) by a method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).
128. The use of item 127, wherein the subject is determined to be at risk of developing adverse secondary neurological outcome where the amount of cell-free Hb in the CSF sample determined in step (ii) is at least about 7 µM.
129. The use of item 127, wherein the adverse secondary neurological outcome is angiographic vasospasm and the subject is determined to be at risk of developing angiographic vasospasm where the amount of cell-free Hb in the CSF sample determined in step (ii) is at least about 7 µM.
130. The use of item 127, wherein the adverse secondary neurological outcome is angiographic vasospasm and the subject is determined to be at risk of developing angiographic vasospasm where the amount of cell-free Hb in the CSF sample determined in step (ii) is at least about 7 µM.
131. The use of item 127, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.
132. The use of item 127, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.
133. The therapeutically effective amount of Hp for use of any one of items 104 to 107, wherein the subject is determined to be at risk of developing an adverse secondary neurological outcome following a haemorrhagic stroke accompanied by extravascular erythrolysis and release of cell-free heme and/or cell-free haemoglobin (Hb) into a cerebral spinal fluid (CSF) by a method comprising (i) obtaining a CSF sample from the subject following the haemorrhage stroke; (ii) measuring the amount of cell-free Hb in the CSF sample obtained in step (i); and (iii) comparing the amount of cell-free Hb in the CSF sample determined in step (ii) with a reference value, wherein the subject's risk of developing an adverse secondary neurological outcome is determined based on the comparison in step (iii).
134. The therapeutically effective amount of Hp for use of item 133, wherein the subject is determined to be at risk of developing adverse secondary neurological outcome where the amount of cell-free Hb in the CSF sample determined in step (ii) is at least about 7 µM.
135. The therapeutically effective amount of Hp for use of item 133, wherein the adverse secondary neurological outcome is angiographic vasospasm and the subject is determined to be at risk of developing angiographic vasospasm where the amount of cell-free Hb in the CSF sample determined in step (ii) is at least about 7 µM.
136. The therapeutically effective amount of Hp for use of item 133, wherein the adverse secondary neurological outcome is angiographic vasospasm and the subject is determined to be at risk of developing angiographic vasospasm where the amount of cell-free Hb in the CSF sample determined in step (ii) is at least about 7 µM.
137. The therapeutically effective amount of Hp for use of item 133, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.
138. The therapeutically effective amount of Hp for use of item 133, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.

### References

1. Bates D, Mächler M, Bolker B, Walker S. Fitting Linear Mixed-Effects Models Usinglme4 [Internet]. Journal of Statistical Software 2015; Available from: http://dx.doi.org/10.18637/jss.v067.i01
2. Behrouz R. The Rise and Fall of Transcranial Doppler Ultrasonography for the Diagnosis of Vasospasm in Aneurysmal Subarachnoid Hemorrhage. J Neurosurg Anesthesiol 2019; 31: 79-80.
3. Bland M. An Introduction to Medical Statistics. Oxford University Press; 2015
4. Connolly ES Jr, Rabinstein AA, Carhuapoma JR, Derdeyn CP, Dion J, Higashida RT, et al. Guidelines for the management of aneurysmal subarachnoid hemorrhage: a guideline for healthcare professionals from the American Heart Association/american Stroke Association. Stroke 2012; 43: 1711-37.
5. Deuel JW, Vallelian F, Schaer CA, Puglia M, Buehler PW, Schaer DJ. Different target specificities of haptoglobin and hemopexin define a sequential protection system against vascular haemoglobin toxicity. Free Radic Biol Med 2015; 89: 931-43.
6. Dewey M. metap: meta-analysis of significance values. 2020
7. Diringer MN, Bleck TP, Claude Hemphill J 3rd, Menon D, Shutter L, Vespa P, et al. Critical care management of patients following aneurysmal subarachnoid hemorrhage: recommendations from the Neurocritical Care Society's Multidisciplinary Consensus Conference. Neurocrit Care 2011; 15: 211-40.
8. Elmer J, Harris DR, Sun G, Palmer AF. Purification of hemoglobin by tangential flow filtration with diafiltration. Biotechnol Prog 2009; 25: 1402-10.
9. Hugelshofer M, Buzzi RM, Schaer CA, Richter H, Akeret K, Anagnostakou V, et al. Haptoglobin administration into the subarachnoid space prevents hemoglobin-induced cerebral vasospasm. J Clin Invest 2019; 129: 5219-35.
10. Kassambara A, Mundt F. factoextra: Extract and Visualize the Results of Multivariate Data Analyses [Internet]. 2020; Available from: https://CRAN.R-project.org/package=factoextra
11. Kikinis R, Pieper SD, Vosburgh KG. 3D Slicer: A Platform for Subject-Specific Image Analysis, Visualization, and Clinical Support. Intraoperative Imaging and Image-Guided Therapy 2014: 277-89.
12. Lawrence MA. ez: Easy Analysis and Visualization of Factorial Experiments [Internet]. 2016; Available from: https://CRAN.R-project.org/package=ez
13. Liberzon A, Birger C, Thorvaldsdóttir H, Ghandi M, Mesirov JP, Tamayo P. The Molecular Signatures Database (MSigDB) hallmark gene set collection. Cell Syst 2015; 1: 417-25.
14. Md Riaz Ahmed. ROCit- An R Package for Performance Assessment of Binary Classifier with Visualization [Internet]. 2019 [cited 2020 Sep 17] Available from: https://openprairie.sdstate.edu/datascience_symposium/2019/posters/6/
15. Robin X, Turck N, Hainard A, Tiberti N, Lisacek F, Sanchez J-C, et al. pROC: an open-source package for R and S to analyze and compare ROC curves [Internet]. BMC Bioinformatics 2011; Available from: http://dx.doi.org/10.1186/1471-2105-12-77
16. Sachs MC. plotROC: A Tool for Plotting ROC Curves [Internet]. J Stat Softw 2017; Available from: http://dx.doi.org/10.18637/jss.v079.c02
17. Sing T, Sander O, Beerenwinkel N, Lengauer T. ROCR: visualizing classifier performance in R.Bioinformatics 2005; 21: 3940-1.
18. Sloan MA, Haley EC Jr, Kassell NF, Henry ML, Stewart SR, Beskin RR, et al. Sensitivity and specificity of transcranial Doppler ultrasonography in the diagnosis of vasospasm following subarachnoid hemorrhage. Neurology 1989; 39: 1514-8.
19. Subramanian A, Tamayo P, Mootha VK, Mukherjee S, Ebert BL, Gillette MA, et al. Gene set enrichment analysis: a knowledge-based approach for interpreting genome-wide expression profiles. Proc Natl Acad Sci U S A 2005; 102: 15545-50.
20. Weidert S, Andress S, Linhart C, Suero EM, Greiner A, Böcker W, et al. 3D printing method for next-day acetabular fracture surgery using a surface filtering pipeline: feasibility and 1-year clinical results. International Journal of Computer Assisted Radiology and Surgery 2020; 15: 565-75.
21. Wood SN. Generalized Additive Models: An Introduction with R, Second Edition. CRC Press; 2017

## Claims

1. An in vitro method of determining whether a subject is at risk of developing an adverse secondary neurological outcome following a haemorrhagic stroke accompanied by extravascular erythrolysis and release of cell-free heme and/or cell-free haemoglobin (Hb) into a cerebral spinal fluid (CSF), the method comprising (i) measuring in a CSF sample, obtained from the subject following the haemorrhagic stroke, the amount of cell-free Hb; and (ii) comparing the amount of cell-free Hb in the CSF sample determined in step (i) with a reference value, wherein the subject's risk of developing an adverse secondary neurological outcome is determined based on the comparison in step (ii).

2. The method of claim 1, wherein the subject is determined to be at risk of developing adverse secondary neurological outcome where the amount of cell-free Hb in the CSF sample determined in step (ii) is at least about 7 µM.

3. The method of claim 1, wherein the adverse secondary neurological outcome is angiographic vasospasm and the subject is determined to be at risk of developing angiographic vasospasm where the amount of cell-free Hb in the CSF sample determined in step (ii) is at least about 7 µM.

4. The method of claim 1, wherein the adverse secondary neurological outcome is delayed cerebral ischemia and the subject is determined to be at risk of developing delayed cerebral ischemia where the amount of cell-free Hb in the CSF sample determined in step (ii) is at least about 3 µM.

5. The method of claim 1, wherein the adverse secondary neurological outcome is delayed ischemic neurological deficit and the subject is determined to be at risk of developing delayed ischemic neurological deficit where the amount of cell-free Hb in the CSF sample determined in step (ii) is at least about 7 µM.

6. The method of claim 1, wherein the cell-free haemoglobin is oxyhemoglobin (oxyHb) or methemoglobin (metHb).

7. The method of claim 6, wherein the cell-free hemoglobin is oxyHb.

8. The method of claim 6, wherein the cell-free hemoglobin is metHb.

9. Hemopexin (Hx) for use in the treatment of a subject being at risk of an adverse secondary neurological outcome following a haemorrhagic stroke, wherein said subject is determined to be at risk of developing an adverse secondary neurological outcome following a haemorrhagic stroke according to the method of any one of claims 1-8, wherein the administration of Hx is for a period of time sufficient to allow the Hx to form a complex with, and thereby neutralise, the cell-free heme.
